(19) Europäisches Patentamt
European Patent Office
Office européen des brevets

(11) **EP 1 162 276 A2**

(12) **EUROPÄISCHE PATENTANMELDUNG**

(43) Veröffentlichungstag:
**12.12.2001 Patentblatt 2001/50**

(51) Int Cl.[7]: **C12Q 1/68**, C07K 14/47, C07K 16/18

(21) Anmeldenummer: **01109427.3**

(22) Anmeldetag: **23.04.2001**

(84) Benannte Vertragsstaaten:
**AT BE CH CY DE DK ES FI FR GB GR IE IT LI LU MC NL PT SE TR**
Benannte Erstreckungsstaaten:
**AL LT LV MK RO SI**

(30) Priorität: **06.05.2000 DE 10021834**

(71) Anmelder: **Lynx Therapeutics GmbH**
**69120 Heidelberg (DE)**

(72) Erfinder:
- **Kramer, Michael Dieter**
  **64319 Pfungstadt (DE)**
- **Winter, Holger**
  **69120 Heidelberg (DE)**
- **Reinartz, Jeannette**
  **69121 Heidelberg (DE)**

(74) Vertreter: **Rudolph, Ulrike, Dr.**
**Patentanwältin**
**In der Schanz 10**
**69198 Schriesheim (DE)**

# (54) mRNA Moleküle zur Verwendung als Indikatoren für den Aktivierungs- und Funktionszustand von T-Lymphozyten

(57) Die Erfindung betrifft mRNA Moleküle zur Verwendung als Indikatoren für den Aktivierungs- und Funktionszustand von T-Zellen, die dadurch gekennzeichnet sind, daß sie bei aktivierten T-Zellen im Vergleich zum Normal- bzw. Ruhezustand vermehrt oder vermindert exprimiert (auf- bzw. abreguliert) sind und auf dem höheren bzw. niedrigeren Konzentrationsspiegel gehalten werden, und daß sie mit einer der in den Sequenzprotokollen SEQ ID NO. 1 bis NO. 334 dargestellten oder einer davon abgeleiteten oder dazu komplementären Nukleotidsequenz oder einer Teilsequenz davon hybridisieren. Die Erfindung betrifft ferner die in den Sequenzprotokollen dargestellten Nukleotidsequenzen sowie davon abgeleitete oder dazu komplementäre Nukleotidsequenzen oder Teilsequenzen davon (i) zur Verwendung als Nachweisreagenz für den Aktivierungs- und Funktionszustand von T-Zellen, (ii) als bzw. zur Herstellung von Diagnostika und/oder Therapeutika, (iii) zur Identifizierung von medizinisch oder pharmakologisch einsetzbaren Substanzen, (iv) zur Herstellung von Bindemolekülen und (v) zur Herstellung von Testbestecken. Die Erfindung betrifft außerdem die durch die dargestellten Nukleotidsequenzen kodierten Polypeptide, dagegen gerichtete Antikörper und die Verwendung solcher Antikörper zum Nachweis oder zur Beeinflussung des Aktivierungs- und Funktionszustands von T-Zellen.

EP 1 162 276 A2

SEQUENZPROTOKOLL

<110> LYNX Therapeutics GmbH

<120> mRNA Moleküle zur Verwendung als Indikatoren für den Aktivierungszustand von T-Lymphozyten

<130> KM11

<140>
<141>

<160> 334

<170> PatentIn Ver. 2.1

<210> 1
<211> 207
<212> DNA
<213> Homo sapiens

<400> 1
gttttgtttt gtttggtttt ttgtttttga gacagggtct cactctttca cccaggctag 60
agtgcagtgg tgccatctta gctcattgca acctccgtct cctgggctca aacaattctc 120
ctgcctcagc ctcccaagta gctgggatta caggcacgtg ccaccacact cgggtaattt 180
ttgtattttt aggagagacg gggtttc 207

<210> 2
<211> 127
<212> DNA
<213> Homo sapiens

<400> 2
agtactacat tttataacaa tagagagtag ctgaaaatac tacatgctaa cacagataat 60
atgatacaca acctcagggg ggaagctggc agggagcacg tggcagaggc cacaggttta 120
gactaag 127

<210> 3
<211> 53
<212> DNA
<213> Homo sapiens

<400> 3
gatctgccaa aaagaactaa cacctgtgag aaataaagtg tatcctgact ctt 53

<210> 4
<211> 78
<212> DNA
<213> Homo sapiens

<400> 4
gatcgcctgc ctcagcctcc caaagtgctg ggattacagg cgtgagccat catgcccggc 60
ctattatttc cttttatt 78

<210> 5
<211> 222
<212> DNA
<213> Homo sapiens

<400> 5
gatcacaagg tcaggagttc gagaccagcc tggccaacat ggtgaaaccc cgtctctcct 60
aaaaatacaa aaattaaccg agtgtggtgg cacgtgcctg taatcccagc tacttgggag 120
gctgaggcag gagaattgtt tgagcccagg agacggaggt tgcaatgagc taagatggta 180
ccactgcact ctagcctggg tgacagagca agactccatc tc 222

<210> 6
<211> 48
<212> DNA
<213> Homo sapiens

<400> 6
gatcatgcca ctgcactcca gcctggtgac agagcgaggc tccatctc 48

<210> 7
<211> 175
<212> DNA
<213> Homo sapiens

<400> 7
gatccaatgg agcctgggag gtgtgcccag aaagcttgtc tgtagcgggt tttgtgagag 60
tgaacacttt ccacttttg acaccttatc ctgatgtatg gttccaggat ttggattttg 120
attttccaaa tgtagcttga aatttcaata aactttgctc tgtttttcta aaaat 175

<210> 8
<211> 233
<212> DNA
<213> Homo sapiens

<400> 8

```
ctttattttg ggacagagtc tcactctgtt gcccaggcta gagtgcagtg gcacaatctt 60
tactcgccgc aacctctgcc tcccaggttc aagcgattct agtgcctcag cctcctgagt 120
agctgggact gcaggcacgt aacaccacac ccagctaatt tttgtatttt tagtagagac 180
agggtttcac catgtttgcc aggctggtct caacctcctg acctcaagtg atc        233
```

<210> 9
<211> 50
<212> DNA
<213> Homo sapiens

<400> 9

```
gatcatgcta cagcactcga agcctgggga acagagcaag aacttgtctc            50
```

<210> 10
<211> 224
<212> DNA
<213> Homo sapiens

<400> 10

```
gagacagagt cttgctcttt ctcccaggct cgtgtgcagt agcacagtct cagctcactg 60
caacctctgc ctcccaggtt catgcgattc tcctgcccca gcctcctgag tagctgggat 120
tacaggcgtg tgccaccacg cacagctagt ttttgtattt ttagtagaga caaggtttca 180
acatgttggc cagcctggtc ttgcactcct gacctcaagt gatc                  224
```

<210> 11
<211> 67
<212> DNA
<213> Homo sapiens

<400> 11

```
aggttaaagt gctgatgcca tttaattaga ttgtttacaa atttaacagg atggctacaa 60
agagatc                                                           67
```

<210> 12
<211> 81
<212> DNA
<213> Homo sapiens

<400> 12

```
gaagcttctc acatctttat tggaaaggca cagctaagcc caccccttgat acagcatttt 60
ccacttctct ctgtagagat c                                           81
```

<210> 13

<211> 203
<212> DNA
<213> Homo sapiens

<400> 13
gatctcagaa caatcagatg caaagctgaa agagattgta acaaatttct tggctggatt 60
tgaagcttaa actcctgtgg attcacatca aataccagtt cagttttgtc attgttctag 120
taaattagtt ccatttgtaa aagggttact ctcatactcc ttatgtacag aaatcacatg 180
aaaaataaag gttccataat gca 203

<210> 14
<211> 223
<212> DNA
<213> Homo sapiens

<400> 14
gaagcacagt ctcactctgt cgcccaggct ggagtgcagt ggcacaaccc tggctcactg 60
caatctccgc ctcccaggtt caagcaattc tcctgcctca acctcctgag tagctaagat 120
tacaggcgcg tatcaccaca caggctaatt tttttatttt tagtagagac aaggtctcac 180
catgttggac aggctggtct tgaactcctg acctcaggtg atc 223

<210> 15
<211> 59
<212> DNA
<213> Homo sapiens

<400> 15
gatctgtggg ttaaccctct acgctactac ctgagagaaa ggggctccag gataaagag 59

<210> 16
<211> 141
<212> DNA
<213> Homo sapiens

<400> 16
gatctagaag yagaggaatc ccagcgcctt ttaaaagttg ttatgtggtt ttcttttaaa 60
aagctcctgt ttttggaaag tagaatttat gggtacaacg tatgttcatt atttgtacat 120
aaaataaaac catttaaaaa g 141

<210> 17
<211> 244
<212> DNA
<213> Homo sapiens

<400> 17
gatcccggaa tgcctgaacc aactgttttg agcctacttt ggggataaag gattatttgg 60
tcttctggat ttggaggcaa tcagcggaca gcatggaaga tgtgtgctct ggctcggata 120
agagatggga catcattcag tcactagttg gatggcacaa ggctcttcac agacgcatct 180
gtagcagagt ggaacttgta ctaacttatg atagaatgta tcagaataaa tgtttttaac 240
agtg 244

<210> 18
<211> 62
<212> DNA
<213> Homo sapiens

<400> 18
aatagagagg gggtttcacc atgttggcca ggctggtctc gaactcctga cttccagtga 60
tc 62

<210> 19
<211> 83
<212> DNA
<213> Homo sapiens

<400> 19
gggtttttg tggcttcctt cgttattgga gccaggccta caccccagca accatgtctg 60
acaaacccga tatggctgag atc 83

<210> 20
<211> 53
<212> DNA
<213> Homo sapiens

<400> 20
aaggcaggag agacaaagaa tgagctttaa agtgcatgtt tacagaaatg atc 53

<210> 21
<211> 223
<212> DNA
<213> Homo sapiens

<400> 21
gaggcggagt ctcgctctgt cacccatacc tgagtgcagt ggcgccatct ccactcactg 60
caacctctgc ctcctgggtt caagtgattc tcctgcttca gcctcccaag tagctgggat 120
tcaggcaccc accaccatgc ccagctaatt tgtgtatttt tagtagaggc agggtttcac 180
catgtgggcc agactggtct cgaactcctg acctcaagtg atc 223

<210> 22
<211> 112
<212> DNA
<213> Homo sapiens

<400> 22
gatctatatc ttatcacaag ttttaaactg caaataattt ctacagaaaa aaaatagctc 60
caaatttcta aattttaaag attaaagaag ctcagtaaac tccaagaagt ac 112

<210> 23
<211> 247
<212> DNA
<213> Homo sapiens

<400> 23
gttttaaaat attatagtaa tggcttattg ataggtaatt tggttagtag agttataaat 60
acaaaaaggg aaaggttacc agaaataaga cactatttct aacaaatctt caagaaaatc 120
ttgattatta aagtcccttt tatcttacaa gtgttgcata tacagttttg gctcttcaga 180
gccctctacc aataaggcac ctgcaatagt agataatttg ctaccgcagc cttagagagc 240
agttctt 247

<210> 24
<211> 142
<212> DNA
<213> Homo sapiens

<400> 24
gatcaagaaa ctggaaaatg tgaagcaatc tctattgaga tagcatctta gtgttttaga 60
gaaatcaaga atttttaaaa acaagaatat caacatttgg ttttgtgtat aagtggtgtt 120
tgtattaaaa tactttttca at 142

<210> 25
<211> 317
<212> DNA
<213> Homo sapiens

<400> 25
gatcatgaag acctgcttta gccccaacag agtgattgga ctctcaagtg acttgcagca 60
agtaggaggg gcgtcagctc gcatccagga tgccctgagt acagtgttgc aatatgcaga 120
ggatgtactg tctggaaagg tgtcagctga caatactgtg ggccgcttcc tgatgagcct 180
ggttaaccaa gtaccgaaaa tagttcccga tgactttgag accatgctca acagcaacat 240
caatgacctt ttgatggtga cctacttggc caacctcaca cagtcacaga ttgcactcaa 300
tgaaaaactt gtaaacc 317

<210> 26
<211> 70
<212> DNA
<213> Homo sapiens

<400> 26
gtatttttag tagagacggg gtttccccat gatggccaga ctggttttga actcctaatc 60
tcaagtgatc 70

<210> 27
<211> 86
<212> DNA
<213> Homo sapiens

<400> 27
gatcatgaag aagaaagtga aggagataca gataaagatg ggacaaatct gctcagtgtg 60
gatgaagatg rggattctga aacctc 86

<210> 28
<211> 61
<212> DNA
<213> Homo sapiens

<300>

<400> 28
gtagagatgt ggtctcccta tgttgcccag gctggtctca aactcctggg ctcaagtgat 60
c 61

<210> 29
<211> 161
<212> DNA
<213> Homo sapiens

<400> 29
aaagacaggg tgttactctg tgaccctggc ttattgtctc tatactgagg ataatctcga 60
gtgtgtcccc taccacccct gccacgccct ccataagaac ctctatagct tcgacctctg 120
gagtaatatt cacctcggcc tcgacctctg tatccttgat c 161

<210> 30
<211> 116
<212> DNA
<213> Homo sapiens

<400> 30
gatccgcatc tccatcaatt agaacactgg aaaagatgtt ttataaaaga ggtatttaat 60
tttgtttgta ggattaactc atgcaaataa taaaaaagat atcctgttgg ttcaac 116

<210> 31
<211> 205
<212> DNA
<213> Homo sapiens

<400> 31
gagatggagt cttgctctgt cgccaggctt gagttcagtg gtgctatctc ggctcactgc 60
aacctccgcc tcgcgggttc aagtgattct cctgcctcag cctgccgagt agctgggact 120
acaggcgcat gccactacac ccagctaatt tttgtatttt tagtagagac agggtttcac 180
catgttggcc aggatggttt tgatc 205

<210> 32
<211> 267
<212> DNA
<213> Homo sapiens

<400> 32
ctcttgagac agggtctcac tttgttgtcc aggctggagt gcaatggcat gaccatggct 60
cactgctgcc ttgaactcct gggctcaagc aatcttttca taggaactgt tggagcaact 120
gtttggagaa ctactatctg aaagacaata acatattttt ccctaaagac attcttgccc 180
ccagggcaca cttttctggc tgccatcaac cctrctgcct agattcaacg aratggggat 240
gtcagatgat tctgtgagca acagatc 267

<210> 33
<211> 112
<212> DNA
<213> Homo sapiens

<400> 33
cctttgaagc agaaaactca cttcaaaata agagagaaaa ttaagtctga gcttcactca 60
tcattaaaac tgaattacaa ccattacata taaactacag tgtcaagaga ca 112

<210> 34
<211> 224
<212> DNA
<213> Homo sapiens

<400> 34
gatcacctga gttcaggatt ttgagaccag cctggccaac atgacaaaac cccatctcta 60

ctaaaaatac aaaaattcac caggcttggt ggcaggcgac tgtagttcca gctactgaag 120
aggctgaggc aggagaatca cttgaacctg ggaggtggag gctgcagtga gctgagatgg 180
caccactgta ctccagcctg ggtgacagag caagactcca tctc 224

<210> 35
<211> 167
<212> DNA
<213> Homo sapiens

<400> 35
gagaatttag ctgttcttta ttgacatgga atttggggtg gcgagggtag ccacaccctc 60
caggaggatg aggtaggttc agtgcttcca gctcacacct ttcctgggtc ttccttcagt 120
gtgacagttc ggttaaagac aagctttccc tgatggctgt ctggatc 167

<210> 36
<211> 185
<212> DNA
<213> Homo sapiens

<400> 36
gatcaggtgg aaaggggagt cattctttga cttttaagac attaagagaa aaaagaattg 60
aagaacaaaa gcaaaaacct tttgaaagat aaagacaaaa agcagatgct ccagggtgtc 120
ttctgcatgt ctgccactgt tctccagacc accttggctt tggtccagaa caagaacagc 180
taagg 185

<210> 37
<211> 208
<212> DNA
<213> Homo sapiens

<400> 37
ggaaatttcc ttgttggcaa attttattga cagctttcag gaaaagattc tcgccccgtg 60
gccactcttg ctgaagcaac acaatcacat gccccaatgc catgtcgtct ctgttgtctg 120
tgaaagctct aagcttaaaa caggctaaca ttaaatggag gcagtatggc atgatagcct 180
tgctggtaca aggcaggagc ttcagatc 208

<210> 38
<211> 227
<212> DNA
<213> Homo sapiens

<400> 38
gatccccttc tttcccatga gagagagaac tggtgatact ccaacaccgt ccagttgtgg 60
cagctctcca gaagtaatag cagctgacaa ctttctgtgc cttttccttt ctgttgaaaa 120

ggcatagaaa gttctgggaa cataaacatt tttacccttt tctatgccat ttattttgta 180
aaaatcctat ttaacagtta tttaataaaa caatattttt agaaacc 227

<210> 39
<211> 248
<212> DNA
<213> Homo sapiens

<400> 39
ctggaaaagc agtttaacat tttctaaatg gattttatcc cacatttact gtataatgta 60
gctgttaata ctgcacaagt acacttagcg ataatgttta ctttacttta aaacaaaggg 120
attttctccc tcaaaacaag ttttcaacat caaacctatg cttataaaaa tttaaaactt 180
tcagcagtct atttcaaatg aaaaccatta caaacttctc aagtgttctc tatattccag 240
gtatgtca 248

<210> 40
<211> 251
<212> DNA
<213> Homo sapiens

<400> 40
gatcccagct catctctcat ttatttcggc ttcttttatt ccaggattaa tgtagtgtaa 60
cattttcatt tcttttcgct tttattctgc ttttgtaaaa gcagtatttt gagatggaca 120
ttgcctcttc attgtatttc tcatcaattc attatttttg tggttatagc ttgacaagca 180
attaacttta aaatggtaga ttccgtaact ttaaattggt agctttcatt tgcttaaaat 240
tttttggcat a 251

<210> 41
<211> 50
<212> DNA
<213> Homo sapiens

<400> 41
gatcgagaca ttgccctcca gcctgggcaa caagagcaaa actccatctc 50

<210> 42
<211> 72
<212> DNA
<213> Homo sapiens

<400> 42
gatcgtgcca ttgcactcca gactgagtga cagagtgaga ctgtgtttaa aaaaaaagtt 60
tgaattaaaa aa 72

<210> 43
<211> 120
<212> DNA
<213> Homo sapiens

<400> 43
aaactggata aaatttttat tttaaaacat cacaagtaat tacaaagaca aatattccaa 60
ctatacaaac tgtttttcac atatacatat atacatacat atatttaagg ttggttgatc 120

<210> 44
<211> 250
<212> DNA
<213> Homo sapiens

<400> 44
gatcggagag tgctcccgaa ggccaggccc aacaacgccg gccctaccgc aggcgaaggt 60
tcccacctta ctacatgcgg agaccctatg ggcgtggacc acagtattcc aaccctcctg 120
tgcagggaga agtgatggag ggtgctgaca accagggtgc aggagaacaa ggtagaccag 180
tgaggcagaa tatgtatcgg ggatatagac cacgattccg caggggccct cctcgccaaa 240
gacagcctag 250

<210> 45
<211> 197
<212> DNA
<213> Homo sapiens

<400> 45
agttttagta gagaccgggt ttcaccgtgt tggtcaggct ggtctcgaac tcctggcctc 60
aagtgatttg ctcacctcgg cctcccaaag tgctgggatt acaggcatga gccaccatgc 120
ccggcctatt tagcataatt cttaaaggca ctaggatttt tggaatgtaa atgagcattg 180
gcttcaactt caagtca 197

<210> 46
<211> 154
<212> DNA
<213> Homo sapiens

<400> 46
gatctgggat ttctaatata ctagcagtaa acaaagcaaa ttcctggcaa caactgtcag 60
gttagtgatg ctaactccct tcccccaacc accataaaat tttagtaaga aaagaattaa 120
aagtaatgac taagcagaac aaattggaaa taac 154

<210> 47

<211> 258
<212> DNA
<213> Homo sapiens

<400> 47
gatccaggtg ataactggga agaaggtgga ggtggtggtg gaggtatgga aaaatcttca 60
ggtccctgga ataaaacagc tccagtacaa gcacctcctg ctccagtaat tgttacagaa 120
accccagaac cagcgatgac tagtggtgtg tataggcctc ctggggccag gttaaccaca 180
acaaggaaaa caccacaagg accaccagaa atctacagtg atacacagtt cccatccctg 240
cagtcaactg ccaagcat 258

<210> 48
<211> 243
<212> DNA
<213> Homo sapiens

<400> 48
gtagtagaag gaggataccc tgatgatgga atcgcagatg agtaagactg agcaagttcc 60
actgagacct gggaggtctt ctgttgtaat cctccgttgc tcacctgagt ggaatctcca 120
attgggctac aggataaaga tgactgcctg gttgtttcct gaaaattaac tacacttgaa 180
tttgatagat aactatgtag ggagtgctgt gaaccagtca gttgcgcctg aattgactgg 240
gta 243

<210> 49
<211> 38
<212> DNA
<213> Homo sapiens

<400> 49
tctgagcaag ggtacccgcc acccagcagc cactgatc 38

<210> 50
<211> 48
<212> DNA
<213> Homo sapiens

<400> 50
gatsgtgcca ctgcactcca gtttggtgac agagcaagac tctgtctc 48

<210> 51
<211> 61
<212> DNA
<213> Homo sapiens

<400> 51
gtacagacag ggtctcgcta tattacccag gctggactta aatttgtggc ttcaagtgat 60
c 61

<210> 52
<211> 50
<212> DNA
<213> Homo sapiens

<400> 52
aaagacggag tcccgttctg tagaccaggc tggagtgcag tggcatgatc 50

<210> 53
<211> 188
<212> DNA
<213> Homo sapiens

<400> 53
ttatttgctg aggagtgctt tacttccaac tatgtggtca attttggaat aagtgcagtg 60
tggtgctgag aagaatgtat attctgttga tttggggtgg agagttctgt agatgyctat 120
taggtctgct tgstgcagag ctgagttcaa ttcctggata tctttgttaa ctttctgtct 180
cgttgatc 188

<210> 54
<211> 58
<212> DNA
<213> Homo sapiens

<400> 54
gagatgaggt cttgctatgt tgcccaggct agtttcaacc tcctgggctc aagtgatc 58

<210> 55
<211> 224
<212> DNA
<213> Homo sapiens

<400> 55
gatcacgctg aggtcaggag tttgagccag cctggccaac atggtgaaac cccatctcca 60
ctaaaaatag aaaaattagc cgggcatggt gacacatgcc tgtagtccta gctacttggg 120
aggctgaggc aggagaattg cttgaatctg ggaggcagag gttgcagtga gccgagattg 180
tgccattgca ctgcagcctg gatgacagag cgagtctcta tctc 224

<210> 56

<211> 182
<212> DNA
<213> Homo sapiens

<400> 56
gatcctgcag ctgtcactca ctgagcactg agccatagcc ccgggagggc tggccaggcc 60
actccctgcc cgcttttgta atttatttat ttataaactc tctgctgctg agcttggggc 120
ctggagcccc aggaatgagc aggcagggga gactgagatg gaaataaaga gactgtcgca 180
gc 182

<210> 57
<211> 107
<212> DNA
<213> Homo sapiens

<400> 57
aaaaagaaag aggcttttta ttacattgtt acaggcaatg cattacgtga gctggtcccg 60
tgtacaatcc ccaagccacg gcaaaacatc aggagaacgt accgatc 107

<210> 58
<211> 225
<212> DNA
<213> Homo sapiens

<400> 58
gtgattgaat ctcactctgt tgtccaggct ggggtgcagt ggcgtaatca ctgctcacta 60
caaacctctg ccttctgggt tcaaatgatt ctcctgcttt agcctcccga gtagctggga 120
ttacaggtgt ccacaaccac atccagctaa tttttgtatt tttagtagag atggggtttc 180
accatgttgt tcatgctggt cttgaacttc tgacctcagg tgatc 225

<210> 59
<211> 168
<212> DNA
<213> Homo sapiens

<400> 59
ctggtcaggt gaagtttatt tacagtaggt taagaaatta ataaaaagtc agaaatccta 60
agtcagttgg aaaacccaag ctgtaatgta cccaaacctt ctgagttttt cgctgagttg 120
cttgcttctg attttcaccc aaggcagttg tcacaaatta ttcagatc 168

<210> 60
<211> 80
<212> DNA
<213> Homo sapiens

<400> 60
gatcagcaag gtcacagtat acaagggaat atgaaaatca attgcattac tagtttctgg 60
caatgaaaat gtcaacactg 80

<210> 61
<211> 81
<212> DNA
<213> Homo sapiens

<400> 61
agacagggac tcactctgtc gctcaagctg gaatcacaat cacagctcac tgcacccttg 60
acctactggg ctcaagggat c 81

<210> 62
<211> 54
<212> DNA
<213> Homo sapiens

<400> 62
tgtatttttt agtagagacg gggtttcacc gtgttagcca ggatggtctc gatc 54

<210> 63
<211> 215
<212> DNA
<213> Homo sapiens

<400> 63
gatcacgcca ctacactcca gcctggtgac agagcaagac tccatctcaa aaaaaaaaaa 60
tctaagcaaa ctatctttga gtcaaaaaaa gttggataac tcatctatat ttgaaataat 120
gctgtctgaa tggccaacat cccaagaatg tattaaaaac agcaaccctg cctaccgttt 180
tgatgacctt tttccatatt aaacaagttg agaac 215

<210> 64
<211> 299
<212> DNA
<213> Homo sapiens

<400> 64
gatctgctta gaatggcaat catgtcactt tattggctaa tttcacaggg tagctatttc 60
ctcttgtgag cttttgagaa gttgaagtct gataatatgt gccagaattt ggacttttga 120
gtactgtagt caaaatctga aagttgaaat ttttgcctta gtaggtacct gtgttttatt 180
gtaacaacca gaagataatc tgaacttccc agcaactaca tggaagctga gattcagtaa 240
aacccaaagt ataatggttt agaatgctta aaacccatca gagccatcct caaaaccaa 299

<210> 65
<211> 179
<212> DNA
<213> Homo sapiens

<400> 65
gatcacctga gcccaggagc tagaggctgc agtgagctat gaatacacca ctgctttcca 60
gcgatcctcc tgcctctgcc tcccaagctg ctgggattac aggcatgaac cactgtgccc 120
agctgaaatg aataaattgg ataggcttaa caaaatggat acaacaaaat ggatacaac 179

<210> 66
<211> 77
<212> DNA
<213> Homo sapiens

<400> 66
gagacagggt ctcgttctgg ggcacagtag ggcaaacatg gctccctgca gcctcaacct 60
cctaggctca actgatc 77

<210> 67
<211> 172
<212> DNA
<213> Homo sapiens

<400> 67
gatccggaaa tatggcctca atatgtgccg ccagtgtttc cgtcagtacg cgaaggatat 60
cggtttcatt aagttggact aaatgctctt ccttcagagg attatccggg gcatctactc 120
aatgaaaaac catgataatt ctttgtatat aaaataaaca tttgaaaaaa cc 172

<210> 68
<211> 61
<212> DNA
<213> Homo sapiens

<400> 68
gtagagaagg ggtcttgtta tgttagccag gctggtctcc aacccctagc ctcaagcgat 60
c 61

<210> 69
<211> 83
<212> DNA
<213> Homo sapiens

<400> 69
gtttaaaatg aaaagtttat ttgctgagta caccaaatag gatgcaagca ctgtcatgac 60
aaatatacag aaatatgcag atc 83

<210> 70
<211> 60
<212> DNA
<213> Homo sapiens

<400> 70
ttgaagggat gttctctgaa gaatgtataa tggacgtcat tggatgttta gaatatgatc 60

<210> 71
<211> 334
<212> DNA
<213> Homo sapiens

<400> 71
gatcaggaca gccttgtggc cagccttgac aacgttagga atctctccac tatcttgaaa 60
gctattcatt tccgagaaca tgccacgtgt ttcgcaacta aaaatggtat caaagtaaca 120
gtggaaaatg caaagtgtgt gcaagcaaat gcttttattc agggacttta actgcacttc 180
gaatgtgtta ccaaggttat ggttaccctt tgatgctgtt cctggaagaa ggaggagtgg 240
tgacagtctg caaaatcaat acacaggaac ctgaggagac cctggacttt gatttctgca 300
gcaccaatgt tattaataaa attattctgc agtc 334

<210> 72
<211> 51
<212> DNA
<213> Homo sapiens

<400> 72
gatcatgcca ctgcactcca gcctgagcta tagagcgaga ctccttctct c 51

<210> 73
<211> 285
<212> DNA
<213> Homo sapiens

<400> 73
gatcagttca gaaaacaagt tctcttaaag ggagaaagga ataaaaaata tgaatgtaat 60
aatatttaaa ctaacatcat ttagggaatg atatgctgca tatatgtgat tccatgtaat 120
tgaaaataca taaatactaa aacattgtat tcattttcga cagaccctct ttctaaagcg 180
tytgytgtgt tgtattacac gttaacacyc atatttgtat gtagcaaata tttatttgat 240

aactgttact tgctggacrc tgagattaaa cataatttaa ccttc 285

<210> 74
<211> 51
<212> DNA
<213> Homo sapiens

<400> 74
cagtttacat gaaggcaatt tattaacaga aaatattttg aggaatcttg t 51

<210> 75
<211> 90
<212> DNA
<213> Homo sapiens

<400> 75
gggcacttga gcccagaagc tcgagaccaa tctgggcaat atagcgagac cccgtctcta 60
aaaaggaaaa aaatattkat ttttaaattg 90

<210> 76
<211> 201
<212> DNA
<213> Homo sapiens

<400> 76
gatcgcgcag gatttcaaaa ccgacctgag gtttcagagc gcagccatcg gtgcgctgca 60
ggaggctagc gaagcgtacc tggtgggtct gttcgaagat accaacctgt gtgccatcca 120
cgctaagaga gtcaccatca tgcccaaaga catccagttg gctcgccgga tacggggaga 180
gagagcttaa gtgaaggcag t 201

<210> 77
<211> 141
<212> DNA
<213> Homo sapiens

<400> 77
ctgtctcctg ggttcacgcc attctcctgc ctcggcctcc caagtagctg ggactacagg 60
cccctgctac cacgcccagc taattttta tatttttagt agagatgggg tttcaccatg 120
ttagccagga tggtctcgat c 141

<210> 78
<211> 74
<212> DNA

<213> Homo sapiens

<400> 78
gatccgggca atgcttcgtt ttctaaagga tgctgctgtt gaagctttga attttacaat 60
aaactttttg aaac 74

<210> 79
<211> 50
<212> DNA
<213> Homo sapiens

<400> 79
gatcgcacca ctgcactcca gcctgggcaa caagagaaac tccatctcac 50

<210> 80
<211> 205
<212> DNA
<213> Homo sapiens

<400> 80
gatcacctgg ggtcgagagt tcaagaccag cctggtcaac atggtgaaac cccatctcta 60
cttaagaata caaaaattag cacgtgccac catgctaatt gggaggctga ggcacgagaa 120
tcgctggaac ctgggaggta gaggttgcag tgagccaaga ttgcgtcact gcactctagc 180
ctgggtgaca gagtgaggct gtctc 205

<210> 81
<211> 58
<212> DNA
<213> Homo sapiens

<400> 81
gatgacacca ctgcactcca gcctgggtga cagaatgagt ccctcattta aaacaaac 58

<210> 82
<211> 38
<212> DNA
<213> Homo sapiens

<400> 82
gatcttccta taagcccaat ttacttggct agaaagcg 38

<210> 83
<211> 144

<212> DNA
<213> Homo sapiens

<400> 83
gatctaaaga aacagttggc tgtagctgag gggaaacccc ctgaagcccc taaaggcaag 60
aagaaaaagt aaaagacctt ggctcataga aagtcacttt aatagatagg gacagtaata 120
aataaatgta caatctctat aaac 144

<210> 84
<211> 86
<212> DNA
<213> Homo sapiens

<400> 84
gatgtggtca gcagtgatga tgaagagcct agcacctctt atactgatga gaatattaaa 60
cgtaaagacc atattgatta tcagaa 86

<210> 85
<211> 79
<212> DNA
<213> Homo sapiens

<400> 85
gatcattgtt aactttctga tgtgtttcct ttcagttaca tagttattta agctaataaa 60
tttggaatgc tgtaactat 79

<210> 86
<211> 223
<212> DNA
<213> Homo sapiens

<400> 86
ctaagacaga atcttgttct gttgcccagg ctggagtaca gtggtgtcat ctcaactcac 60
tgcaacctcc gcctcccagg tttaagcaat tctcctgctt caccctcctg agtagctggg 120
attacaggca cctgccacca cacctggcta atttttgtat tttagtagag aaagggtttc 180
actctgttgg ccaggctggt cttgaactcc tgacctcgtg atc 223

<210> 87
<211> 62
<212> DNA
<213> Homo sapiens

<400> 87
agtagagaca gggtttcgcc gtgttggcca gcttggtctc aaactcctgg cctcaagtga 60

tc 62

<210> 88
<211> 28
<212> DNA
<213> Homo sapiens

<400> 88
gatcaaagaa gaaaagaaga aggggaag 28

<210> 89
<211> 186
<212> DNA
<213> Homo sapiens

<400> 89
gaaaccctgt ctctactaaa aatacaaaaa ttagctgggc atagtggtac gtgcctgtag 60
tcccagctac ttgggaggca gaggcaggag aattgcttga acccgggaga tggaggttgc 120
agtgagccga gatcgcgcca ctgcactcta gcctggcaac agagtgagac tccatctcan 180
aaaaag 186

<210> 90
<211> 32
<212> DNA
<213> Homo sapiens

<400> 90
gatcaacaaa attgatagac tgctagcaag ac 32

<210> 91
<211> 68
<212> DNA
<213> Homo sapiens

<400> 91
gatcacttga ggtcaggagt tggagaccag cctggccaac atgacgaacc ccagtctcta 60
ccaaaaaa 68

<210> 92
<211> 61
<212> DNA
<213> Homo sapiens

<400> 92
gatcgcttca gcccaggagt ttgagaccaa tctgggcaac atagtgagac cttgtctcta 60
c 61

<210> 93
<211> 51
<212> DNA
<213> Homo sapiens

<400> 93
gatcatgcta ctacactcca gactgggaga cagagcaata ccttgtttgt c 51

<210> 94
<211> 55
<212> DNA
<213> Homo sapiens

<400> 94
gatcacgcca ttgcactcca gtctgggtga tgagtgagac tccatctcan aaaaa 55

<210> 95
<211> 155
<212> DNA
<213> Homo sapiens

<400> 95
gatctgaacc tccaattaaa aaagccaaag aggagactcc gaagactgac aatactaaat 60
catcatcttc ctctcagaag gatgaaaaaa tcactggaac ccccagaaaa gctcactcta 120
aatcagcaaa agaacaccaa gaaacaaaac cagtc 155

<210> 96
<211> 229
<212> DNA
<213> Homo sapiens

<400> 96
gatctcatga ggcgaataag ctgcaagaca tgttggatgg attcattaaa aaatttgttc 60
tctgtcctga atgtgagaat cctgaaacag atttgcatgt caatccaaag aagcaaacaa 120
taggtaattc ttgtaaagcc tgtggctatc gaggcatgct tgacacacat cataaactct 180
gcacactcat tctcaaaaac ccacctgaga atagtgacag tggtacagg 229

<210> 97
<211> 37

<212> DNA
<213> Homo sapiens

<400> 97
gatcacacca ctgcactcca gcttgggctc aaaaaag 37

<210> 98
<211> 81
<212> DNA
<213> Homo sapiens

<400> 98
gatccctcaa aacctcacta actggaagga tgattttgtc tcagtttgta ctcctaaata 60
aaaagtaaac akgacacctc t 81

<210> 99
<211> 224
<212> DNA
<213> Homo sapiens

<400> 99
gatcacttga ggtcaggagt tcgagaccag cctggccagc atggtgaaac cccgtctcta 60
ctgaaaatac aaaacttagc taggcatgat ggcgggcgcc tgtaatccca gctacctggg 120
aggctgaggc aggagaatcg cttgaacctg ggaggcagag gttgctgtga gcaaggattg 180
tgccactgca ctccagcctg ggtgacagag tgagactcca tctc 224

<210> 100
<211> 70
<212> DNA
<213> Homo sapiens

<400> 100
ggtgagtgtt ttagtggggt tagcgatgga ggtaggattg gtgctgtggg tgaaagagta 60
tgatggggtg 70

<210> 101
<211> 51
<212> DNA
<213> Homo sapiens

<400> 101
gatcacacca ctgcatccca gcatgggaga caaaatgaga ccctgtctct t 51

<210> 102
<211> 266
<212> DNA
<213> Homo sapiens

<400> 102
gatcctgtca cgaacacatt tagcacacat ggaaccacca taggccctgc tgacatgttt 60
ctttgttttg gacaatctca taagaacttt aggtcttaca gctcgaaccc ctcgaagtct 120
gcctgggcac acaccacatg cagattttgg tgctttccca accttcttgg tataaaggta 180
aacaattcta ttaccagggg ttcgggacag cctagttttg ttagaggctg tattgtagga 240
aagcctacgt cggtatgtca aacgct 266

<210> 103
<211> 72
<212> DNA
<213> Homo sapiens

<400> 103
gatcttgcra actctgcaat cgcaagctgt tcccgagacc cctaggaaaa gaagaaaatt 60
aggtgacttc ac 72

<210> 104
<211> 55
<212> DNA
<213> Homo sapiens

<400> 104
ggaataatct aaagctatta tttccaaaac tttgttacca ttttatagca tgatc 55

<210> 105
<211> 236
<212> DNA
<213> Homo sapiens

<400> 105
ccctgtcttt atttccttac caatcggctg ccatccgagg agctgaggaa gcctagagct 60
ctcagaagca gtcctttgag ctggtgtagg ggcactcaga atggtccagc gtttgacata 120
ccgacgtagg ctttcctaca atacagcctc taacaaaact aggctgtccc gaacccctgg 180
taatagaatt gtttaccttt ataccaagaa ggttgggaaa gcaccaaaat ctgcat 236

<210> 106
<211> 133
<212> DNA
<213> Homo sapiens

<400> 106
gatctcagcg gcattaagct gtgcctgagc gagtttgtag tgactcactg cacagcaccc 60
ccagactagc atgtggttct atatttgtaa agttattggg ataagaaaca attaaacagt 120
ttgtagtaaa cac 133

<210> 107
<211> 199
<212> DNA
<213> Homo sapiens

<400> 107
gagatggagt gcaatggcgt gatattggct cactgcaacc gccgcctcct gggttcaagc 60
ggttctcctg cctcagcctc ttgagtagct gggattacag gcttgtgcca ccatacctgg 120
ctaatttttg tatttttagt acagacgggg tttcaccata ttggccaggc tggtctcgaa 180
ctcctgacca caggtgatc 199

<210> 108
<211> 268
<212> DNA
<213> Homo sapiens

<400> 108
tyttcctctt ccggggacgt tgtctgcagg cactcagaat ggtccagcgt ttgacatacc 60
gacgtaggct ttcctacaat acagcctcta acaaamctag gctgtcccga acccctggta 120
atagaattgt ttacctttat accaagaagg ttgggaaagc accaaaatct gcatgtggtg 180
tgtgcccagg cagacttcga ggggttcgtg ctgtaagacc taaagttctt atgagattgt 240
ccaaaacaaa gaaacatgtc agcagggc 268

<210> 109
<211> 102
<212> DNA
<213> Homo sapiens

<400> 109
gatcctacat ctgttgggtc ttaggcctcc ttccctcctc agtgtctttc aaatgacttt 60
catcaaatga ctttcaaaat aaaaccttat tttggcaaag gc 102

<210> 110
<211> 183
<212> DNA
<213> Homo sapiens

<400> 110

gatcacaaca cctctacagc argggcacag tsctgggwgg aggttgagat ggatgaattg 60
acagaagtag gcttcagarg gtgggtagta gcraacttca ctgagctaaa ggagyacgyt 120
ctaacccaac ayattggaac gaatcccaga acttaaagat tggttctcta aaataagaca 180
gac 183

<210> 111
<211> 173
<212> DNA
<213> Homo sapiens

<400> 111
ccttttgcgg ccatcaccga agcgggagcg gccaaaatga agtttaatcc ctttgtgact 60
tccgaccgaa gcaagaatcg caaaaggcat ttcaatgcac cttcccacat tcgaaggaag 120
attatgtctt cccctctttc caaagagctg agacagaagt acaacgtgcg atc 173

<210> 112
<211> 224
<212> DNA
<213> Homo sapiens

<400> 112
gacacagagt cttgctctgt cacccaggct ggagtgcagt ggcgcaatct gggctcactg 60
caaactccac ctcctgggtt taagtgattc tcctgcctca gcttctcgag tagctgggat 120
tacaggtgcc caccaccacg tctggctaat ttttgcattt ttagtagaga tggggtttca 180
ccatgttggc caggctggtc tcgaactcct gacctcaagc gatc 224

<210> 113
<211> 75
<212> DNA
<213> Homo sapiens

<400> 113
gatcacttct ctcatttcag cttcactttc tgacacagga gcaataatcc taaagtgagg 60
agggggagtc actty 75

<210> 114
<211> 106
<212> DNA
<213> Homo sapiens

<400> 114
gatcttattg aaggacatct tacagcttcc caatgagagg ccaggaagtg tgaacatact 60
gatagaaaaa gactatattt tatccctcat aaaatgtttt aaatgc 106

<210> 115
<211> 121
<212> DNA
<213> Homo sapiens

<400> 115
ctctctttcc tttaattttt ctgtaggaca cccactcgca cttcgttttt cagcagcagt 60
cctgagagag gaaccctttt gattttgaag ttattcagtg taacaggcag taatgatgat 120
c 121

<210> 116
<211> 228
<212> DNA
<213> Homo sapiens

<400> 116
gatctaaagt tctggctgtc cattaacctc caactatggt ctttatttct tgtggtaata 60
tgaygtgcct ttccttgcct aaatcccttc ctggtgtgta tcaacattat ttaatgtctt 120
ctaattcagt catttttttta taagtatgtc tataaacatt gaactttaaa aaacttattt 180
atttattcca ctactgtagc aattgacaga ttaaaaaaat gtaacttc 228

<210> 117
<211> 229
<212> DNA
<213> Homo sapiens

<400> 117
gacagtgtgg caagatttaa ttgcaacggc atctgaaaat ggtgtgtggc gtcgcgcgcg 60
ccagctatcg tcagtgcctt tattgccatt gggtttgtga ctgttgatat agtgacgacc 120
ccaggagcaa caggtgggtt aaaaacccgt ggctgggcag ttcttccgag gagagggacc 180
acatgaggag ccaagaagga ctcagaacct gacgaacgtg gcatcgatc 229

<210> 118
<211> 87
<212> DNA
<213> Homo sapiens

<400> 118
gatcagtcta caggtcacag tggatttctt ttcaaactga caatgtttag gttttaagca 60
aataaagtyc cagttaatgt gaaactc 87

<210> 119
<211> 214

<212> DNA
<213> Homo sapiens

<400> 119
gagacggaat cttgctctgt cacccaggct ggagtgcaat ggtgcgatct cagctgactg 60
taacctccgc ctcctgggtt cgagattctc ctgcctcagc ctcccaagta gctgggacta 120
caggcaccca ccaccacacc tggctaattt ttttgtattt ttagtaaaga cggggtttca 180
ctatgttggc caggctggtc tcgaactcct gacc 214

<210> 120
<211> 267
<212> DNA
<213> Homo sapiens

<400> 120
gatcgcgcca ctgtactcca gcctgggcga ctcaaaaaaa aagtgccaga cttggtggct 60
gaggtggaag gattgcttga gcccaataga ccagtctgag caacatagca agaccccatc 120
tctacaaatg gcatgtgtct gtggtcccag ctacctggga ggctgaggca ggattgcttg 180
aacccaggag attgaggctg cagtgagcca tgattatgcc actgtactcc agcctgtgct 240
atagagcaag accttgtccc cctgtgc 267

<210> 121
<211> 170
<212> DNA
<213> Homo sapiens

<400> 121
cctagtcctg tatgcccttt tcctaacact cacaacaaaa ctaactaata ctaacatctc 60
agacgctcag gaaatagaaa ccgtctgaac tatcctgccc gccatcatcc tagtcctcat 120
cgccctccca tccctacgca tcctttacat aacagacgag gtcaacgatc 170

<210> 122
<211> 56
<212> DNA
<213> Homo sapiens

<400> 122
cttaactgag acagggtctc actctgtcac tcaggctgca gtacagtggc atgatc 56

<210> 123
<211> 193
<212> DNA
<213> Homo sapiens

<400> 123
gatcatgaca aagaagatta aaatttcatt agcatgaatg caatttgtta aagcagactg 60
atttgtttct aagatatttt tggttttttt aaaactgata ataatgctga attatcttaa 120
gtgagatgtt aagcccactt tgttctttta atgtaatgga gcttatgggt agaagaccat 180
gtctactaat tac 193

<210> 124
<211> 204
<212> DNA
<213> Homo sapiens

<400> 124
acgcttcttt tattagtttt tattagtttt tcaaaagtat cagaagtaac agaatgttgg 60
gagaagatag atctgccttt tgaaaccaaa tatttaatat tttcattaaa ttgtttcaat 120
acaacttcag gcaaatgcca actggaagac caagcccaga aattcagagg aaataatctt 180
ccaaacaagg aacaccaagg tgat 204

<210> 125
<211> 51
<212> DNA
<213> Homo sapiens

<400> 125
gatcgcgcca ctgcactcca gcctgggcaa caaagagcga aactccgact c 51

<210> 126
<211> 120
<212> DNA
<213> Homo sapiens

<400> 126
ggttttaaac ctttaatgag aaaaaaatat ataataccga gctcaaaaac actgtgtttg 60
gtgtcattgg gtcaagggtt ggggatacac acggcagaat tcggaaacag ggtatagatc 120

<210> 127
<211> 33
<212> DNA
<213> Homo sapiens

<400> 127
gatcagtaat ttcaagacac caagcaaatt atc 33

<210> 128

<211> 125
<212> DNA
<213> Homo sapiens

<400> 128
gcaggttagc attttattag ataaaaacag gaaggagggt gcctacccac ccctgcctgc 60
tactggctct gaagggcact cctcaacttc cccaagaaag aggacgcgtc tctgacactg 120
tgatc 125

<210> 129
<211> 111
<212> DNA
<213> Homo sapiens

<400> 129
gatctcagca atgcccatgg ggcaatttac atcgggactc gtttcatctc tagaccttca 60
cttacctgag aaaggcaagt gtcttttgat aaataaaata ttgttaaatg c 111

<210> 130
<211> 98
<212> DNA
<213> Homo sapiens

<400> 130
gatctgctaa tgctttttag gagtctgcct ggaaactttg acatggttca tgtttttact 60
caaaatccaa taaaacaagg tagtttgrct gwgcaaaa 98

<210> 131
<211> 52
<212> DNA
<213> Homo sapiens

<400> 131
gatcgcgcta ctgcactcca gcctgagcga tagagcaaga ctccatctca tt 52

<210> 132
<211> 216
<212> DNA
<213> Homo sapiens

<400> 132
gatcatgccg aaaaagtggc ggaaaagcta gaagctctct cggtgaagga ggagaccaag 60
gaggatgctg aggagaagca ataaatcgtc ttattttatt ttcttttcct ctctttcctt 120
tcctttttttt aaaaaatttt accctgcccc tctttttcgg tttgttttta ttctttcatt 180

```
tttacaaggg acgttatata aagagctgaa ctcaac                          216


<210> 133
<211> 50
<212> DNA
<213> Homo sapiens

<400> 133
gatctattca tatgactata tgaggttaaa actcaataaa gtctgatgac           50


<210> 134
<211> 113
<212> DNA
<213> Homo sapiens

<400> 134
gatcacttaa gcccaggggt caaggctgca gtgagctgtg atgacatcag tgcatcagtg 60
cattccagcc tgggcgacag agtgagaccc tgtctctaaa caaacaaaca aac       113


<210> 135
<211> 223
<212> DNA
<213> Homo sapiens

<400> 135
gatcaataac aggttctgaa attgaggcaa taattaatag cctatcaact aaaataagtc 60
caggaccaga tggattcaca gctgaattct accagaggta caaagaggag ctggtaccat 120
tccttctgaa actattccaa ccaatagaaa aagagggaat cctccctaac tcattttatg 180
aggccagcat catcctgata ccaaagcctg gcggagacac aac                  223


<210> 136
<211> 102
<212> DNA
<213> Homo sapiens

<400> 136
gatcttcagc tctgcgaaat tccttcgctt tttcttaagg gtttctggca cagcaggaac 60
ctccttcttc ttctcttcta caccctccat ggttccagcc gg                   102


<210> 137
<211> 198
<212> DNA
<213> Homo sapiens
```

<400> 137
gatcatgtgt ttgggggctt ttaggggacc cagctgcact ggggcactgc ccgtggcctg 60
ggtaggacat ttcccagcaa gggctggagg agttgccgtg ccttcagcct gaatcgaatg 120
tcagaaccag ccagcggtgc ttcaccctct tggggataac ttgcttagtt ttttaataaa 180
tgttcctggt tggttttc 198

<210> 138
<211> 72
<212> DNA
<213> Homo sapiens

<400> 138
gatcacttaa gtccaggagt tcaagaccag cctagkcgat ataggcaagc ctcctcatct 60
ctactaaaaa gt 72

<210> 139
<211> 269
<212> DNA
<213> Homo sapiens

<400> 139
gcactgattc agactttaat ggaggtgctc atttcaatgc cacagaggtg gtggcaactg 60
tggaacgtgg catggggagt gagaggttgc tctggtgcag ctggaggaag aacaggggac 120
ctagggttgg ggagagatgt atagaggaaa actcccccag gcacacagcc tccgctctgg 180
accaacgcag gcttcagtga gtacacacaa aggaactgat gtcaaggccc tttctatgac 240
ccttcccatt ctagcaagac ctcccaccc 269

<210> 140
<211> 194
<212> DNA
<213> Homo sapiens

<400> 140
gatcccaccg tggagacatc tgtagtgtgt atgtccttgt aacactctgt tttcagggac 60
tacaaccttt ttccttctgt gaccagcccc ggattcaggc tgtactaata ccaggtatat 120
trtggaattt agtataaagg ccaatctgtc ctgaagtcca tctaatgtga tgaatcactg 180
aaagtctcag aaag 194

<210> 141
<211> 89
<212> DNA
<213> Homo sapiens

<400> 141
atattaataa cagatatgtt tattattaca tatccatcag tgcggctttc aataccactt 60
gaaacatgca tatcatccta gagacgatc 89

<210> 142
<211> 62
<212> DNA
<213> Homo sapiens

<400> 142
gtagagacaa ggtttggcca cattgcccaa gctggtctca aactccttgg gctcaagtga 60
tc 62

<210> 143
<211> 203
<212> DNA
<213> Homo sapiens

<400> 143
ggctttaaca tttattacaa agataagagc agaggctggt gagttacact tcttcctccc 60
caccaggtgc tctctgcagc tctggaaaaa tggtgtcctc tttgttgtcc caccaggggg 120
cgccacctcc agccccgccc cagcctcata cccagttctt cagctcggcc agcggtaact 180
gaagcctccc agaatcctgg atc 203

<210> 144
<211> 81
<212> DNA
<213> Homo sapiens

<400> 144
tttttttttt tgttttttta gtagagacac ggtctcacta tgttgcccag gctggtctta 60
aactcctggc ctcaagtgat c 81

<210> 145
<211> 311
<212> DNA
<213> Homo sapiens

<400> 145
gatcactttt gagctctgaa gctttgaatc attcagtggt ggagatggcc ttctggtaac 60
tgaatattac cttctgtagg aaaaggtgga aaataagcat ctagaaggtt gttgtgaatg 120
actctgtgct ggcaaaaatg cttgaaacct ctatatttct ttcgttcata agaggtaaag 180
gtcaaatttt tcaacaaaag tcttttaata acaaaagcat gcagttctct gtgaaatctc 240
aaatattgtt gtaatagtct gtttcaatct taaaaagaat caataaaaac aaacaagggg 300

aaaaaaaaaa a 311

<210> 146
<211> 150
<212> DNA
<213> Homo sapiens

<400> 146
gatcacgtag gttcgtggct gcagcaaaag ttgggtttca caaagttgaa aaacagccgg 60
tttctcaaac aattgtgatt tggaaggttt cgctgggttt gcccagatgt ttacgaataa 120
ataagaaatg cagttttaaa aaaaaaaaaa 150

<210> 147
<211> 281
<212> DNA
<213> Homo sapiens

<400> 147
gatccgtatg gccaacgaga agcacagcaa gaacatcacc cagcgcggca acgtcgccaa 60
gacctcgaga aatgcccccg aagagaaggc gtctgtagga ccctggttat tggctctctt 120
catttttgtt gtctgtggtt ctgcaatttt ccagattatt caaagtatca ggatgggcat 180
gtgaagtgac tgaccttaag atgtttccat tctcctgtga attttaactt gaactcattc 240
ctgatgtttg ataccctggt tgaaaacaat tcagtaaagc a 281

<210> 148
<211> 77
<212> DNA
<213> Homo sapiens

<400> 148
tttttttttg atttagagtt taatgtaaat tttgtatttt atacacacca atacaaacaa 60
gtaattacaa acagatc 77

<210> 149
<211> 233
<212> DNA
<213> Homo sapiens

<400> 149
tttttttttt tgaggcggag tcttgctctt ctcgcccagg ctggagtgca atgacacaat 60
cttggcccac tgcaaagtct gcctccctgg gtcaagcaat tctgcctcag cctccccagc 120
aactgggact acaggtgcgt gccatcacgc ccagctactt tttgtattct cggcagagac 180
agggtttcac catgttggcc aggctggtct caaactccta ccctcaagtg atc 233

<210> 150
<211> 298
<212> DNA
<213> Homo sapiens

<400> 150
ttttttttt ttgaaaaagt catggaggcc atggggttgg cttgaaacca gctttggggg 60
gttcgattcc ttcctttttt gtctagattt tatgtatacg ggttcttcga atgtgtggta 120
gggtgggggg catccatata gtcactccag gtttatggag ggttcttcta ctattaggac 180
ttttcgcttc gaagcgaagg cttctcaaat catgaaaatt attaatatta ctgctgttag 240
agaaatgaat gagcctacag atgataggat gtttcatgtg gtgtatgcat cggggtag 298

<210> 151
<211> 285
<212> DNA
<213> Homo sapiens

<400> 151
ttttttttta gtacagaaga cagtttactg tacaagcaag ttgtgcgtta aaaacaaaca 60
ccaagcaaac gatagtgcaa agcagtttcc acccagctcc atcctctcgc cagctctggg 120
atggttttac atcagatgag tgcagcaggt gtcacacctc agcatgacaa tatgtcacaa 180
aagattggta cccactactg acaggctcac agtaacacta tatcaaaacg tcttcctttc 240
ctcgtgcttc ctacatcagt gtgtttgcct agtacaactt taaca 285

<210> 152
<211> 184
<212> DNA
<213> Homo sapiens

<400> 152
gatcccagca agataatgtc ctgtcttcta agatgtgcat caagcctggt acatactgaa 60
aaccctataa ggtcctggat aatttttgtt tgattattca ttgaagaaac atttattttc 120
caattgtgtg aagtttttga ctgttaataa aagaatctgt caaccatcaa aaaaaaaaaa 180
aaat 184

<210> 153
<211> 78
<212> DNA
<213> Homo sapiens

<400> 153
ttgtttttta ttttttagta aagatggggt ttcaccatgt tggacagtct ggtctcaaac 60
tcctgacctc aagtgatc 78

<210> 154
<211> 270
<212> DNA
<213> Homo sapiens

<400> 154
gatctaaaaa aattcagaag aaatatgatg aaaggaaaaa gaatgccaaa atcagcagtc 60
tcctggagga gcagttccag cagggcaagc ttcttgcgtg catcgcttca aggccgggac 120
agtgtggccg agcagatggc tatgtgctag agggcaaaga gttggagttc tatcttagga 180
aaatcaaggc ccgcaaaggc aaataaatcc ttgttttgtc ttcacccatg taataaaggt 240
gtttattgtt ttgttcccac aaaaaaaaaa 270

<210> 155
<211> 165
<212> DNA
<213> Homo sapiens

<400> 155
gatctgtgtt tgggaagaaa agaaggaaac taagacgtgc gaggggaaga aaaaggaaaa 60
cctaattaaa aaatatgtat cctctataat tagttatgac agccatttgt aatgaatttg 120
tcgcaaagac gtaataaaat taactggtag cacaaaaaaa aaaag 165

<210> 156
<211> 192
<212> DNA
<213> Homo sapiens

<400> 156
tttttttttt tttggctcta gagggggtag agggggtgct atagggtaaa tacgggccct 60
atttcaaaga tttttagggg aattaattct aggacgatgg gcatgaagct gtggtttgct 120
ccacagattt cagagcattg accgcagtat acccccggtc gtgtagcggt gaaagtggtt 180
tggtttagac gt 192

<210> 157
<211> 118
<212> DNA
<213> Homo sapiens

<400> 157
gatcatggtt catcaacgat tgagcaaggc ctcgctgctc tggatgctga aatgacccaa 60
aagttaatag acttgaagga caaacaacag cagcagctgc ttaatcttcg gcaagaac 118

<210> 158

<211> 258
<212> DNA
<213> Homo sapiens

<400> 158
tttttttgac taccgactga ttccaagtcc ccaggagggc tttattttt cttttcaaca 60
tcctgttctg cggcttcctt ggctctttt gcccgtatgc cgaagagccg ggcgttggca 120
cgggccatac ggagactagc gaaggctttg aaattcttct cttcctcaat gatgactcga 180
gctttctcct tcttatagac gttccggacg ggcatgaccg gtccggtcag ctgggtggcc 240
agtttcagtt cttcagca 258

<210> 159
<211> 113
<212> DNA
<213> Homo sapiens

<400> 159
tttttttttt ttaattaaag ggacagggcc tcactctgtt gctcaggtgg gagtgaggtg 60
gtatgactat agctcactgc agcctcaccc tcctctcctg ggcttctgtg atc 113

<210> 160
<211> 246
<212> DNA
<213> Homo sapiens

<400> 160
tttttttttt ttaattattt ctgtgttttt atttagtggg tagcagaagt tgttcagaag 60
agcagaaata tgtagaaaac atctctaaat ttttggcaat ttgaaatagc aattctgagg 120
cacacagctc atctacaaaa atcttttgca gaagcagcac gcttagtttt agggtgagga 180
aaatctgccg aaaaataaaa tgcagttggt tatcttatgc ataataagaa aaagaaaagg 240
atgatc 246

<210> 161
<211> 234
<212> DNA
<213> Homo sapiens

<400> 161
gatcacctga ggtcaggagt ttgagaccag cctggccaac atggcaaaac actgtctcta 60
ctaaaaatat aaaaattagc caggcatggt ggcggacacc tgtagtccca gctactcagg 120
agactgaggc aggagaatcg cttgaaccca ggaggtggag gttgcagtga gctgagactg 180
tgccactgca ctccagcctg ggtgatagag tgagactctg cctccaaaaa aaaa 234

<210> 162

<211> 276
<212> DNA
<213> Homo sapiens

<400> 162
gatcaagaac aatgcctcca ctgactatga cctatctgac aagagcatca accctctggg 60
tggctttgtc cactatggtg aagtgaccaa tgactttgtt atgctgaaag gctgtgtggt 120
gggaaccaag aagcgggtgc tcaccctccg caagtccttg ctggtgcaga cgaagcggcg 180
ggctctggag aagattgacc ttaagttcat tgacaccacc tccaagtttg gccatggccg 240
cttccagacc atggaggaga agaaagcatt catggg 276

<210> 163
<211> 116
<212> DNA
<213> Homo sapiens

<400> 163
tttttttttt gttttttgt ttttttgttt tttttttgct caggactatt tgctttcaga 60
gcacaaaaca ggttacagac aggtgtgtgc caggagtcgc aagatttggc tggatc 116

<210> 164
<211> 193
<212> DNA
<213> Homo sapiens

<400> 164
gatctcttat ttatactata ttattgtccc atgtactttc taaactgagc ttggaacatt 60
tagtattcct gcaattggac ttcccactta acaattatac agactttgct tttagaaata 120
gattaggttc caaacagaaa gttcaagtgt aacaacaaca ataaaaatag attatgaaac 180
aggcaaaaaa aaa 193

<210> 165
<211> 181
<212> DNA
<213> Homo sapiens

<400> 165
gatcaagctg tccgaaccta ccaagagcac aaagcaagca tgcatcccgt gactgcaatg 60
ctggtgggga aagacttaaa agtggattaa agacctgcgt attgatgatt ttagagattt 120
ctctttttta aatggaattc gtaaagaaat ataaaacttc agctcacaat taaaaaaaaa 180
a 181

<210> 166
<211> 163

<212> DNA
<213> Homo sapiens

<400> 166
tttttttttt ttattcggct cagtctaatc ctttttgtag tcactcatag gccagactta 60
gggctaggat gatgattaat aagagggatg acataactat tagtggcagg ttagttgttt 120
gtagggctca tggtaggggt aaaaggaggg caatttctag atc 163

<210> 167
<211> 134
<212> DNA
<213> Homo sapiens

<400> 167
tttttttttt ttacaatgtt aaattagtac aggtttactt ttaaatattt aataatataa 60
aagacttgca tagcttcaat tcatttaagc ttcccaataa tgggtaaatt ttaagtttcc 120
tgaaaagtga gatc 134

<210> 168
<211> 153
<212> DNA
<213> Homo sapiens

<400> 168
tttttttttt tttgatttaa aagattttat tttctttatg caggtaggca gttagaaatt 60
tcaaagtcta acaatgacat tcttgaagtg ggcacagcct ttttaaactca ggctatgtat 120
acagtaacct tgtggaactg gttcagccag atc 153

<210> 169
<211> 65
<212> DNA
<213> Homo sapiens

<400> 169
gatctttctt ctcccagcta agagttcttc aataaattta agaaatacct ggaaaaaaaa 60
aaaaa 65

<210> 170
<211> 179
<212> DNA
<213> Homo sapiens

<400> 170
tggttttttt taaataacaa acatttattg gggtcactta tggtagaaaa aacttcctac 60

accagatgca catgacccag ttgttaaata gaacattttg aaggtgaaca cacaccctaa 120
cccaggtttt ttacccgctt tttaagatgg ccaattcttc ttctcccccc cacccaaag 179

<210> 171
<211> 250
<212> DNA
<213> Homo sapiens

<400> 171
ttgggggcca gtggaaggag attagtgcaa tcattgggaa gaggaagaag atggaagctg 60
atggggttga agtcaaaaga ccaaaatact aatcactagt tacaaccaga gatgctccac 120
aaggatatgc tccccacggt tttctttcta caatttccaa aggttgcaag atgttttttt 180
gtggatgaat ataaaatttt attgtgtaat tacttggttc cattaaaatt ggttaacttg 240
caaaaaaaaa 250

<210> 172
<211> 392
<212> DNA
<213> Homo sapiens

<400> 172
tttttttttt gggtaccaaa tttctttatt tgaaggaatg gtacaaatca aagaacttaa 60
gtggatgttt tggtacaact tatagaaaag gtaaaggaaa ccccaacatg catgcactgc 120
cttggtgacc agggaagtca ccccacggct atggggaaat tagcccgagg cttagctttc 180
attatcactg tctcccaggg tgtgcttgtc aaagagatat tccgccaagc cagattcggg 240
cgctcccatc ttgcgcaagt tggtcacgtg gtcacccaat tctttgatgg ctttcacctg 300
ctcattcagg taatgtgtct caatgaagtc acacaaatgg gggtcatttt tgtcagtggc 360
cagtttgtgc agttccagta gtgactgatt ca 392

<210> 173
<211> 197
<212> DNA
<213> Homo sapiens

<400> 173
ttgatgagaa aaagggggaa gccaaagaga aaggtacctg ggttcaacta aagcgccagc 60
ctgctccacc cagagaagca cactttgtga gaaccaatgg gaaggagcct gagctgctgg 120
aacctactcc ctatgaattc atggcataat aggtggtaaa aaaaaataaa ggacctctgg 180
gctacaaaaa aaaaaaa 197

<210> 174
<211> 104
<212> DNA
<213> Homo sapiens

<400> 174
tttttggttt tgggtttttt tggttttttt ttttttaatt tttatagaga cagggtttca 60
ctatgttgcc caggctggtc ttgaactcat tggctcaagg gatc 104

<210> 175
<211> 182
<212> DNA
<213> Homo sapiens

<400> 175
gagccttctt ctttcactgg tcgtgcctcc cagcaggtgc agagattctt agaagaggag 60
gtgtatcccc tgttaaaacc atatgaaagc gtgatgaagg tgaaagcaga attatgtctg 120
tagagttgga agagaattaa acgaaaatca ttgttaattg ctgaggcatg aaaaaaaaaa 180
aa 182

<210> 176
<211> 116
<212> DNA
<213> Homo sapiens

<400> 176
gagccanttc tagactttgg gatatttttc ttcaattttg aagagaaaat ggtgaagcca 60
tacaaaagtt acccgaggga aaataaatac agtgatattc ttaagcaaaa aaaaaa 116

<210> 177
<211> 288
<212> DNA
<213> Homo sapiens

<400> 177
gatctagttc ccctggaaaa gctgctgtat ttttaatttt taatggaatg tagcttttaa 60
aatcctgtca ctggcatcaa caaaaggaat tataccatga gaccttatag ctgtacttaa 120
aagccattca gttcagctat tgggagttca tgatgaatta gcatatgcca gaaaggttgc 180
taaccttaac atctgagagc agtaacactg attttatctg ctgtataaga ctttgtgcat 240
tttactttga aataaagatt tttttccacg ctgaaaaaaa aaaaaaaa 288

<210> 178
<211> 78
<212> DNA
<213> Homo sapiens

<400> 178
gatctcaaca caattctgtg aggttttttt ttcttttttg cttttaggaa gaggctgaat 60

ttaagacaaa aaaaaaaa 78

<210> 179
<211> 249
<212> DNA
<213> Homo sapiens

<400> 179
tttttttttt tttttttttt ttttttttga gacagagttt tttgctcgtt gcccaggctg 60
gagtacaaag gggcaatctc ggttcaccac aacctccgcc ttttgggttc aagcaattct 120
cctgcctcag cctcctgagc agttgggatt acaggcatgt gccaccacgc ctggctaact 180
tttctgtatt ttcagtagag actgggtttc tccatggtgg tcaggctggt ctcaaactcc 240
cgacctcag 249

<210> 180
<211> 263
<212> DNA
<213> Homo sapiens

<400> 180
tttttgtttt atgagaaaaa gagactttat taggcacaga gggcgactgg taggcagtta 60
caaaatggct actgggcttc ctcccaagct ggagagtagt accccaggga aaggaagggc 120
agaggagcgt gtgctttctc tgccttctcc cttcaaggct gcatacgaat ggccccatcc 180
agccggatga cctctccatt gaggaatggg ttctcgatga tggcctgtac gaggtgagca 240
tactcagcag ggtcacccag tcg 263

<210> 181
<211> 85
<212> DNA
<213> Homo sapiens

<400> 181
gggggtgtac ccgctaaaac tgcacagctc gaccttcatg gcgacagctc cgcggaaaga 60
caaaagatga cgaaaagaaa aaaaa 85

<210> 182
<211> 160
<212> DNA
<213> Homo sapiens

<400> 182
gatcacttct gcttgctggg agtggggctg cctccctggc tagagcagca accaagacac 60
acagagaagc cctggaggct gggaggcttt ccctttgact ctaaggctct caaacccaga 120
gataaaatgc cagggctctt cgctcctcca aaaaaaaaaa 160

<210> 183
<211> 335
<212> DNA
<213> Homo sapiens

<400> 183
gatcttcttt ataattctac tttgagtgct gtctccatgt ttgatgtatc tgagcaggtt 60
gctccacagg tagctctagg agggctggca acttagaggt ggggagcaga gaattctctt 120
atccaacatc aacatcttgg tcagatttga actcttcaat ctcttgcact caaagcttgt 180
taagatagtt aagcgtgcat aagttaactt ccaatttaca tactctgctt agaatttggg 240
ggaaaattta gaaatataat tgacaggatt attggaaatt tgttataatg aatgaaacat 300
tttgtcatat aagattcata tttacttctt ataca 335

<210> 184
<211> 184
<212> DNA
<213> Homo sapiens

<400> 184
ttgttttttt ttgggaatgc aacaacttta ttgaaaggaa agtgcaatga aatttgttga 60
aaccttaaaa ggggaaactt aaacaccccc cctcaagcgc aggaccaagt gcagagtgga 120
ctctttctgg atgttgtagt cagacagggt gcgtccatct tccagctgtt tcccagcaaa 180
gatc 184

<210> 185
<211> 58
<212> DNA
<213> Homo sapiens

<400> 185
tttttgtttt tttttaaaat tttaatttgt ggtttatttc ataatcccaa actggatc 58

<210> 186
<211> 237
<212> DNA
<213> Homo sapiens

<400> 186
tttttttttt tctgagatgg agtttcgctg ttgttgccca ggctggagtg caatggtgca 60
atctcagctc atcgcaacct ctgcctccca ggttcaagcg attctcctgc ctcagcctcc 120
cgagtagctg ggattacagg catgtgccac cacatccagc taactttgta ttttgagtag 180
agatggggtt tctccatgtt ggtcaggctg gtctcgaact ccagaactca gatgatc 237

<210> 187
<211> 341
<212> DNA
<213> Homo sapiens

<400> 187
gatccgagga ggcggaacaa gtccacggag tccctgcagg ccaacgtgca gcggctgaag 60
gagtaccgct ccaaactcat cctcttcccc aggaagccct cggcccccaa gaagggagac 120
agttctgctg aagaactgaa actggccacc cagctgaccg gaccggtcat gcccgtccgg 180
aacgtctata agaaggagaa agctcgagtc atcactgagg aagagaagaa tttcaaagcc 240
ttcgctagtc tccgtatggc ccgtgccaac gcccggttct tcggcatacg ggcaaaaaga 300
gccaaggaag ccgcagaaca ggatgttgaa aagaaaaaat a 341

<210> 188
<211> 131
<212> DNA
<213> Homo sapiens

<400> 188
gatcaacttg gccaacctgg aagtaaatga aggaattcag gctctctcaa atagtgagga 60
ggagaagaaa ggggtggcag catcgctgct tgctccttta ttgcctgagg gaataaagga 120
aaggggaaa a 131

<210> 189
<211> 86
<212> DNA
<213> Homo sapiens

<400> 189
ttttttttt gtttgtttgt ttttagcgga gacagggttt tgtcatgttg gccaggctgg 60
tcttgaactc ctagcctcaa gtgatc 86

<210> 190
<211> 245
<212> DNA
<213> Homo sapiens

<400> 190
tttttttttt tcaaggttaa taaacagctt tatttgcctt ggacagcatc aattttctta 60
cattctcagt taattggcca ttaaagggct ggaaattttc ttaatcatga taacatttgt 120
taaaaagaaa tcagaactaa tatcaggaac atggcggcat gaaggaaaca gttcccttac 180
aaaacacaga aaatggaagt ccctcatgtt gagggggtgg gttggacaat ttgcaaacag 240
attct 245

<210> 191
<211> 119
<212> DNA
<213> Homo sapiens

<400> 191
tttttgtttt gggggaaggc gctttgtgaa gtaggcctta tttctcttgg cctttcgtac 60
agggaggaat ttgaagtaga tagaaaccga cctggattac tccggtctga actcagatc 119

<210> 192
<211> 63
<212> DNA
<213> Homo sapiens

<400> 192
tttttttttt tgaattttta gtagatacgg ggtttcaccg tgttagcctg gatggtctcg 60
atc 63

<210> 193
<211> 144
<212> DNA
<213> Homo sapiens

<400> 193
gaggcaggtg actctgagat cattagaagc atgccagaac agactggtga aaagtaaacc 60
ttttcaccta caaaatttca cctgcaaacc ttaaacctgc aaaattttcc tttaataaaa 120
tttgcttggt ttaaaaacaa aaaa 144

<210> 194
<211> 159
<212> DNA
<213> Homo sapiens

<400> 194
gatggtttct tgactctccc acatgctctg ttatgggaga gaatccccta ccctactctg 60
atgtatagac cattctccct tcaccagccg aaccaatgtc aaaattaata aagaaatgga 120
ctaatggcaa aaaaaaaaaa aaaaaaggaa aaaaaaaaa 159

<210> 195
<211> 124
<212> DNA
<213> Homo sapiens

<400> 195
tttttttttt tttagataaa cgctttatta ttttctcaaa atgttataaa agtcatttct 60
ccttagtaca gagagcttta tacattttat ctagtaatga atacaatcta gtttaacacc 120
gatc 124

<210> 196
<211> 248
<212> DNA
<213> Homo sapiens

<400> 196
gatgacttga gcggaggagt ttgagaccat cctgggcaac atagtgaaac tctctcacta 60
ccaaagtaca aaaggaatta gctgggcatg atggtacaca cttgtagtcc cagctacttg 120
ggggactgag atgggaaaat tgcttgagcc cgggagtcaa aggttgcagt aagccaagat 180
tgcaccacta cactccagcc tgggtgacaa agtgagaccc tgtctcaaaa aaaaacaaaa 240
aaaaaaaa 248

<210> 197
<211> 209
<212> DNA
<213> Homo sapiens

<400> 197
tttttttttt ttttaaaggt tataaagctt tattaaacat ttcaaacagc tgtgcaacga 60
acacaccaaa taaaagctct agaatagcag tccagacgtt tcacaagtat ggcctcacag 120
tcccattccc tagatggact gcctccagtt ctgttctctg cgctggccca tctctctttc 180
ccctcaggca agagagagat ggatggatc 209

<210> 198
<211> 89
<212> DNA
<213> Homo sapiens

<400> 198
gatctcccta gggccccacc tggaccattt tccctccgtt ttattttgtt aattaaattc 60
tttccaaatt ggaaaaaaaa aaaaaaaaa 89

<210> 199
<211> 237
<212> DNA
<213> Homo sapiens

<400> 199
gatcacttga ggccaggagt ttgagacaag cctggccaac atggtgaaac cccatctcta 60

ctaacagtac aaaaataagc caggtatagt ggcacacacc tttaatccca gctactcagg 120
aggttgaggt gggagaatca cttgaatctg ggaggtggag gtttcattga gccaagattg 180
caccgctgca ctccagccta ggtgacagag tgagaccttg tctcaaaaaa aaaaaaa 237

<210> 200
<211> 332
<212> DNA
<213> Homo sapiens

<400> 200
gatcttaccc gtgacaaaat gtgttccatg gtcaaaaaat ggcagacaat gattgaagct 60
cacgttgatg tcaagactac cgatggttac ttgcttcgtc tgttctgtgt tggttttact 120
aaaaaacgca acaatcagat acggaagacc tcttatgctc agcaccaaca ggtccgccaa 180
atccggaaga agatgatgga aatcatgacc cgagaggtgc agacaaatga cttgaaagaa 240
gtggtcaata aattgattcc agacagcatt ggaaaagaca tagaaaaggc ttgccaatct 300
atttatcctc tccatgatgt cttcgttaga aa 332

<210> 201
<211> 224
<212> DNA
<213> Homo sapiens

<400> 201
gatcttgttg aggcatttag ctgccatgca cctgtccccc tttaatactg ggcattttaa 60
agccatctca agaggcatct tctacatgtt ttgtacgcat taaaataatt tcaaagatat 120
ctgagaaaag ccgatatttg ccattcttcc tatatcctgg aatatatctt gcatcctgag 180
tttataataa taaataatat tctaccttgg aaaaaaaaaa aaag 224

<210> 202
<211> 171
<212> DNA
<213> Homo sapiens

<400> 202
tttttttttt ttgaagcaag gtctcactct attgcccagg ctggagtgtg atggcacaat 60
cacagctcac cgcacccttg acctcctgtg ctgaggcaat cctcccacct catcctcgcg 120
agtagggggg actacagatg ctcaccacca cacatggcta atttttggtt t 171

<210> 203
<211> 154
<212> DNA
<213> Homo sapiens

<400> 203

gagtgttaat ccagttcaca ctgtcttcat tactgcagat tttcatgggt tgggtaaggc 60
cagtcctctt tgttctttta aaaaattaag tatttttggt cctttgcatt tttatataaa 120
gtttgctttt gccacttcct acaaaaaaaa aaaa 154

<210> 204
<211> 321
<212> DNA
<213> Homo sapiens

<400> 204
gatctttgct ggcaagcagc tggaagatgg acgtactttg tctgactaca atattcaaaa 60
ggagtctact cttcatcttg tgttgagact tcgtggtggt gctaagaaaa ggaagaagaa 120
gtcttacacc actcccaaga agaataagca caagagaaag aaggttaagc tggctgtcct 180
ggaatattat aaggtggatg agaatggcaa aattagtcgc cttcgtcgag agtgcccttc 240
tgatgaatgt ggtgctgggg tgtttatggc aagtcacttt gacagacatt attgtggcaa 300
atgttgtctg acttactgtt t 321

<210> 205
<211> 190
<212> DNA
<213> Homo sapiens

<400> 205
gatcctgggt attgaaactg atatttctag attcttgtcc ttccctgaga gggaagtttg 60
gtcaacagaa actgatattt ctggacaata ttcctatcag gaaacttgta cctcaaaata 120
tgcccccttg tgcagatatt tttgtattta tttgtcaaaa ataaattgtg gaggaaatta 180
ataaaaaaaa 190

<210> 206
<211> 228
<212> DNA
<213> Homo sapiens

<400> 206
tttttggttt tttatgttag gctgatttat aagtgctgct ttgggcagtt acacagtttg 60
tttacaatga ggagttattt gactttgaac atactgtaca tggcaattag aagttgtcat 120
ggcaaaagaa aaccacagct ggcctgccac agccaacaca agaaccagaa aatggtagat 180
gaaatgaagg aataaaggtg gggtttattc cttattataa aagaaaaa 228

<210> 207
<211> 52
<212> DNA
<213> Homo sapiens

<400> 207
atttgacaat aatattaaat tccactcatt agttcctgga catctgttga tc 52

<210> 208
<211> 213
<212> DNA
<213> Homo sapiens

<400> 208
gatcaagaaa aataaggaca acgtgaagtt taaagttcga tgcagcagat acctttacac 60
cctggtcatc actgacaaag agaaggcaga gaaactgaag cagtccctgc cccccggttt 120
ggcagtgaag gaactgaaat gaaccagaca cactgattgg aactgtatta tattaaaata 180
ctaaaaatcc taaaaaagaa aaaaaaaaga atc 213

<210> 209
<211> 170
<212> DNA
<213> Homo sapiens

<400> 209
ctcggctcac agcaatctcc gcctcttgag ttcaagtgat tctcctgcct cagcctcctg 60
agcagctggg attataggca cctgccacca tgcctggcta atttttgtat ttttagtgga 120
gacagggttt tgtcacgttg gctgggctgg tctcctgacc tcaagtgatc 170

<210> 210
<211> 234
<212> DNA
<213> Homo sapiens

<400> 210
tttttttttt tgaggtggag tctcactgtg ttgcccaggc tggagtgcag tggcacagtc 60
tcagctcact gcaacctccg cctcccgggt tcaagtgatt ctcctgcctc agcctcctga 120
gtagctggga ctacaggcgc ccaccaccac atctggctaa tttttgtatt tttgtagaga 180
cagggataca ccatgttggc caggatggtc tcgaattcct gacttcaagt gatc 234

<210> 211
<211> 235
<212> DNA
<213> Homo sapiens

<400> 211
gatcagccac attttgacag tggagactca gaggcctagt taggaacaca gaaggaaaaa 60
tgttaaaggc agacctttat aaccaaaaca atcaacaggt ctaaaagcca aagaaaccag 120
tatttcagaa ttgttaactt actcctagat gtgtgttagg catcgggctt atcttaaaga 180

agtgtcttag accacagaaa catagaccca tgatatttgg accaagcata ggtta 235

<210> 212
<211> 62
<212> DNA
<213> Homo sapiens

<400> 212
tttttttttt ttgagaccga gtctcgctct gtcacccagg cttggagtgc aatggcatga 60
tc 62

<210> 213
<211> 66
<212> DNA
<213> Homo sapiens

<400> 213
gatcacgagg tcaggacttc gagactagcc tggccagcat agtgaaaccc catctctcca 60
aaaaaa 66

<210> 214
<211> 184
<212> DNA
<213> Homo sapiens

<400> 214
gatcgggcta ctacaaagtt ctgggaaagg gaaagctccc aaagcagcct gtcatcgtga 60
aggccaaatt cttcagcaga agagctgagg agaagattaa gagtgttggg ggggcctgtg 120
tcctggtggc ttgaagccac atggagggag tttcattaaa tgctaactac ttattcaaag 180
aaaa 184

<210> 215
<211> 228
<212> DNA
<213> Homo sapiens

<400> 215
gatccccagc tgtttatgca tagataatct ctccattccc gtggaacgtc tttcctgttc 60
ttaagacgtg attttgctgt agaagatggc acttataacc aaagcccaaa gtggtataga 120
aatgctggtt tttcagtttt caggagtggg ttgatttcag cacctacagt gtatagtctt 180
gtattaagtt gttaataaaa gtacatgtta aacttaaaaa aaaaaaaa 228

<210> 216

<211> 76
<212> DNA
<213> Homo sapiens

<400> 216
gatctcgcca ctgcactcca gtctggcgac atagcgagac tccatctcac aaaaaaaaaa 60
aagaaaaaaa aaaaaa 76

<210> 217
<211> 87
<212> DNA
<213> Homo sapiens

<400> 217
gatcatgtca gtcccttctc agaaccttca atggctcccc actacccttg gaataaaatc 60
caaacacttc cacaaaaaaa aaaaaaa 87

<210> 218
<211> 176
<212> DNA
<213> Homo sapiens

<400> 218
gatctcatca gaatgtgctg gtttcagtct ggagatagat ttctgaggag actaatacaa 60
gggagagaat gtccaaagaa cactactgtg gaaagttctt acgatggaaa ttgacatctt 120
ttgtatctca ttctccagcc gtgaaataaa ggcagaggtt ccctgtaaaa aaaaaa 176

<210> 219
<211> 171
<212> DNA
<213> Homo sapiens

<400> 219
gatctgttca aacagcaaac gcccacagat ggcccagagg tggtggtagt cagggtgtgt 60
gggtgttttt agggttcttt agtgttgntt ctttcaccca ggggtggtgg tcccagccag 120
tttggtgctg acggtgagag gaaattagaa tctgtttgca aattgtccaa c 171

<210> 220
<211> 255
<212> DNA
<213> Homo sapiens

<400> 220
gatgaaacta gaactcatat gccatactag atatgtttgt caataaactt atgacgtgaa 60

tgcttaatgc ctctttttg aaatagggaa tgtaataatt ggccatttgc ctactttatt 120
atttgggtaa cattccagta ttactctctg tgatttagct tatttaatgg tgttaaactg 180
aggttatatt aaatttttga ttcccaggtc aggattttgt tggtaattta tataataaaa 240
gggaaataca aaaaa 255

<210> 221
<211> 345
<212> DNA
<213> Homo sapiens

<400> 221
gatcaacctg gagctctacg cctcctacgt ttacctgtcc atgtcttact actttgaccg 60
cgatgatgtg gctttgaaga actttgccaa atactttctt caccaatctc atgaggagag 120
ggaacatgct gagaaactga tgaagctgca gaaccaacga ggtggccgaa tcttccttca 180
ggatatcaag aaaccagact gtgatgactg ggagagcggg ctgaatgcaa tggagtgtgc 240
attacatttg gaaaaaaatg tgaatcagtc actactggaa ctgcacaaac tggccactga 300
caaaaatgac ccccatttgt gtgacttcat tgagacacat tacct 345

<210> 222
<211> 146
<212> DNA
<213> Homo sapiens

<400> 222
tttttttttt tttaaggaga tagtattctt gctagggttt aaatggattt tgcttgctat 60
tcaaatcaca atacagaaat ttactttagg ccaaatattc ttcactggct ttgagacact 120
tctctgtgtt ctgaaaacca ttgatc 146

<210> 223
<211> 84
<212> DNA
<213> Homo sapiens

<400> 223
tttttttttt ttttgaattg ttggtagaga cagggtctct ttatgttgcc caggctggtc 60
ttgaattcct ggcctcaagt gatc 84

<210> 224
<211> 345
<212> DNA
<213> Homo sapiens

<400> 224
gatcttaggc aaaataccag ctgatgaagg catctgatgc cttcatctgt tcagtcatct 60

ccaaaaacag taaaaataac cactttttgt tgggcaatat gaaattttta aaggagtaga 120
ataccaaatg atagaaacag actgcctgaa ttgagaattt tgatttctta aagtgtgttt 180
ctttctaaat tgctgttcct taatttgatt aatttaattc atgtattatg attaaatctg 240
aggcagatga gcttacaagt attgaaataa ttactaatta atcacaaatg tgaagttatg 300
catgatgtaa aaaatacaaa cattctaatt aaaagctttg caaca 345

<210> 225
<211> 205
<212> DNA
<213> Homo sapiens

<400> 225
gatccttgtg atgatggaaa tgttctatat cacagctatc gatgtcagta tcccagttgt 60
gatattgtgc tatagttttg caagatgtta ccactggggg aaattgggtg aagggcacat 120
gggaatctct gtattatttc aactgcatgt aaatctacaa ttatctcaaa ataaaaataa 180
aagttaaaaa aacacaaaaa aaaaa 205

<210> 226
<211> 110
<212> DNA
<213> Homo sapiens

<400> 226
gaggatttca agaaacctct gtttactgtg tggcacccag gcaaaacatg ctccacaaat 60
tcaacttgta tatttggcag attaaacttg acattatcgt aaaaaaaaaa 110

<210> 227
<211> 234
<212> DNA
<213> Homo sapiens

<400> 227
gatcacaagg tcaggaaatt gagaccatcc tggctaatac agtgaaaccc catgtctgct 60
aaaattacaa aaaattagct gggcgtggtg gtgcacgctt gtagtctcag ctactcagga 120
ggctgaggca ggagaatcgc ttgaacccgg gaggcagagg tagtagtgag ctgaagtccc 180
gccactgcac tccagcctgg gtgacacagt aagactccat ctcaaaaaaa aaaa 234

<210> 228
<211> 68
<212> DNA
<213> Homo sapiens

<400> 228
gatccagctg tgcttaagag ccaataatgt cttaataaac atgtggcagc ttttgtttga 60

aaaaaaaa 68

<210> 229
<211> 99
<212> DNA
<213> Homo sapiens

<400> 229
gatcaggtga gctgtgtcca gaaaaccaac gagaaggagt ggaaggagga atgaacgttt 60
cattctcgtt aataaaggca ttatcctaat caaaaaaaa 99

<210> 230
<211> 72
<212> DNA
<213> Homo sapiens

<400> 230
gatcacacca ctgcactcca gtctggtgac agagcgaggc tccatctcaa aaacagaaca 60
aaaaaaaaaa aa 72

<210> 231
<211> 158
<212> DNA
<213> Homo sapiens

<400> 231
ttttttttgg tgggggaagc acaagcttta ttggctgaaa gttcttctca ggagcctggt 60
ttgctgggac tgcatgttcc tggatgggct cccccaggcc taagctccag gtttcctctg 120
gccttccgaa ggattttgtg ggttacgacc aattgatc 158

<210> 232
<211> 194
<212> DNA
<213> Homo sapiens

<400> 232
tttttttttt ttccctgacg gactgggaag tcatcttttt tgaaggcatt tggccagagc 60
gagaggcatc caggcccctg agaaacaggg gaggcagagc cagagggagg agagagtaga 120
ggccagagcc caggcaggat acagcaccgt gccaccgcca caggcataag gggtggggtt 180
cctaaagggt ggct 194

<210> 233
<211> 115

<212> DNA
<213> Homo sapiens

<400> 233
tttttgtttt ttcttacact aatttataca ttttaattgg ttgcatatat taacatgtac 60
tgtaagattc ttttctaaga agcattacat aataaatgga tactgtaaaa agatc 115

<210> 234
<211> 114
<212> DNA
<213> Homo sapiens

<400> 234
gatcgcttga acccaggagg ttgaggttgc agtgagccaa gatggtacca gcctaggtga 60
caaagtgaca ccctgtctca aaaaagaaac caaacaaaca taaaaaaaaa aaaa 114

<210> 235
<211> 70
<212> DNA
<213> Homo sapiens

<400> 235
ttttttttt tggagacagg gtttagccat gttgcccagg ctggtctcga actcctgggc 60
tcaggcgatc 70

<210> 236
<211> 233
<212> DNA
<213> Homo sapiens

<400> 236
ttttttttt ttgaggtttt attattttta tttatttttg agactgagtc ttgctccgtc 60
atcaggctgg agtgcagtgg ctcactgcaa cctccgcctc ccaggttcaa gcaattctcc 120
tgcctcagcc tccctagtag ctgggattac aggtgtccac caccatgccc aattaatttt 180
tgtatttttg gtacagacag ggtttcacca tgttggccag gatggtctcg atc 233

<210> 237
<211> 279
<212> DNA
<213> Homo sapiens

<400> 237
ttttttttt ttaacagccg cttaatgttt tatcctagca ggaaccctac catgagccat 60
ttaaagtgtt tcaaatctta gggggctgag cctggtggct cacacctgta atctcagcac 120

tttgggaggc tgaggcagga ggattgcttg aggttgggag ttcaagacca gtctgggcaa 180
catggcaaaa actcatttct taaaaaaaaa aaaaaattag acctgtagtc ccagctactc 240
ggcaggctgt gtgaatagta aggggtcagt ctggttggg 279

<210> 238
<211> 155
<212> DNA
<213> Homo sapiens

<400> 238
ttttgttttt tggcgtaacg agactttact gaaaacagaa aaccgggcga accaaggatt 60
acaaacagga atgtggactc gtagcaaaac aaaacaaaac agaacaaaaa agggaggggg 120
gaccggtcga aagaaccgaa ggggggagcg agatc 155

<210> 239
<211> 224
<212> DNA
<213> Homo sapiens

<400> 239
ttttttctgg aaggttctca ggtctttatt tgctctctca acttctagga attgacttat 60
ttaattaatc catcaacgct tcatagcaaa tatttgagaa cacaaattta tattcaggtt 120
cttaacttca ttagggaagt aagaagttgc agctcagtgc accatgaatt tgagacagag 180
atggagacat ccagccccac ctctctggaa caggaaagat gatc 224

<210> 240
<211> 204
<212> DNA
<213> Homo sapiens

<400> 240
tttttttttt ggggggaagt gagctcttta tttagacata gctctgctga gtggaaagtg 60
ggcaccagcc ccattaatgc ttgctggctg gtggcttcca agcacgcccc actgccaagg 120
ctcagctctg cggttctgcc actgcaggtc cctagcacag cccccagggt ccttaaaagt 180
catcatcatc acagatgtca aggt 204

<210> 241
<211> 160
<212> DNA
<213> Homo sapiens

<400> 241
gatccacttc cagaagccca ccccccccgcc aaggttctca cactggcctg ggcttgggtg 60
cccatatagg aggtctgtat gttcaccaac agtgcggagg ggtcacacat tgcaaaacac 120

tgcccagaac agtaaaaaga gcctgcatgc caaaaaaaaa 160

<210> 242
<211> 277
<212> DNA
<213> Homo sapiens

<400> 242
gatcattgat gcaggtgact aaacagattg tcagtgtaga ggaaacagcc ttccattgga 60
agaaggtgcc gtctaggact ttcataacta gagagaagac aacatctgct ttgaaaggac 120
atgctaactc tcattagtgg ataatgctgc tggtcacttt taagtggaag ctagtgctca 180
tttatcattc tgataatcct aggaccctta gaatttgctg aatctactct gcctgtgctt 240
tataaatgga acaacaaagc ctggatgaca gcatgtc 277

<210> 243
<211> 135
<212> DNA
<213> Homo sapiens

<400> 243
gatcacgggg tgggagggaa ccgtggcgtc cctgcgtggg gcccatgggt gagacactcc 60
agtactgaga cctagagtcc agatgcttgt aggagccaag tcgtgttcta agtatttatt 120
taaaacaaaa aaaaa 135

<210> 244
<211> 308
<212> DNA
<213> Homo sapiens

<400> 244
tttttttttt tagatgtgta aaaaggcttt atttgcaggg gagcaggaat ttaatcaaaa 60
aggccaaatc ccatgtcatc atctgactct tcagactcct ccttcttctc atctttcttc 120
tcctctgctg cagcaggggc agaaccagca gcaggggctg cagagcctgg ggcagcagag 180
acggctacag ccccaccagc aggtacactg gcaagcttgc caataccctg ggcaatgacg 240
tcttcaatgt tttttccatt cagctcactg ataaccttgt tgagccggtc gtcgtccgcc 300
tcgatacc 308

<210> 245
<211> 223
<212> DNA
<213> Homo sapiens

<400> 245
ttggtttttg ttagttcatg tcttttatta actcatacag ttacttgtct tctggtttgt 60

tgaaacagta agtcagacaa catttgccac aataatgtct gtcaaagtga cttgccataa 120
acaccccagc accacattca tcagaagggc actctcgacg aaggcgacta attttgccat 180
tctcatccac cttataatat ttcaggacag ccagcttaac ctt 223

<210> 246
<211> 74
<212> DNA
<213> Homo sapiens

<400> 246
ttttgttttt tttgtttttg agacggagtt ctatgttatc caggctggtg tcgaactcct 60
gggctcaagc gatc 74

<210> 247
<211> 237
<212> DNA
<213> Homo sapiens

<400> 247
gatcacctga ggtcaggagt tcgagaccag cctagccaac atggtgaaac cctgtctcta 60
ctaaaaatac aaaaaagtag ccaggcgtgg tggtgcgcac ctgtactccc agctactcgg 120
gaggctgagg caggagactc gcctgggcct gggaggcaga ggttgcagtg agccaagatg 180
atgctgctgt actccagcct gggcaacaga gcaaaactcc ttctcaaaaa aaaaaaa 237

<210> 248
<211> 322
<212> DNA
<213> Homo sapiens

<400> 248
gatctggctc ccataggtat ccgggtgatg accattgccc caggtctgtt tggcacccca 60
ctgctgacca gcctcccaga gaaagtgtgc aacttcttgg ccagccaagt gcccttccct 120
agccgactgg gtgaccctgc tgagtatgct cacctcgtac aggccatcat cgagaaccca 180
ttcctcaatg gagaggtcat ccggctggat ggggccattc gtatgcagcc ttgaagggag 240
aaggcagaga aaacacacgc tcctctgccc ttcctttccc tggggtacta ctctccagct 300
tgggaggaag cccagtagcc at 322

<210> 249
<211> 170
<212> DNA
<213> Homo sapiens

<400> 249
ggcgtgatcg gcccagctct gactgaaggg gcttggcttc cactcagcat cagcgtggca 60

gtcaccaccc cagtgaggac cttgatgtcc agctgctgtc aggtctgata gtcctctgct 120
aaaacaacac gatttacata aaaaatctta cacatctgcc aaaaaaaaaa 170

<210> 250
<211> 230
<212> DNA
<213> Homo sapiens

<400> 250
tttttttttg ggacggagtc tccctctgtc acccaggctg gagtgcagtg gcgcaatctc 60
agctcactgc aacctctgcc tcctgagctc aagagattct cttgcctcag cctcccgagt 120
aactgggatt acaggtgccc accaccactc ccggctaatt tttgtaattt tttttagtag 180
agatggggtt tcaccacgtt ggccaggctg gtctcaaact cctgacctca 230

<210> 251
<211> 315
<212> DNA
<213> Homo sapiens

<400> 251
gatcccatgg gttgtgtgtt tatttcactt gatgatacaa tgaacactta taagtgaagt 60
gatactatcc agttactgcc ggtttgaaaa tatgcctgca atctgagcca gtgctttaat 120
ggcatgtcag acagaacttg aatgtgtcag gtgaccctga tgaaaacata gcatctcagg 180
agatttcatg cctggtgctt ccaaatattg ttgacaactg tgactgtacc caaatggaaa 240
gtaactcatt tgttaaaatt atcaatatct aatatatatg aataaagtgt aagttcacaa 300
ctaaaaaaaa aaaaa 315

<210> 252
<211> 140
<212> DNA
<213> Homo sapiens

<400> 252
ttttcaagat gcctgaggaa gtgcaccatg gagaggagga ggtggagact tttgcctttc 60
aggcagaaat tgcccaactc atgtccctca tcatcaatac cttctattcc aacaaggaga 120
ttttccttcg ggagttgatc 140

<210> 253
<211> 172
<212> DNA
<213> Homo sapiens

<400> 253
tttttttttt tgatttaatt cttcagctaa aacagcggaa gaggtgattt attatatggt 60

tgttacactc ggccacaaat aaacacagaa atagtccaga atgtcacagg tccagggcag 120
aggaccaaca tgggcatttt gtttatgagc aaggtgggtc tcagaggtga tc 172

<210> 254
<211> 272
<212> DNA
<213> Homo sapiens

<400> 254
gatcttcccc cgaccatgct gggaatccag aaacagggac cccatttgtc ttcccatatc 60
tggtggaggt gagggggctc ctcaaaaggg aactgagagg ctgctcttag ggagggcaaa 120
ggttcggggg cagccagtgt ctcccatcag tgcctttttt aataaaagct ctttcatcta 180
tagtttggcc accatacagt ggcctcaaag caaccatggc ctacttaaaa accaaaccaa 240
aaataaagag tttagttgag gaaaaaaaaa aa 272

<210> 255
<211> 172
<212> DNA
<213> Homo sapiens

<400> 255
gatctcagtt tcctggcttt tcctccctca gccccttctc accccttgc tgtcctgtgt 60
agtgatttgg tgagaaatcg ttgctgcacc cttcccccag caccatttat gagtctcaag 120
ttttattatt gcaataaaag tgctttatgc cggcttttct caaaaaaaaa aa 172

<210> 256
<211> 350
<212> DNA
<213> Homo sapiens

<400> 256
gatcccccct cttgagggcg atgaggatgc gtctcgcatg gaagaagtcg attaggttag 60
gagttcatag ttggaaaact tgtgcccttg tatagtgtcc ccatgggctc ccactgcagc 120
ctcgagtgcc cctgtcccac ctggctcccc ctgctggtgt ctagtgtttt tttccctctc 180
ctgtccttgt gttgaaggca gtaaactaag ggtgtcaagc cccattccct ctctactctt 240
gacagcagga ttggatgttg tgtattgtgg tttattttat tttcttcatt ttgttctgaa 300
attaaagtat gcaaaataaa gaatatgccg tttttataca aaaaaaaaaa 350

<210> 257
<211> 163
<212> DNA
<213> Homo sapiens

<400> 257

gatctattac atgataggat ggaaatatca tgagtcacag gcaagaccag ctgaaagagg 60
ttccgcaact gtgtcatttg gagagctagg aaaaataaac aaccttatgc caccggggaa 120
aaaatcacaa taaatattta ttcagtgtta aaaaaaaaaa aaa 163

<210> 258
<211> 217
<212> DNA
<213> Homo sapiens

<400> 258
tttctttttt tttgtttttt tgtttgtttt tgttttttgt tttttttttg gaacagtctg 60
gtccctgatg gggcctctc cccctgcccc tccccagtct ggttacagct cagttcgtcg 120
ctctattttg agcagctcca cctcgaacac cagggttgca ccgcctggaa tctttggggg 180
agctccccgc tctccatacc ctagctcgga tgggatc 217

<210> 259
<211> 54
<212> DNA
<213> Homo sapiens

<400> 259
gatcctaaat catgacttac ctgctaataa aaactcattg gaaaaaaaaa aaaa 54

<210> 260
<211> 156
<212> DNA
<213> Homo sapiens

<400> 260
gatccgtgat gccacttacc tgtgtgtttg gtaacaacaa accaacatca tggaggtccc 60
tggattgaaa aaggagcctc tcccactcct cctaccaccg aagtggttag gactctatat 120
aaataaaaac aaggcttttg gaaaataaaa aaaaaa 156

<210> 261
<211> 321
<212> DNA
<213> Homo sapiens

<400> 261
gatcattgac ctctgtgctt tcattcctag agatgtttta tagttacatg agcaaaagct 60
gttgccccaa agtgatggcc ctggaggcgg ggctgaggaa cagggaaatg ccgctgtgaa 120
gtcttaaagc acttctgctt aaactccatg tgtgaggagt gtgcctccct gtgccctctc 180
agctctgagg ctggccgtct ttcggggtgt tccttttggc aaatatacac tgtaatcttg 240
agtctaaatt tatatgttga aatgctacct tttttaaaat aagaaactaa ataaaattat 300

tttactatca aaaaaaaaaa a 321

<210> 262
<211> 266
<212> DNA
<213> Homo sapiens

<400> 262
tttttttttt ttgactatta caaaagttta tttaacaaaa agtctaatat gaaaatgtac 60
atgacctaat ttttacatca tagtaaaaca ggccctatgg agagaggaca tgagtttctc 120
tgctgaacag ccattattta tactcgttcc aaggcttcta acatgatgat actatttcct 180
cgtattacca ccattccaat attgttctgt tgtccactag tcgccatctc cacacattca 240
tctatcacaa ggttcataaa gggatc 266

<210> 263
<211> 50
<212> DNA
<213> Homo sapiens

<400> 263
tttttttttt ttaaagtagt aaaatgctga taggcagtaa cacggggatc 50

<210> 264
<211> 74
<212> DNA
<213> Homo sapiens

<400> 264
tttttttttt tttttttttt tttttttttg cttcactgct ttatttttga aatcacaagc 60
aattcaaagt gatc 74

<210> 265
<211> 360
<212> DNA
<213> Homo sapiens

<400> 265
gatcaagcct ttctttcatt ccctctctga aaagtattcc aacgtgatat tccttgaagt 60
agatgtggat gactgtcagg atgttgcttc agagtgtgaa gtcaaatgca tgccaacatt 120
ccagtttttt aagaagggac aaaaggtggg tgaattttct ggagccaata aggaaaagct 180
tgaagccacc attaatgaat tagtctaatc atgttttctg aaaacataac cagccattgg 240
ctatttaaaa cttgtaattt ttttaattta caaaaatata aaatatgaag acataaaccc 300
agttgccatc tgcgtgacaa taaaacatta atgctaacac ttttttaaaa aaaaaaaaaa 360

<210> 266
<211> 73
<212> DNA
<213> Homo sapiens

<400> 266
gatcatccta cctggcaaat aaattcccgt ttctatccaa aagagcaata aaaagttttc 60
agtgaaaaaa aaa 73

<210> 267
<211> 310
<212> DNA
<213> Homo sapiens

<400> 267
tttttttttt ttagtagaat ctttttttatt cagaaaaaaa aaaccccaaa aaacaaaagt 60
tttccaacca cacacgggag ggatatgggt agggggaggt gtctgtccat ccagccctgg 120
cccccagccc atgtggtttt ggcagcaata aggggtatgg ggtaatggcc caaaaaataa 180
aatggtttgt gtgtgtatgg ggaggaaagg ggtgcaaagc tgtggggagc ggtgaagggg 240
aagggacaga cgaggtcagt actgggaacg ccgaaggtgg gaggccattt cataacattt 300
cttgttgatc 310

<210> 268
<211> 333
<212> DNA
<213> Homo sapiens

<400> 268
gatcaagttt aaatgactgt gctgcccctt tcacatcaaa gaactactga caacgaaggc 60
cgcgcctgcc tttcccatct gtctatctat ctggctggca gggaaggaaa gaacttgcat 120
gttggtgaag gaagaagtgg ggtggaagaa gtggggtggg acgacagtga aatctagagt 180
aaaaccaagc tggcccaagg tgtcctgcag gctgtaatgc agtttaatca gagtgccatt 240
tttttttttg ttcaaatgat tttaattatt ggaatgcaca attttttttaa tatgcaaata 300
aaaagtttaa aaacttaaaa aaaaaaaaaa aaa 333

<210> 269
<211> 66
<212> DNA
<213> Homo sapiens

<400> 269
gatctcaagt gcttaatgat aaggtgttga cttgttaaat taaaccattt ggaatagaaa 60
aaaaaa 66

<210> 270
<211> 96
<212> DNA
<213> Homo sapiens

<400> 270
gatcagaggg tagtacagaa gccctgactc atgccttgag tacataccat acagcaaata 60
aatggtagca aaacattcta aaaaaaaaaa aaagaa 96

<210> 271
<211> 136
<212> DNA
<213> Homo sapiens

<400> 271
tttttttttt gcagaaaaat aaaaatttaa tataattaaa agcaatttat atatcatcat 60
gaaataaaga gagatttgaa actatgtagt caacagagga caaaaaaaaa tctctcaagc 120
aagagaattt ctgatc 136

<210> 272
<211> 149
<212> DNA
<213> Homo sapiens

<400> 272
gatcagaaat aagcccacaa ttagtcagaa gagctagtac atgtccagtt gagaaattga 60
ccttacttac taaggtcgtt actgaattta tccattaact catcaaatac caagcagtct 120
tcaaagccct gaaacaagaa aaaaaaaaa 149

<210> 273
<211> 225
<212> DNA
<213> Homo sapiens

<400> 273
tttttttttt ttgtgattga gataataaac ttttatttat tgaagagtaa aatagtacat 60
taacaaaatc ttatctgata gagagataat accagaagtc ttcttactgt gattatcagc 120
taagtgactg gttgaaatca ggatataaat cactggatag tttttttctg gaaagactta 180
gaacacatca ataaggccct ctaataaaga aattgaaatg agatc 225

<210> 274
<211> 43
<212> DNA

<213> Homo sapiens

<400> 274
gatctcacca aagacgacat cctagagatg gagaggagaa aaa 43

<210> 275
<211> 178
<212> DNA
<213> Homo sapiens

<400> 275
tttttttttt tttgaaggaa aatttgtatt atttcaatta tttttatgta cagaaaactc 60
aacagtgtac atttaaccca gtttagtggc aagttcttta gcctttgcct tttcgagctt 120
ggcgatacga gccacagact taggacccag gacattgcca ccccagtgac ggcggatc 178

<210> 276
<211> 76
<212> DNA
<213> Homo sapiens

<400> 276
gatcaagcag ttatttgatt tgtgctcact tttgatatgg ccaataaaac cataccgact 60
gaaaaaaaaa aaaaag 76

<210> 277
<211> 66
<212> DNA
<213> Homo sapiens

<400> 277
gatcgggacc agagactcaa gatagcctca aatacagtga aagattatgt atccaaaaaa 60
aaaaaa 66

<210> 278
<211> 65
<212> DNA
<213> Homo sapiens

<400> 278
gatcttaaac ataggaaaac catactgttc tgataataaa atgctttcta taaaaaaaaa 60
aaaaa 65

<210> 279

<211> 125
<212> DNA
<213> Homo sapiens

<400> 279
catgaaatca ttccatggat ggctgcctta taattttgtc tctttccact ttaattgtga 60
atggttaaaa aaatgctgct ttctgatatt aaatttttat tagtgcatac cttaaaaaaa 120
aaaaa 125

<210> 280
<211> 125
<212> DNA
<213> Homo sapiens

<400> 280
ttgttttttt ttacaaagaa tcaactttat tgaacattca gggtcagttt ctcttcttgc 60
tcttgcctgt gaccttggct ggtgtgagga ctggagctgc tgcctggtac agggtggagg 120
agatc 125

<210> 281
<211> 69
<212> DNA
<213> Homo sapiens

<400> 281
tgtgtttttt taatatgcca caatttttat tgcaacgtgg ccatttttgt gagggtgagg 60
agtttgatc 69

<210> 282
<211> 295
<212> DNA
<213> Homo sapiens

<400> 282
ggcgtgaggg ggttctaaaa gaaagggtgg agagattcaa ccagttagcg tgaaagttgg 60
agataaagtt cttctcccag aatatggagg caccaaagta gttctagatg acaaggatta 120
tttcctattt agagatggtg acattcttgg aaagtacgta gactgaaata agtcactatt 180
gaaatggcat caacatgatg ctgcccattc cactggagtt ctgaaatctt tcgtcatgta 240
aataatttcc atatttctct tttataataa actaatgata actaatgaca aaaaa 295

<210> 283
<211> 60
<212> DNA
<213> Homo sapiens

<400> 283
tttttttttt agagacaggg tcttgctctg tctcccagcc tagactgcag tggtacgatc 60

<210> 284
<211> 126
<212> DNA
<213> Homo sapiens

<400> 284
gatctccccc atgcctggcc taccacccta atgggtccgg aattggtggg ttcttggtct 60
tgctgacttc aagaatgaag ccgtggaccc tcacggtgag tgttacaatt cttaaaaaaa 120
aaaaaa 126

<210> 285
<211> 166
<212> DNA
<213> Homo sapiens

<400> 285
tttttttttt ttgtgtctca cgcttcattt aatctgaaga atattacagg ctctccgtct 60
tcagatataa attatttata acgttacaga acaagcagcg aattcaacca cttaagaact 120
aaggaagtct acatggtgtt aattgctggc cacagtcctc ccgatc 166

<210> 286
<211> 313
<212> DNA
<213> Homo sapiens

<400> 286
gatctttaaa tttttgtcct tagttaagtt ctgtatttag tgaattaatt gcatcctaaa 60
aagtcaaact tgaaaagcac attttaaatg gcaaatctat tttttacatg tttgtgaagt 120
ttttattttt tagtaaacag accatcagaa gagaacaatg gtacagagca gaggtcagca 180
gatggatttt gtaaagcatc aacttgtaaa tattttcaag tttattagct gtatggctct 240
ggtttctgtt ccctgttcca aatgttaaag tctactgttg tattctaaaa gcagccatgg 300
actgaatgta gct 313

<210> 287
<211> 240
<212> DNA
<213> Homo sapiens

<400> 287
tttttttttt tttttctttt taagacggag tctcactcta ttgccaggct ggagtgcaat 60

ggcgcaatct tggctcactg caaactccac ctcccgggct taaatggttc tcctgcctca 120
gcctcccgag tagctgggat tacaagcatg cgccaccacg ccaggctaat ttttgtatat 180
ttagtagaga cggggtttca ccatgttggt caggctcgtc ttgaactcct gacctcagat 240

<210> 288
<211> 100
<212> DNA
<213> Homo sapiens

<400> 288
cttttttttt tttcagtaga aaccattgaa gtttattggc ggtcacaatg cttacactgt 60
atgcagcaaa cacccttaca agtctatccg caacctgatc 100

<210> 289
<211> 131
<212> DNA
<213> Homo sapiens

<400> 289
gatcctttat ctttccatat ggcacacgta agaaagtgtt tttctactat taatattaaa 60
ttaaaacctt tacttttgta taataaatta aaactcagaa taaacctgtg accacgtaaa 120
aaaaaaaaaa a 131

<210> 290
<211> 102
<212> DNA
<213> Homo sapiens

<400> 290
tttttttttt ttaaagggag ggtctcactc tgtcacccag gctcaagtgc agtgacacaa 60
tcatggctca ctgcagcctt gaactcctgg gctcaagtga tc 102

<210> 291
<211> 267
<212> DNA
<213> Homo sapiens

<400> 291
ttttttgctg cgtctactgc gagaatgagg actattctca gcaatcagac tgtcgacatt 60
ccagaaaatg tcgacattac tctgaaggga cgcacagtta tcgtgaaggg ccccagagga 120
accctgcgga gggacttcaa tcacatcaat gtagaactca gccttcttgg aaagaaaaaa 180
aagaggctcc gggttgacaa atggtggggt aacagaaagg aactggctac cgttcggact 240
atttgtagtc atgtacagaa catgatc 267

<210> 292
<211> 238
<212> DNA
<213> Homo sapiens

<400> 292
tttttttttt ttaagtttaa aattttaata tttataaatt taaaaaattt tacacgtgct 60
gagtggtagc agtgctaaca tttttgtgac cattaaacca caaactcact gcatagagct 120
tctttctttg tacagataat caaatcacaa taccaacatt tgcaatttgt aataatttta 180
tgtggtaaaa gacaccacac caggaagcct gggctcagaa aacacttgag gtcagatc 238

<210> 293
<211> 197
<212> DNA
<213> Homo sapiens

<400> 293
gatcgggaat acctgccaat tcctctgaca aggacagggt tgcggctgca atggggcttc 60
cccttcttgg gctttttagc tttgaggtta cccttttttca ccttgccacc agcatcaacc 120
ttcttggctt cgggtttctt ctctttagta tctggcttct caactttttc acccgccatc 180
ttgcaagatg ggaaaaa 197

<210> 294
<211> 221
<212> DNA
<213> Homo sapiens

<400> 294
gggttttttt ttttttttgt ttaaacagat tcttttaata caatgaatac aaaagcagtt 60
gagatagtac ccccacgatt acacagcttt gaacaagtga aactatggtt accaaacagg 120
tatttttatt atagcatcta cagtgtctgg aaaaggatgt aacaataaat aactgtagac 180
gcatcacgag acatccattt acttaatcac agaggtggat c 221

<210> 295
<211> 127
<212> DNA
<213> Homo sapiens

<400> 295
gatcaagaat ttgggtggga gaaaagaaag tgggttatca agggtgattt gaaattttct 60
gcagcattaa agctggcgct taataagaat aagtaataat aaagaaattt ctaacaaata 120
aaaaaaa 127

<210> 296
<211> 120
<212> DNA
<213> Homo sapiens

<400> 296
cctgcgggtg ctgccccacg tcccccacca aagcccatgt aaggagctga gttcttaaag 60
actgaagaca ggctattctc tggagaaaaa taaaatggaa attgtactca aaaaaaaaaa 120

<210> 297
<211> 168
<212> DNA
<213> Homo sapiens

<400> 297
gatcaccccc tccccaataa agctaaaact cacctgagtt gtaaaaaact ccagttgaca 60
caaaatagac tacgaaagtg gctttaacat atctgaacac acaatagcta agacccaaac 120
tgggattaga taccccacta tgcttagccc taaaccaaaa aaaaaaaa 168

<210> 298
<211> 138
<212> DNA
<213> Homo sapiens

<400> 298
gatcctcgaa cggaaagcca aatctcgcca agtaggaaag gaaaagggca aatacaagga 60
agaaaccatt gagaagatgc aggaataaag taatcttata tacaagcttt gattaaaact 120
tgaaacanng aanaaaaa 138

<210> 299
<211> 114
<212> DNA
<213> Homo sapiens

<400> 299
gatgctagcc tctgctttag ccagagaagg tttacttttc tgatttgtgt tgggttttga 60
gtaagtttgc atctgggaaa aaggcatttt aaaaaataaa taaacaaaaa aaaa 114

<210> 300
<211> 233
<212> DNA
<213> Homo sapiens

<400> 300

```
ttttttttg ttacgcaaac aaggaaaaaa ttatttttat tatacaacta tttaataatt 60
ttacccatgc attctcggtc tgaagaggtt tgggaaatat tcctttttgt aatgatgtcc 120
ggagctaaaa gtaaatattc ctcctgggcc aaaagtctct ggaacctatt cagtttctgc 180
tgcctcttca ccttctccac attcaaaccg cacaaagtct actaccgaca ccc        233
```

<210> 301
<211> 90
<212> DNA
<213> Homo sapiens

<400> 301

```
tttttttttt tttaggaaat ctgcctattt tatttccatt agaaaaatat tatctatata 60
acattttgtt ccactatctt ctccttgatc                                  90
```

<210> 302
<211> 134
<212> DNA
<213> Homo sapiens

<400> 302

```
gatgccctca gaaggcagga gtgctgccct ctcccatggt gcccgtgcct ctgtgctgtg 60
tatgtgaacc acccatgtga gggaataaac ctggcactag gtcttgtggt ttgtctgcct 120
tcaaaaaaaa aaaa                                                   134
```

<210> 303
<211> 170
<212> DNA
<213> Homo sapiens

<400> 303

```
tttttttttt ttggagggtg ggcagcactc gctttattgt ccagcattcc acatggatag 60
acgcaggaca gcaggccagg gtggtgggct aagaggcagc ctgccgggcc tgatgctgct 120
gggcaaaccg ctgcatccgc tcctcgagct ccggccgaaa gtggcggatc            170
```

<210> 304
<211> 179
<212> DNA
<213> Homo sapiens

<400> 304

```
gatgtgctaa gtgaccacga ggggctctga actgtagctg atgttatcag caggccatgc 60
atcctgctgc caagggtgga cacggctgca gacttctggg ggaattgtcg cctcctgctc 120
ttttgttact gagtgagata agggtgttca ataaagactt ttatccccaa aaaaaaaaa  179
```

<210> 305
<211> 259
<212> DNA
<213> Homo sapiens

<400> 305
gatcagctcc ttgacctctg aggggcagga gtgcttcctg gtgtgtgtat tagaatccct 60
tcctgccttg tttcatggca gtgaaatgcc tcttggtcct gtccaagtgt atctttcact 120
gatttctgaa tcatgttcta gttgcttgac cctgccacat gggtccagtg ttcatctgcg 180
cataactgta ctaaatcctt tttccatatc agtataataa aggagtgatg tgcantaaaa 240
aaaaaaaaaa gaatcggat 259

<210> 306
<211> 231
<212> DNA
<213> Homo sapiens

<400> 306
gatggaatga cctgtgagga atatgttgcc ttcatcctag ctgctgggga agcggggaga 60
ggggtcaggg aggctaatgg ttgctttgct gaatgtttct ggggtaccaa tacgagttcc 120
cataggggct gctccctcaa aaagggaggg tacagatggg gagcttttct tacctattca 180
aggaatacgt gcctttttct taaatgcttt catttattga aaaaaaaaaa a 231

<210> 307
<211> 74
<212> DNA
<213> Homo sapiens

<400> 307
gatcatagga atattaggag ttacctctct gtggaggtat tgacttgtaa tctcattttc 60
ctttcaaaaa aaaa 74

<210> 308
<211> 61
<212> DNA
<213> Homo sapiens

<400> 308
ttttttgggg gttgtgggga agggaagtgt caagctttgc taagacacgg tgacaaagat 60
c 61

<210> 309
<211> 272

<212> DNA
<213> Homo sapiens

<400> 309
tttttttggc atcagtaatt ttaataaaga aaagcatgct ctgagagaaa gctcgctcct 60
tggtctgcag tcctttaaac aaagcagtgc agttcttagc caagggtaag tactgcaact 120
gtcgagagca tcttgtcttc cacacagttg ggtgactctc cgttttgaca caaagataag 180
ccttgccctt gtttcctttt gggagggata tatccactga gatgagaggc caaactccgt 240
ttttcacgag attttttgac tttgagcttc at 272

<210> 310
<211> 187
<212> DNA
<213> Homo sapiens

<400> 310
gatcgcactg cgcaggggtg ccacggagct cacccacgag gactacatgg aaggcatcct 60
ggaggtgcag gccaagaaga aagccaacct acaatactac gcctagggca cacaggccag 120
ccccagtctc acggctgaag tgcgcaataa aagatggttt agggtccctg ccaaaaaaaa 180
aaaaaaa 187

<210> 311
<211> 87
<212> DNA
<213> Homo sapiens

<400> 311
gatccctgca tgtcccgtta ttaagttagc tgtatgacgt tggccaagtt acttaactgt 60
taggagtctt ttctcaaaaa aaaagaa 87

<210> 312
<211> 146
<212> DNA
<213> Homo sapiens

<400> 312
cttttttttt tttttgcgct tcttagattt ctcccctgac tctgccttag agcttttctc 60
ttctgtgtca gccttgtgtt tacgtttgtg tttgctggat tttttgtgct tctttttctt 120
tttgtgccgg tgggacttcc cagatc 146

<210> 313
<211> 69
<212> DNA
<213> Homo sapiens

<400> 313
tttttttttt taagatggag tcttgctctg tcacccaggc tggtctcaaa ctcctggcct 60
caagtgatc 69

<210> 314
<211> 112
<212> DNA
<213> Homo sapiens

<400> 314
tttttttttt ttatttattc atttatttta aaatagagat gggggtctag ctatgttgcc 60
caggctggtc acaaactcct aagttcaaat ggtcctcctg cctcaaatga tc 112

<210> 315
<211> 167
<212> DNA
<213> Homo sapiens

<400> 315
ttgttttttt ttttaacttt ttaaaagttt ttatttacct aaaaacataa atttatcaaa 60
catatcaatt tgctttgcag agcaatttac ataaaaattc atgatgtata ccaacgacag 120
catagcatta tctacctcag tttgtgaagc atcctttcct acagatc 167

<210> 316
<211> 138
<212> DNA
<213> Homo sapiens

<400> 316
gatctcaaga tttctaaatt gtcaagattt acatggcatt gtggtggaac tagttaacac 60
ttagagcttt tggtatgtaa taactatttg ctatggactg attaaatgtt tcaaaagatt 120
gtgttcttca aaaaaaaa 138

<210> 317
<211> 79
<212> DNA
<213> Homo sapiens

<400> 317
ttgttttttt ttacatgcgt tttaacaatc ccccaccctc cagagcccta ggtctatatc 60
cgtatgaaat gcattgatc 79

<210> 318
<211> 102
<212> DNA
<213> Homo sapiens

<400> 318
tttttttttt ttacaaaaca ccaattttat tataaatatc aagaacctac agggtgttta 60
tgggccagca tatgccttca gttatgttga aaatagctga tc 102

<210> 319
<211> 146
<212> DNA
<213> Homo sapiens

<400> 319
tttttttttt tgtgggagtt gagattttaa tatcataaat cggtgtgggg ccacacctct 60
tactgccagg cccgtgggcc tcctatcttc atattgcctg gaaggctgcc tgcagccagg 120
gctcccccca tcccctagga aggatc 146

<210> 320
<211> 87
<212> DNA
<213> Homo sapiens

<400> 320
tttttttttt ttttggagct gggggatgtt tactggtgtg gggggacctg ggaacatcac 60
tgtcgttcgt acatctctcg taggatc 87

<210> 321
<211> 87
<212> DNA
<213> Homo sapiens

<400> 321
tttttttttt tttggaacta aaggaatata tacatttttt tattttgggg ttggtgtggg 60
ctgctctgga ggccaagcct ggagatc 87

<210> 322
<211> 145
<212> DNA
<213> Homo sapiens

<400> 322
tttttttttt ttttggaggc ccgggtggtt gctgccgaaa tgggcaagtt catgaaacct 60

gggaaggtgg tgcttgtcct ggctggacgc tactccggac gcaaagctgt catcgtgaag 120
aacattgatg atggcacctc agatc 145

<210> 323
<211> 135
<212> DNA
<213> Homo sapiens

<400> 323
ttttttttt tgaatcaata aatttatttt ctgcttaagc aggttgagtt gggttttcta 60
tttgcaatag caaaagtcct gactggcaag gtttaaaagt ttgaagactc tcacaggtaa 120
gtgcagctca ggatc 135

<210> 324
<211> 80
<212> DNA
<213> Homo sapiens

<400> 324
gatctgcacc tacgcctggt ctgtatggtg gaatttgtca gctggaactc agaaacaaca 60
acttgaaaaa aaaaaaaaaa 80

<210> 325
<211> 264
<212> DNA
<213> Homo sapiens

<400> 325
gatgtgtcaa cggatactag aaaatgaaaa agacttggaa gaagctgagg aatataaaga 60
agcacgttta gtactggatt cagtgaagtt agaagcctga aacttttctc gtatggggtg 120
gtttttgcat taaatcctgg ggtccatttt acaatccatt atttttgacc actgctatgt 180
gttcaagtag tatgagaatg tgattgtttt tatctggtta catatatatt tctttgtcta 240
atttaatatg tcaaataaat gagt 264

<210> 326
<211> 207
<212> DNA
<213> Homo sapiens

<400> 326
gatccagtga atattcaaga gagctacatt tgaagcctgt acaaaagctt atccctgtaa 60
cacatgtgcc ataatataca aacttctact ttcgtcagtc cttaacatct acctctctga 120
attttcatga atttctattt cacaagggta attgttttat atacactggt agcagcatac 180
aataaaactt agtatgaaaa aaaaaaa 207

<210> 327
<211> 195
<212> DNA
<213> Homo sapiens

<400> 327
gatctagagc caatcgtatc ttgccagtat cattttcaag cccttacttg tctacttcca 60
ctgttgccca taagtatcct gataaaattt gttttctcaa atatttgcta tgagaggttg 120
atggattaat taaataagtc aattcctgga atttgagaga gcaaataaag acctgagaac 180
cttccaaaaa aaaaa 195

<210> 328
<211> 185
<212> DNA
<213> Homo sapiens

<400> 328
gatcagatga ccattgagga cttgaatgaa gctttcccag aaaccaaatt agacaagaaa 60
aagtatccct attggcctca ccaaccaatt gagaatttat aaaattgagt ccaggaggaa 120
gctctggccc ttgtattaca cattctggac attaaaaata ataattatac aacaaaaaaa 180
aaacc 185

<210> 329
<211> 73
<212> DNA
<213> Homo sapiens

<400> 329
gatcactagc cgaacatcac ccttatctgc tctcctgtgc acatcaatta aatgaagagg 60
gagggaaaaa aaa 73

<210> 330
<211> 125
<212> DNA
<213> Homo sapiens

<400> 330
tcttatttaa ggggaacgtg tgggctattt aggctttatg accctgaagt aggaaccaga 60
tgtcggatac agttcacttt agctaccccc aagtgttatg ggcccggagc gagaaaaaaa 120
aaaaa 125

<210> 331

<211> 73
<212> DNA
<213> Homo sapiens

<400> 331
gatcatgcaa ttccttcaat tatgatggaa agacttgaac tttctgaaat aaaacaaaaa 60
tacaaaaaaa aaa 73

<210> 332
<211> 242
<212> DNA
<213> Homo sapiens

<400> 332
tttttttttt ttctttggga gatggagttt tgctcttgtt gcccaggctg gagtgcaatg 60
gcaccatctc gactcaccgc aacctccacc tcccaggttc aaacaattct cccgcctcag 120
cctcccgagt agctgggatt acaggcatgc accaccatgc ctggctaatt ttgtattttt 180
agtagagatg gggtttctcc atgctggtca ggctggtctc aaactcctga cctcaggtga 240
tc 242

<210> 333
<211> 97
<212> DNA
<213> Homo sapiens

<400> 333
tttttttttt ctttggaagc actggaatac agctttattc ctacacgatt agacccgtta 60
ccccgtgggt ctggccgacc gtcctgactc ggagatc 97

<210> 334
<211> 149
<212> DNA
<213> Homo sapiens

<400> 334
ggggggggacc cttaggtctc actgctctct gattcagagc tcagctgggc ccccagttcc 60
agaccccagc attcccggtc actccctccc taatctgagc atcactcaag ctctttatta 120
aactcaattt gggccagaaa aaaaaaaaa 149

## Beschreibung

Die Erfindung betrifft mRNA Moleküle zur Verwendung als Indikatoren für den Aktivierungs- und Funktionszustand von T-Zellen.

Die sog. "Immunantwort", d.h. die vom Körper aufgebauten antigenspezifischen Abwehrreaktionen, gewährleistet unter physiologischen Bedingungen eine Abwehr krankmachender Faktoren, wie Mikroorganismen (Viren, Bakterien, Parasiten) und Tumorzellen. Unter pathologischen Bedingungen kann sich die Abwehrreaktion jedoch auch gegen körpereigene Strukturen richten; man spricht dann von einer "Autoimmunreaktion". Ebenfalls unerwünscht unter medizinischen Gesichtspunkten ist die Abwehrreaktion gegen ein transplantiertes Organ oder Gewebe, man spricht dann von einer "Transplantat-Abstoßungsreaktion". Träger der Abwehrreaktion sind spezialisierte hämatopoietische Zellen, insbesondere Lymphozyten und davon wieder eine Untergruppe, die sog. T-Lymphozyten (synonym: T-Zellen).

Für die erwünschten Abwehrreaktionen aber auch die unerwünschten (pathogenen) Reaktionen werden die grundsätzlich bereits im Körper vorhandenen T-Lymphozyten in einen aktivierten Zustand versetzt. Die Aktivierung geschieht durch die Einwirkung von Aktivierungssignalen auf die T-Zellen. Die Aktivierungssignale werden durch die Einwirkung sog. Rezeptorliganden bewirkt, die auf der Oberfläche benachbarter Zellen oder in der extrazellulären Matrix gebunden sind, oder sie werden von löslichen Faktoren in der perizellulären Flüssigkeit (u.a. durch sog. Zytokine) vermittelt. Die Einwirkung der Aktivierungssignale führt zur Umwandlung ruhender T-Zellen in aktivierte, funktionell aktive Zellen, die dann in der Lage sind, Immunantworten zu unterstützen (T-Helferzellen), zu unterdrücken (T-Suppressorzellen) oder aber direkt andere Zellen abzutöten (zytotoxische T-Zellen, synonym: T-Killerzellen). Auch einige Lektine, wie z.B. Phytohämagglutinin (PHA), können T-Zellen aktivieren; PHA wurde in der Vergangenheit als Standardsubstanz zur Aktivierung von T-Zellen in vitro herangezogen. Die Gesamtheit der molekularen Veränderungen bei der Aktivierungsreaktion und funktionellen Differenzierung von T-Zellen ist jedoch auch derzeit noch nur in Umrissen bekannt. Insbesondere die Erfassung und damit die Diagnostik des Aktivierungs- und Funktionszustands der T-Zellen und auch aller anderen immunkompetenten Zellen ist beim gegenwärtigen Stand der Technik nur unzureichend möglich .

Ein Schwachpunkt in der Immundiagnostik ist beispielsweise die Tatsache, dass immunsuppressive Behandlungen, die auf die Unterdrückung der T-Zell-Aktivierung abzielen, nach Organtransplantationen, bei Autoimmunerkrankungen, und chronischen Entzündungen wegen fehlender diagnostischer Parameter, die eine signifikante Ausage über den Funktionszustand der T-Zellen erlauben, nicht individuell eingestellt werden können. Konsequenz: jeder Patient erhält dieselbe Medikamentendosis. Anschließend wird die Verabreichung immunsuppressiver Medikamente durch die Messung von Medikamenten-Blutspiegeln und nicht etwa des suppressiven Effekts auf T-Zellen verfolgt. Die Entwicklung diagnostischer Verfahren für die individuelle Steuerung immunmodulierender, insbesondere immunsuppressiver Therapien, die T-Zellfunktionen beeinflussen, ist außerdem auch deshalb erforderlich, weil immunsuppressive Medikamente höchst bedenkliche Nebenwirkungen auslösen, und weil entsprechende Patientinnen und Patienten über sehr lange Zeiträume - üblicherweise lebenslang - behandelt werden müssen.

Zwar sind eine Reihe von Biomolekülen bekannt, die als Indikatoren (sog. "Marker" oder speziell "Aktivierungsmarker") für aktivierte T-Lymphozyten/T-Zellen herangezogen werden können (wie z.B. das Zytokin "Interleukin-2 [IL-2]" oder der "Interleukin-2-Rezeptor [IL-2R,CD25]"), jedoch ist es derzeit aufgrund der begrenzten Zahl und Qualität der verfügbaren Aktivierungsmarker z.B. nicht möglich, durch Messung eines Markers oder einer Markerkombination zu unterscheiden, ob die von einem Patienten isolierten T-Lymphozyten sich in einem Zustand befinden, (i) der eine ausreichende nicht jedoch übermäßige (und damit nebenwirkungsreiche) Immunsuppression gewährleistet, oder (ii) ob eine Aktivierung zu einer physiologisch erwünschten oder unerwünschten Folgereaktion führt, d.h. ob die Aktivierung bedingt ist durch eine virale oder bakterielle Infektion (erwünschte, "gesundmachende" Aktivierung der T-Zellen), durch eine Autoimmunreaktion und/oder durch eine Transplantatabstoßung (unerwünschte "krankmachende" Aktivierung der T-Zellen). Eine solche Unterscheidung ist jedoch zum einen unter medizinisch/diagnostischen Gesichtspunkten wünschenswert, da die therapeutischen Konsequenzen bei diesen Zuständen deutlich voneinander verschieden sind; je nach Ursache sollte entweder gar keine, eine antivirale bzw. antibakterielle oder eine immunsuppressive Therapie durchgeführt werden. Zum anderen ist diese Unterscheidung auch aus rein wirtschaftlichen Gesichtspunkten erstrebenswert: geeignete Analyseverfahren könnten durch die Vermeidung unnötiger und die optimale Ausrichtung notwendiger Therapieverfahren eine Kostenreduktion ermöglichen.

Außerdem könnte ein solches Analyseverfahren in der Medikamentenentwicklung für die Wirkstoffsuche eingesetzt werden, nämlich indem man das Verfahren in sog "Screening"-Verfahren anwendet, um Wirkstoffe (Pharmaka) zu identifizieren, die den Aktivierungszustand von T-Lymphoyzten in eine gewünschte Richtung verändern, sei es, daß sie eine unerwünschte Aktivierungsreaktion unterdrücken oder eine erwünschte Aktivierungsreaktion induzieren bzw. verstärken.

Die Probleme bei der Erfassung des Aktivierungs- und Funktionszustands von T-Lymphozyten sind - wie vorstehend bereits angedeutet - insbesondere dadurch bedingt, daß die zu bestimmten Zeitpunkten der Aktivierung in der jeweiligen T-Zellpopulation vorliegenden ("exprimierten") Muster (Profile) aktiver bzw. inaktiver Biomoleküle (der genannten Marker) nur unvollständig bekannt sind. Der zelluläre Aktivierungs- und Funktionszustand entspricht jedoch einem Integral aus den zu einem betreffenden Zeitpunkt in Zellen vorliegenden funktionellen Biomolekülen. Die Art und Menge (das "Profil") der in T-Zellen verfügbaren Biomoleküle wiederum beruht - wie bei allen anderen Zellen auch - auf den zu einem gegebenen Zeitpunkt aus der gesamten genetischen Information der Zelle über den Proteinsynthese-Mechanismus synthetisierten Proteinen. Die in einer Zelle als Proteine vorliegenden Moleküle können indirekt über ihre mRNAs (=Transkripte) erfaßt werden. Vereinfacht und in erster Näherung kann man sagen: aus einer verminderten Expression X-spezifischer mRNA folgt eine verminderte Syntheserate des Proteins X und eine verminderte Verfügbarkeit der X-spezifischen Funktion(en) in der Zelle. Das Umgekehrte gilt für die vermehrte Expression spezifischer mRNAs. Eine möglichst umfassende und detaillierte Beschreibung des Profils hoch- bzw. herabregulierter mRNAs ("Transkript-Profilierung") kann demnach den zellulären Aktivierungs- und Funktionszustand indirekt abbilden.

Aufgabe der vorliegenden Erfindung ist deshalb die Bereitstellung zellulärer Biomoleküle und/oder Kombinationen von Biomolekülen (sog. Molekül-"Profile"), die es erlauben aktivierte T-Lymphozyten unter diagnostischen Gesichtspunkten zu charakterisieren.

Eine Lösung dieser Aufgabe besteht in der Verwendung von mRNA-Molekülen als Indikatoren für den Aktivierungs- und Funktionszustand von T-Zellen, die dadurch gekennzeichnet sind, daß sie bei aktivierten T-Zellen im Vergleich zum Normal- bzw. Ruhezustand vermehrt oder vermindert exprimiert (auf- bzw. abreguliert) sind und auf dem höheren bzw. niedrigeren Konzentrationsspiegel gehalten werden, und daß sie mit einer der in den Sequenzprotokollen SEQ ID NO. 1 bis NO. 334 dargestellten Nukleotidsequenzen oder einer davon abgeleiteten Nukleotidsequenz oder einer hierzu komplementären Nukleotidsequenz oder einer Teilsequenz einer dieser Nukleotidsequenzen hybridisieren.

Der Begriff "hybridisiert" bezieht sich auf die im Stand der Technik bekannten Hybridisierungsverfahren unter üblichen, insbesondere unter hoch stringenten Hybridisierungsbedingungen. Die konkreten Hybridisierungsparameter wählt der Fachmann anhand der eingesetzten Nukleotidsequenz und seines allgemeinen Fachwissens (vgl.: Current Protocols in Molecular Biology, Vol. 1, 1997, John Wiley & Sons Inc., Suppl. 37, Chapter 4.9.14).

Der Begriff "davon abgeleitete Nukleotidsequenz(en) " steht hier für solche Nukleotidsequenzen, die anhand der in den Sequenzprotokollen SEQ ID NO. 1 bis NO. 334 dargestellten Nukleotidsequenzen durch Anwendung einzelner oder einer Kombination verschiedener im Stand der Technik geläufiger Verfahren, nämlich rein computergestützter bioinformatischer Datenbank-Recherchen und/oder experimenteller Verfahren erhalten werden, und die die komplette Primärstruktur - inklusive der 3'- und 5' -nichtkodierenden Abschnitte - oder Varianten der Primärstruktur, insbesondere allelische und Splice-Varianten, darstellen.

Für die Durchführung der rein computergestützten bioinformatischen Datenbank-Recherchen können insbesondere sowohl Datenbanken, die bereits Informationen über Volllängen-cDNAs enthalten (z.B. Genbank oder EMBL-Datenbank), als auch sog. EST-Datenbanken (EST steht für "expressed sequence tag") herangezogen werden . EST-Datenbanken enthalten Informationen über relativ kurze (bis max. ca. 400 Nukleotide lange) cDNA-Fragmente (sog. "ESTs"), die durch massenhaftes zufälliges Sequenzieren von cDNA-Genbanken gewonnen wurden. Diese EST-Datenbanken bieten die Möglichkeit, durch computergestütztes Zusammenfügen von EST-Sequenzen komplette Volllängen-cDNA-Sequenzen zu ermitteln (sog. "EST clustering"). Weitere Datenbanken liefern Informationen über Splice-Varianten (z.B. LabonWeb der Fa. Compugen, 25 Leek Crescent Richmond Hill, Ontario, Canada; www. compugen. com) oder biologische Funktionen (z.B. "GeneCards - a database of human genes" über HUSAR, s.u.). Sehr geeignet für diese bioinformatischen Analysen ist zum Beispiel das Programmpaket HUSAR (GENIUSnet User Support, Im Neuenheimer Feld 370, D-69120 Heidelberg, Deutschland;www.genome.dkfz-heidelberg.de).

Zur Durchführung experimenteller Verfahren eignen sich insbesondere das 5'- und das 3' -RACE (rapid amplification of cDNA ends) - Verfahren [Frohman, M.A., Dush, M.K., Martin, G.R. (1988): Rapid production of full-length cDNAs from rare transcripts: amplification using a single gene-specific oligonucleotide primer; Proc. Natl. Acad. Sci. (USA) 85 pp. 8998]. Mit diesen Verfahren kann das 5'- bzw. 3' -Ende von mRNA Transkripten ermittelt werden.

Die erfindungsgemäßen Indikator-mRNAs wurden überraschenderweise bei der Kombination von Verfahren der in vitro-Aktivierung und der Transkript-Profilierung von T-Zellen gefunden.

Die Polynukleotide mit einer in den Sequenzprotokollen SED IQ NO. 1 bis NO. 334 angegebenen Nukleotidsequenz oder einer davon abgeleiteten Nukleotidsequenz oder einer hierzu komplementären Nukleotidsequenz oder einer Teilsequenz einer dieser Nukleotidsequenzen können selbst als Nachweisreagenz (Sonde, Marker, Detektor) für den Aktivierungs- und Funktionszustand von T-Zellen verwendet werden.

Die Erfindung umfaßt insofern insbesondere auch die Verwendung von Polynukleotiden mit einer in den Sequenzprotokollen SEQ ID NO. 1 bis NO. 334 angegebenen Nukleotidsequenz oder einer davon abgeleiteten Nukleotidsequenz (a) als Diagnostikum und/oder als Therapeutikum (insbesondere Aktivator oder Inhibitor) für den Nachweis und/oder die Beeinflussung und/oder Steuerung des Aktivierungs- und Funktionszustand von T-Zellen (b) als Diagnostikum und/oder Therapeutikum (insbesondere Aktivator oder Inhibitor) für Immunität, Autoimmunität, Transplantatabstoßungsreaktionen, akute und/oder chronische, immunologisch bedingte Entzündungsreaktionen und/oder Immunsuppression, (c) zur Herstellung von Diagnostika und/oder Therapeutika (insbesondere Aktivator oder Inhibitor) für den Nachweis und/oder die Beeinflussung und/oder Steuerung des Aktivierungs- und Funktionszustand von T-Zellen.

Der Begriff 'Diagnostika' umfaßt in diesem Zusammenhang nicht nur Arzneimittel zur diagnostischen Anwendung am oder im menschlichen oder tierischen Körper, sondern auch sogenannte 'diagnostische Testbestecke', bei denen die für die Diagnose notwendigen Informationen/Daten/Meßwerte anhand von Testreaktionen in vitro gewonnen werden. Der Begriff umfaßt hier außerdem auch die Ermittlung/Feststellung bzw. den Nachweis toxischer Einflüsse auf das Immunsystem.

Die erfindungsgemäßen Polynukleotide können ferner zum Aufbau von Screening-Systemen für die Austestung von pharmakologisch und/oder toxikologisch interessanten Substanzen (Pharmaka, Chemikalien mit immunstimulierender / immuntoxischer Wirkung) herangezogen werden.

Die Erfindung betrifft deshalb auch die Verwendung eines Polynukleotids mit einer in den Sequenzprotokollen SEQ ID NO. 1 bis NO. 334 angegebenen Nukleotidsequenz oder einer davon abgeleiteten Nukleotidsequenz oder einer dazu komplementären oder damit hybridisierenden Nukleotidsequenz zur Identifizierung von medizinisch oder pharmakologisch einsetzbaren Substanzen, die an das Polynukleotid bzw. an das/die von diesem kodierte Protein(e) binden und dadurch den Aktivierungs- und Funktionszustand von T-Zellen beeinflussen.

Gegenstand der Erfindung ist desweiteren ein Reagenz zum Nachweis des Aktivierungs- und Funktionszustand von T-Zellen, dadurch gekennzeichnet, daß das Reagenz unter Verwendung wenigstens einer der in den Sequenzprotokollen SEQ ID NO. 1 bis NO. 334 angegebenen Nukleotidsequenz oder einer davon abgeleiteten Nukleotidsequenz oder einer dazu komplementären oder damit hybridisierenden Nukleotidsequenz hergestellt ist.

Eine bevorzugte Ausführungsform dieses Reagenz ist dadurch gekennzeichnet, daß es eine Substanz enthält, die spezifisch mit einer der in den Sequenzprotokollen SED IQ NO. 1 bis NO. 334 angegebenen Nukleotidsequenz oder einer davon abgeleiteten Nukleotidsequenz oder einer dazu komplementären oder damit hybridisierenden Nukleotidsequenz reagiert.

Bei dieser Substanz kann es sich insbesondere um ein Polynukleotid handeln, das einen detektierbaren Marker, z.B. ein Lumineszenzgen o.ä. aufweist.

Ebensogut kann die Substanz ein modifiziertes Oligo- oder Polynukleotid sein.

Die erfindungsgemäßen Polynukleotide können auch in Vektoren insertiert werden und mit Hilfe dieser in menschliche oder tierische T-Zellen eingebracht werden, z.B. zu dem Zweck, diese gezielt zu aktivieren oder zu inhibieren. Die Erfindung betrifft deshalb auch ein rekombinantes DNS-Vektormolekül, das wenigstens eine der in den Sequenzprotokollen SEQ ID NO. 1 bis NO. 334 dargestellten Nukleotidsequenzen oder davon abgeleiteten Nukleotidsequenzen oder dazu komplementären oder damit hybridisierenden Nukleotidsequenzen aufweist, und das in T-Zellen transkribierbar ist.

Ein weiteres erfindungsgemäßes Einsatzgebiet der in den Sequenzprotokollen SEQ ID NO. 1 bis NO. 334 dargestellten Nukleotidsequenzen ist ihre Verwendung zur Herstellung von monoklonalen oder polyklonalen oder rekombinanten Antikörpern gegen das/die von der/den betreffenden Nukleotidsequenz(en) kodierte(n) Protein(en). Die Antikörperherstellung erfolgt vorzugsweise durch Immunisierung von insbesondere Mäusen, wobei die Immunisierung entweder nach Expression des entsprechenden Proteins mit dem Protein selbst oder aber durch genetische Immunisierung unter Verwendung geeigneter Vektoren mit der für das Protein kodierenden cDNA bzw. mRNA erfolgt. Die gewonnenen Antikörper, die spezifisch mit Proteinen reagieren, die durch eine oder mehrere der in den Sequenzprotokollen SEQ ID NO. 1 bis NO. 334 dargestellten Nukleotidsequenzen oder davon abgeleiteten Nukleotidsequenzen oder dazu komplementären oder damit hybridisierenden Nukleotidsequenzen kodiert werden, und die Verwendung solcher Antikörper zum Nachweis oder zur Beeinflussung des Aktivierungs- und Funktionszustand von T-Zellen (insbesondere im Zuge einer diagnostischen und/oder therapeutischen Behandlung von Autoimmunerkrankungen oder von unerwünschten natürlichen Körperabwehrreaktionen) sind infolgedessen ebenfalls Gegenstand der vorliegenden Erfindung.

Ein weiteres erfindungsgemäßes Einsatzgebiet der in den Sequenzprotokollen SEQ ID NO. 1 bis NO. 334 dargestellten Nukleotidsequenzen ist ihre Verwendung zur Herstellung von Bindemolekülen, insbesondere RNA-Antisense-Molekülen, modifizierten Nukleinsäuren mit Antisense-Funktion (z.B. "peptide nucleic acids" [Nielsen, P.E., "Peptide nucleic acids: on the road to new gene therapeutic drugs. Pharmacol. Toxicol 2000, 86:3-7 und Soomets, U., Hallbrink, M., Langel, U.: Antisense properties of peptide nucleic acids. Front Biosci. 1999, 4: D782-786]) und/oder Ribozymen. Die Erfindung umfaßt infolgedessen auch Bindemolekükle, insbesondere RNA-Antisense-Moleküle, modifizierte Nukleinsäuren mit Antisense-Funktion und/oder Ribozyme, die spezifisch mit einer oder mehrerer der in den Sequenzprotokollen SEQ ID NO. 1 bis NO. 334 dargestellten Nukleotidsequenzen oder davon abgeleiteten Nukleotidsequenzen oder dazu komplementären oder damit hybridisierenden Nukleotidsequenzen reagieren, die betreffenden Bindemoleküle selbst und die Verwendung solcher Bindemoleküle zum Nachweis oder zur Beeinflussung des Aktivierungs- und Funktionszustands von T-Zellen, insbesondere im Zuge einer diagnostischen und/oder therapeutischen Behandlung (i) einer Immunreaktion gegen Mikroorganismen, Impfstoffe oder Allergene, (ii) einer akuten und/ oder chronischen immunologisch bedingten Entzündung, (iii) einer Immundefizienz bzw. -suppression, (iv) einer malignen Erkrankung des Immunsystems, (v) einer Graft-vs.-Host-Reaktion, oder (vi) einer Transplantat-Abstoßungsreaktion.

Eine weitere erfindungsgemäße Anwendung der in den Sequenzprotokollen SEQ ID NO. 1 bis NO. 334 dargestellten Nukleotidsequenzen oder einer davon abgeleiteten Nukleotidsequenz oder dazu komplementären oder damit hybridisierenden Nukleotidsequenz sind Testbestecke zur Messung eines Genexpressionsprofils in ex vivo isolierten Immunzellen, insbesondere T-Zellen, bei Reaktionen und Erkrankungen des Immunsystems (Immunreaktion gegen Mikroorganismen, Impfstoffe oder Allergene, akute und/oder chronische immunologisch bedingten Entzündung, Immundefizienz bzw. -suppression, malignen Erkrankung des Immunsystems, Graft-vs.-Host-Reaktion, Transplantat-Abstoßungsreaktion). Der dem Testbesteck zugrundeliegende Test umfaßt erfindungsgemäß entweder (A) die folgenden Schritte:

(i) Isolation von mRNA aus Geweben, Zellen oder Körperflüssigkeit bei Gesunden,
(ii) Isolation von mRNA aus Geweben, Zellen oder Körperflüssigkeiten bei Erkrankten,
(iii) Einsatz dieser mRNAs, bzw. davon abgeschriebener cDNA, in Bindungsverfahren zur Messung der Interaktion mit den in SEQ ID NO. 1 bis NO. 334 beschriebenen Nukleinsäuresequenzen,
(iv) Auswertung zur Feststellung derjenigen mRNA-Spezies, die bei Gesunden und Erkrankten eine unterschiedliche Expressionsstärke zeigen und die demnach als Marker für "gesund" bzw. "erkrankt" eingesetzt werden können.

oder (B) die folgenden Schritte:

(i) Durchführung einer bioinformatischen Analyse in verschiedenen Datenbanken hinsichtlich Vollängensequenz und Sequenzvarianten zur Nukleotidsequenz des Polynukleotids,
(ii) Synthese von Oligonukleotiden anhand der gewonnenen Vollängensequenz und Sequenzvarianten,
(iii) Aufbringen der Oligonukleotide auf ein Trägermaterial,
(iv) Isolation von mRNA aus Geweben, Zellen oder Körperflüssigkeiten bei Gesunden,
(v) Isolation von mRNA aus Geweben, Zellen oder Körperflüssigkeiten bei Erkrankten,
(vi) Einsatz dieser mRNAs bzw. davon abgeschriebener cDNA in Bindungsverfahren zur Messung der Interaktion mit den auf dem Trägermaterial aufgebrachten Oligonukleotiden,
(vii) Detektion und Identifikation derjenigen mRNA-Spezies, die bei Gesunden und Erkrankten eine unterschiedliche Expressionsstärke zeigen.

oder (C) die folgenden Schritte:

(i) Synthese von Oligonukleotidprimern für die Polymerase-Kettenreaktion (PCR) anhand
entweder der Nukleotidsequenz des Polynukleotids
oder der mittels bioinformatischer Analyse in verschiedenen Datenbanken gewonnenen Vollängensequenz und/oder Sequenzvarianten des Polynukleotids,
(ii) Isolation von mRNA aus Geweben, Zellen oder Körperflüssigkeiten bei Gesunden,
(iii) Isolation von mRNA aus Geweben, Zellen oder Körperflüssigkeiten bei Erkrankten,
(iv) Einsatz dieser mRNAs bzw. davon abgeschriebener cDNA in einer Polymerase-Kettenreaktion (PCR) und Feststellung von Unterschieden in der Expressionsstärke einzelner Gene.

Die Erfindung wird im folgenden anhand von Herstellungs- und Anwendungsbeispielen näher erläutert. Von den im Rahmen dieser Beispiele erwähnten Figuren zeigen:

**Fig. 1:** Qualitätskontrolle der für das Experiment eingesetzten mRNA nichtstimulierter und Phytohämagglutinin-stimulierter T Zellen

**Fig. 2:** Prinzip der Herstellung einer Microbead-gebundenen Genbank mit cDNA aus ruhenden und Phytohämagglutinin-stimulierten T-Zellen mit anschließender Anreicherung der auf- und abregulierten Transkripte: cDNA-Synthese und Klonierung

**Fig. 3:** Prinzip der Herstellung einer Microbead-gebundenen Genbank mit cDNA aus ruhenden und Phytohämagglutinin-stimulierten T-Zellen mit anschließender Anreicherung der auf- und abregulierten Transkripte: Enzymbehandlung nach Sortierung

**Fig. 4:** Prinzip der Herstellung einer Microbead-gebundenen Genbank mit cDNA aus ruhenden und Phytohämagglutinin-stimulierten T-Zellen mit anschließender Anreicherung der auf- und abregulierten Transkripte: Beladung der Microbeads

**Fig. 5:** Beispiel für eine Filterhybridisierung zur Erfassung eines Transkriptprofils anhand einer Auswahl der in den Sequenzprotokollen SEQ ID NO. 1 bis NO. 334 genannten Nukleinsäuresequenzen.

Die hier verwendeten Abkürzungen sind wie folgt definiert:

Microbead(s) = 5 µm große Kügelchen aus Glycidal-Methacrylate

Tag = aus 8 präformierten 4er-Nukleotidblöcken (TTAC, AATC, TACT, ATCA, ACAT, TCTA, CTTT, CAAA) aufgebaute 32 Nukleotide lange Oligonukleotide

anti-Tag = die zu dem Tag komplementäre Nukleotid-Sequenz

**Beispiel 1**

**Isolierung und Aktivierung von T-Lymphozyten, sowie Isolation und Qualitätskontrolle von mRNA**

Für die Isolierung von T Zellen wurde freiwilligen Spendern Blut entnommen und mit Heparin antikoaguliert. Aus dem Heparinblut wurden die mononukleären Leukozyten mittels Ficoll-Dichtegradienten-Zentrifugation (Pan GmbH; Bestellnummer PO4-60500) nach Standardmethoden isoliert. Danach wurde die Zellzahl durch Auszählung in einer Neubauer-Zählkammer bestimmt.

Anschließend wurden aus diesen Zellen die T Lymphozyten mittels "Schafserythrozyten-Rosettierung" isoliert. Hierfür wurden 3 - 4 ml Schafserythrozyten (ICN Biomedicals GmbH, Bestellnummer 49-36145) durch zweimaliges Waschen mit PBS (phosphatgepufferte Saline) vorbereitet und auf eine 5 %-ige (Vol.Nol.) Lösung in PBS eingestellt. Anschließend wurden die isolierten Zellen mit der Erythrozyten-Lösung im Verhältnis 5 x $10^7$ Zellen/1.5 ml Schafserythrozyten-Lösung gemischt, für 10 Minuten bei Raumtemperatur inkubiert, zentrifugiert (90 x g, bei Raumtemperatur für 10 Minuten ohne Bremse) und dann für eine weitere Stunde bei Raumtemperatur inkubiert. Danach wurden die sedimentierten Zellen durch leichtes Schwenken resuspendiert. Die Gesamtlösung wurde anschließend mit 15 ml Ficoll unterschichtet und bei 180 x g bei Raumtemperatur ohne Bremse zentrifugiert. Der Überstand wurde abgenommen und verworfen. Die sedimentierten Zellen wurden in 10 ml ACK-Lysepuffer (4,145 g $NH_4Cl$, 0,5 g $KHCO_3$, 0,186 g EDTA ad 500 ml Aqua bidestillata) 5 Minuten lang resuspendiert. Nach Lyse der Erythrozyten (erkennbar an der Klärung der Lösung) wurde sofort mit RPMI auf 50 ml aufgefüllt und für 10 Minuten bei 250 x g bei Raumtemperatur zentrifugiert. Die sedimentierten Zellen wurden in Vollmedium resuspendiert und entweder direkt für die mRNA-Präparation (= ruhende T Zellen) oder für die Stimulation mit Phytohämagglutinin (PHA) (zur Gewinnung von mRNA aus PHA-stimulierten T Lymphozyten) eingesetzt.

Für die Stimulation mit PHA wurden die isolierten T Lymphozyten in Vollmedium bei Konzentrationen zwischen 2 x $10^6$ bis 3 x $10^7$ Zellen/Schale in Gewebekulturschalen (Falcon GmbH, Bestellnummer 3004) eingesät. Für die Stimulation wurde Phytohämagglutinin ("PHA"; Murex GmbH, Bestellnummer HA 16/17) in einer Konzentration von 1 µg/ml zugesetzt. Danach wurden die Zellen geerntet und einmal in PBS gewaschen. Anschließend erfolgte die Isolation von mRNA nach Herstellerangaben unter Verwendung eines kommerziell verfügbaren Isolationsbestecks (FastTrack 2.0 mRNA Isolationskit; Invitrogen GmbH, Bestellnummer K-1593-03).

Die Qualitätskontrolle der isolierten mRNA erfolgte anhand des bekannten T-Zell-Aktivierungsmarkers Interleukin-2 (IL-2) und Interleukin-2-Rezeptor (IL-2R; CD25). Hierfür wurde eine PCR nach reverser Transkription (rt-PCR) durchgeführt. Die mRNA wurde zunächst in einzelsträngige cDNA umgeschrieben und in einem zweiten Schritt unter Verwendung IL-2- und IL-2R-spezifischer Primer in einer Standard-PCR-Reaktion amplifiziert. Als interne Kontrolle wurde in einem Parallelansatz mit den gleichen cDNA-Proben eine PCR mit kommerziell verfügbaren GAPDH-spezifischen Primem (Clontech GmbH; Bestellnummer 5406-1) durchgeführt. Die Amplifikationsprodukte wurden nach Standardverfahren dokumentiert: Agarose-Gelelektrophorese und Photographie nach Ethidiumbromid-Markierung und UV-Illumination. Das Ergebnis dieser Qualitätskontrolle ist in Fig. 1 gezeigt: IL-2- bzw. IL-2-R-spezifische mRNA war nur nach PHA-Stimulation (Tp), nicht jedoch vor PHA-Stimulation (T0) nachweisbar. Die Expression des "housekeeping" Gens GAPDH war zu allen Zeitpunkten nachweisbar.

**Beispiel 2**

**Isolierung und Charakterisierung von Indikator mRNA aus PHA-aktivierten T-Lymphozyten**

Als erster Schritt zur Darstellung von erfindungsgemäßen Indikator-mRNA-Molekülen in/aus aktivierten T-Zellen wurde die mRNA ruhender und aktivierter T-Zellen nach Standardprotokollen in doppelsträngige cDNA umgeschrieben (cDNA Synthese Kit; Stratagene, Bestellnummer 200401). Dabei wurde ein Oligo(dT)-Primer eingesetzt, der eine BsmBI-Schnittstelle und am 5'-Ende eine Biotin-Markierung aufweist (Fig.2.A). Anschließend wurde die cDNA mit DpnII geschnitten (Fig.2.B), die resultierenden biotinmarkierten DNA-Fragmente wurden mittels Streptavidin-Magnetkügelchen (Deutsche Dynal GmbH; Bestellnummer 112.05) isoliert und mit Bindungs- und Waschpuffer Puffer (20 mM Tris, 2 mM EDTA 2 M NaCl) gewaschen (Fig.2.C). Anschließend wurde die DNA durch BsmBI-Verdau von den Magnetkügelchen getrennt (Fig.2.D). Die so gewonnenen cDNA Fragmente wurden in den linearisierten (BbsI und BamHI-Restriktionsverdau) sog. "Probenvektor" (CTI1; LYNX Therapeutics Inc., Hayward, USA, beschrieben in den Druckschriften WO99/35293 und Proc. Natl. Acad. Sci. USA, Vol 97, No. 4, 2000, p. 1665-1670: S.Brenner et al. "In vitro cloning of complex mixtures of DNA on microbeads: Physical separation of differentially expressed cDNAs") bzw. den linearisierten sog. "Tag-Vektor" (NCV2; LYNX Therapeutics Inc., Hayward, USA , beschrieben in den Druckschriften WO99/35293 und Proc. Natl. Acad. Sci. USA, Vol 97, No. 4, 2000, p. 1665-1670, a.a.O.) kloniert (Fig.2.E).

Für die weiteren Experimente wurde eine Methode eingesetzt, die die parallele Klonierung von Millionen Nukleinsäuren und deren anschließende Sortierung und Analyse auf der Oberfläche 5 μm großer Glycidal Methacrylate Kügelchen (im folgenden als "microbeads" bezeichnet) erlaubt. Diese Methode ist in der WO99/35293 und in Proc. Natl. Acad. Sci. USA, Vol 97, No. 4, 2000, p. 1665-1670, S.Brenner et al. "In vitro cloning of complex mixtures of DNA on microbeads: Physical separation of differentially expressed cDNAs" ausführlich beschrieben. Auf den diesbezüglichen Inhalt dieser Druckschriften wird hiermit ausdrücklich Bezug genommen. Die parallele Klonierung wird dadurch erreicht, daß mit dieser Methode jedes DNA-Molekül mit einem Markierungs-DNA-Stück mit spezifischer Sequenz (im folgenden als "Tag" bezeichnet) versehen wird, welches in einem Selbstsortierungsprozeß durch ein präformiertes Anti-Tag mit komplementärer Sequenz auf der Oberfläche der Microbeads gebunden wird.

Die Tags sind 32 Nukleotide lange Nukleotidsequenzen, die aus 8 präformierten 4er-Nukleotidblöcken (TTAC, AATC, TACT, ATCA, ACAT, TCTA, CTTT, CAAA) aufgebaut sind. Die freie Kombination der Viererblöcke erlaubt die Generierung von 16.77 Millionen Tags mit individueller DNA-Sequenz. Das Repertoire an Tags ist groß genug, um eine individuelle Tag-Markierung von DNA-Molekülen in einer Probe mit sehr vielen solcher DNA-Moleküle zu ermöglichen. Aufgrund der gewählten Nukleotidkombinationen ist die Schmelztemperatur aller möglichen Tags isothermisch. Bei der Hybridisierung zwischen Tag und anti-Tag ist die Schmelztemperatur zwischen einer perfekten Paarung ("match") und einer nichtperfekten Paarung ("mismatch") groß genug, um unter geeigneten Bedingungen praktisch nur perfekte Paarungen zuzulassen.

Grundlage für die Herstellung tag-markierter DNA-Moleküle ist die Herstellung einer Tag-Plasmidbank mit Hilfe des Vektors NCV2 (LYNX Therapeutics Inc.; Hayward, USA, beschrieben in der Druckschrift WO99/35293), bei der jedes Plasmid eine spezifische Tag-Kombination erhält (Fig.2.E). Mit anderen Worten: durch Klonierung der DNA in dieses Plasmid wird jedes cDNA-Molekül mit einem individuellen Tag markiert. Die Herstellung der komplementären anti-Tag-tragenden Microbead-Population erfolgt durch kombinatorische chemische Synthese.

Der Prozeß erlaubt, daß $10^6$ oder mehr Moleküle in einer Genbank in eine entsprechende Anzahl von Microbeads umgewandelt werden, wobei jedes Microbead ca. $10^5$ identische Kopien eines individuellen DNA-Moleküls trägt. Bei der Verwendung von cDNA entsteht eine Microbead-gebundene cDNA-Bank, die für kompetitive Hybridisierungsexperimente zur differentiellen Genexpressionsanalyse herangezogen werden kann: sollen zwei Expressionszustände (Expressionszustand 1 und 2) analysiert werden, so werden microbead-gebundene Genbanken der zwei Zustände hergestellt. Eine 1:1-Mischung der Microbead-gebundenen Banken wird dann als Substrat für die kompetitive Hybridisierung mit unterschiedlich fluoreszenzmarkierten cDNA-Proben herangezogen: cDNA von Expressionszustand 1 = FAM-markiert [Grünfluoreszenz] und cDNA von Expressionszustand 2 = Cy5-markiert [Rotfluoreszenz]. Die kompetitive Hybridisierung wird unter saturierenden Bedingungen durchgeführt, so daß entsprechend der differentiellen Expression jedes einzelne Microbead die spezifische cDNA aus beiden Proben in dem Verhältnis bindet, in dem die cDNA in den beiden Proben vorliegt. Auf- bzw. Abregulation wird durch die hauptsächliche Emission einer der beiden Fluoreszenzen angezeigt: Microbeads, die cDNAs binden, die stärker in Expressionszustand 1 exprimiert sind, werden grünfluoreszent erscheinen und Microbeads die cDNAs binden die in Expressionszustand 2 stärkert exprimiert sind, werden stärker rotfluoreszent erscheinen. Mittels eines Zweifarben Fluoreszenzaktivierten Zellsortier-Geräts können die Microbeads individuell analysiert und getrennt werden. Die auf den microbeads vorliegenden cDNA-Fragmente werden anschließend durch DNA-Sequenzierung identifiziert.

**Um Indikator-m-RNA-Moleküle für PHA-aktivierte T-Zellen zu identifizieren wurde im einzelnen folgendermaßen vorgegangen:**

Die nach cDNA-Synthese aus der mRNA ruhender bzw. PHA-aktivierter (1, 6, 12 und 48 Stunden) T-Lymphozyten gewonnenen cDNA-Fragmente wurden in den linearisierten (BbsI und BamHI-Restriktionsverdau) sog. "Probenvektor" (CTI1) bzw. den linearisierten sog. "Tag-Vektor" (NCV2) kloniert und in den <u>E. coli</u> Stamm TOP10 (Invitrogen; Bestellnummer C664-24) transformiert (Fig. 2).

Zunächst wurde mit der Genbank in dem Tag-Vektor weiter gearbeitet. Die Kulturbedingungen für die transformierten Bakterien wurden so gewählt, daß 50 ml Übernachtkultur einer Inokulumdosis von 160 000 Klonen entsprachen. Auf diese Weise wurden pro cDNA-Bank sechs Teilmengen ä 160 000 Tag-markierter Klone amplifiziert. Die 160 000 Tags repräsentieren nur 1 % des gesamten Tag-Repertoirs, so daß die Wahrscheinlichkeit, daß zwei verschiedene DNA-Moleküle dasselbe Tag besitzen sehr gering (0.01 %) ist; die Mehrzahl besitzt ein individuelles Tag.

Nach Amplifikation in den Bakterien wurden die Plasmide mit einem kommerziell verfügbaren Isolationsbesteck (Qiagen, Bestellnummer 12263) isoliert. Die gesamten cDNA Inserts wurden mit Hilfe flankierender Primer (forward: PCR-F biotin-markiert; reverse: PCR-R FAM-markiert) amplifiziert (Fig. 3.A). Die Amplifikate wurden mit dem Restriktionsenzym Pacl geschnitten (Fig.3.B). Überschüssiger Primer und biotinyliertes Schnittprodukt wurde mittels Streptavidin-Sepharose-Chromatographie entfernt (Fig.3.C). Die gebildete DNA wurde mit T4 Polymerase und dGTP behandelt. Da die Tags kein G enthalten wird aufgrund der 3'-5'-Exonuklease und der 5'-3' Syntheseaktivität der T4-Polymerase der Anti-Tag-Strang entfernt (Fig.3.D).

Die resultierenden Tag-markierten DNA-Moleküle wurden an die Microbeads gebunden. Da nicht bekannt ist, welche 160 000 Tags in den Tag-gekoppelten DNA Inserts enthalten sind, muß die Beladung der Beads mit dem gesamten Repertoire der 16.77 Millionen anti-Tag-tragenden Microbeads (Fa. LYNX Therapeutics Inc., Hayward, USA) durchgeführt werden (Fig.3.E). Nach der Beladung wurde deshalb die Mehrzahl der Microbeads, die keine DNA gebunden hatten von den 160 000 beladenen Beads (ungefähr 1 % der Gesamtpopulation) in einem fluoreszenzaktivierten Zellsortierer abgetrennt. Die beladenen Beads wurden gesammelt. Die pro Bank durchgeführte gleichzeitige Präparation von 6 Aliquots ä 160 000 Klonen und Durchführung 6 paralleler Bead-Beladungen stellte sicher, daß ca. $10^6$ mRNA Molekülen und damit auch schwach exprimierte Transkripte in der Bank repräsentiert waren.

Die beladenen Microbeads wurden einer weiteren enzymatischen Behandlung unterzogen. Zuerst wurde durch T4-Polymerase-Behandlung die Lücke zwischen Anti-Tag und Gegenstrang aufgefüllt (Fig.4.A) und durch Behandlung mit T4-Ligase wurde der Strang kovalent geschlossen (Fig.4.B). Anschließend wurde mit Dpnll geschnitten, um die FAM-Markierung am 5'-Ende des Gegenstrangs zu entfemen (Fig.4.C) und es erfolgte eine Ligation mit einem FAM-markierten Adapter, dergestalt, daß sich das Fluoreszenz-Molekül am 3'-Ende des oberen Strangs befand (Fig. 4.D). Anschließend wurde der obere Strang durch Alkali-Behandlung (2mal 15 min mit 0.15 N NaOH bei Raumtemperatur) entfernt (Fig.4.E). Zwischen jedem Behandlungsschritt wurde mit Pronase behandelt, um die jeweiligen Enzyme zu inaktivieren. Dieser Beladungsprozeß wurde sowohl für cDNA aus ruhenden T-Zellen als auch für cDNA aus PHA-aktivierten T-Zellen durchgeführt. Die beladenen Microbeads waren jetzt für die nachfolgenden Hybridisierungsreaktionen einsatzbereit.

Für die kompetitive Hybridisierung mit unterschiedlich fluoreszenzmarkierten cDNA-Proben (FAM bzw. Cy5) aus ruhenden bzw. PHA-aktivierten T-Zellen wurde eine 1:1-Mischung der cDNA-beladenen Microbeads von ruhenden und PHA-aktivierten T-Zellen eingesetzt. Für die Herstellung der fluoreszenzmarkierten cDNA-Proben wurde die aus ruhenden bzw. PHA-aktivierten T-Zellen im CTI1-Vektor hergestellte Genbank in Bakterien amplifiziert. Aus $10^7$ amplifizierten Klonen wurden die Plasmide isoliert. Anschließend wurden die Plasmide mittels einer vektorinternen Schnittstelle linearisiert und in einer linearen PCR mit Hilfe fluoreszenzmarkierter Primer (FLin1-FAM bzw. Flin1-Cy5; LYNX Therapeutics Inc., Hayward, USA) amplifiziert. Die solcherart gewonnene fluoreszenzmarkierte cDNA (1:1 Mischung der FAM-markierten cDNA aus ruhenden T-Zellen und der Cy5-markierten cDNA aus PHA-aktivierten T-Zellen) wurde für die kompetitive Hybridisierung mit der o.g. 1:1-Mischung beladener Microbeads herangezogen.

Nach der kompetitiven Hybridisierung (65 °C über Nacht) wurde ungebundenes Material durch mehrmaliges Waschen entfernt und die Microbeads mittels eines Zweifarben-Fluoreszenzaktivierten Sortiergeräts (MoFlo® MLS Flow Cytometer; Cytomation Bioinstruments GmbH) analysiert und 1 % derjenigen Microbeads, die die stärkste Rot- bzw. Grünfluoreszenz aufwiesen wurden sortiert.

Die auf den Microbeads gebundenen cDNA-Fragmente wurden mit Hilfe flankierender Primer (forward: "Comp top"; reverse: "Comp bot") amplifiziert. Die Amplifikationsprodukte wurden unter Verwendung des kommerziell verfügbaren TA Klonierungs-Bestecks (Invitrogen, Bestellnummer K4500-04) kloniert und in Bakterien transformiert. Mittels Blau/Weiß Selektion wurden Insert-positive Klone identifiziert. Nach Amplifikation der Bakterien im Microtiterplatten-Format ("96-well"-Platten) wurden die Plasmide mit einem kommerziell verfügbaren Isolationsbesteck isoliert (Qiagen; Bestellnummer 27191). Die so aufgereinigte Plasmid DNA wurde nach Standardprotokoll unter Verwendung der "Cycle-Sequencing"-Methode und eines ABI PRISM® 3700 DNA Analyzer (PE Applied Biosystems) unter Verwendung kommerziell verfügbarer Sequenzierchemikalien sequenziert. Die erhaltenen Sequenzen der cDNA-Fragmente sind in den Sequenzprotokollen SEQ ID NO. 1 bis NO. 334 aufgeführt.

**Beispiel 3**

**Anwendung einer Auswahl der in den Sequenzprotokollen SEQ ID NO. 1 bis NO. 334 genannten Polynukleotide zur Bestimmung eines Genexpressionsprofils in (a) ruhenden Phytohämagglutinin (PHA) aktivierten T-Zellen,**

**und in (b) PHA-aktivierten T-Zellen, die mit dem Immunsuppressivum Ciclosporin A behandelt wurden, mit der Methode der Filterhybridisierung**

Aus der Gruppe der in den Sequenzprotokollen SEQ ID NO. 1 bis NO. 334 angegebenen Polynukleotide wurde eine Kombination von cDNAs ausgewählt (SEQ ID NO. 1, NO. 8, NO. 26, NO. 83, NO. 256, NO. 261, NO. 267, NO. 291), die in einer sog. "Filterhybridisierung" zur Analyse der Genexpression in nichtaktivierten, PHA-aktivierten und PHA/Ciclosporin-A behandelten T-Zellen herangezogen wurden. Die verwendeten nichtaktivierten und PHA-aktivierten T-Zellen wurden gemäß Beispiel 1 hergestellt. Für die Gewinnung der PHA/Ciclosporin-A behandelten T-Zellen wurde wie in Beispiel 1 beschrieben vorgegangen mit der Abwandlung, daß ein Teil der mit PHA stimulierten T-Zellen zusätzlich mit dem Immunsuppressivum Ciclosporin A behandelt wurde. Hierfür wurde den in Vollmedium in Kulturschalen ausgesäten T-Zellen das Immunsuppressivum Ciclosporin A (Sigma GmbH, Bestellnummer C-1832) in einer Konzentration von 5 µg/ml zugesetzt. Die Zellen wurden für 1 Stunde mit Ciclosporin A vorinkubiert und anschließend für weitere 5 Stunden in Gegenwart von PHA inkubiert.

Zusätzlich wurden noch die cDNAs dreier bekannter T-Zellaktivierungsmarker (1 = CD69, 2 = CD95 und 3 = IL-2) für die Filterhybridisierung eingesetzt.

Im einzelnen wurde folgendermaßen vorgegangen: zur Beschickung der Filter wurden die ausgewählten cDNA-Klone mittels PCR und insertflankierender Primer ("CompTop" und "CompBot"; LYNX Therapeutics Inc., Hayward, USA) nach Standardmethoden amplifiziert.

Die cDNAs für die bekannten T Zellantigene (CD69, CD95 und IL-2) wurden mittels PCR nach Auswahl spezifischer Primerpaare aus einer Genbank aktivierter T Zellen amplifiziert.

Die PCR-Produkte wurden mittels Äthanol-Fällung (EtOH/3 M NaOAc pH = 5.2) aufkonzentriert und in 20 µl Aqua bidestillata resuspendiert. Die Amplifikationsprodukte wurden unter Verwendung eines "Spotting"-Roboters (Labman Automation Ltd, Stokesley, England) und einem 384 PinTool (BioRobotics, Bestellnummer 11-8-2) auf Nylonmembranen (Amersham Pharmacia, Hybond-N+, Bestellnummer 2250B) punktförmig aufgebracht (10 x 10 Nanoliter/Punkt). Anschließend wurde die aufgebrachte DNA mittels NaOH-Behandlung (0.4 M, 5 min) in Einzelstrangform überführt. Nach kurzer Behandlung (2 min) mit aqua bidestillata erfolgte eine Fixierung auf der Membran durch UV-vermittelte Kreuzvernetzung.

Die mRNA aus den stimulierten Zellpopulationen (s.o.) wurde nach Standardprotokoll isoliert. Jeweils 200 ng mRNA wurden in cDNA umgeschrieben (Superscript II Reverse Transcriptase, Life Technology, Bestellnummer 18064-014; dATP, dGTP,dTTP-Mix Pharmacia, Bestellnummer 27-2035-01). Die cDNA wurde durch Verwendung von α-[$^{33}$P]dCTP (Amersham Pharmacia Biotech; Bestellnummer AH9905) radioaktiv markiert. Nach 90 Minuten Inkubation bei 38°C wurde die Reaktion durch Zusatz von 45 µL 50 mM EDTA gestoppt. Die radioaktiv markierte cDNA wurde mittels Biospin 6-Säulen (BioRad; Bestellnummer 732-6002) aufgereinigt.

Die Filter wurden zunächst bei 90°C für 3 min mit 0.1 SSC (1 x SSC: 0.15 M NaCI, 0.015 NaCitrate-Puffer), 0.1 % SDS behandelt ("Strippen"), anschließend wurde mit "Church-Puffei" (0.25 M $Na_2HPO4$, pH 7.2, 10 mM EDTA, 7 % SDS) gewaschen. Die Filter wurden in eine Hydridisierungsröhre überführt und dann für eine Stunde in Church-Puffer (1,2 ml) bei 68°C unter Rotation in einem Hybridisierungsofen (Biometra, Model OV3, Bestellnummer 052-200) vorhybridisiert.

Die radioaktiv markierte cDNA wurde mit 50 µl Salmon Sperm DNA (10 mg/ml, Stratagene, 201190) versetzt, bei 95 °C denaturiert (10 min), kurz auf Eis gesetzt und dann vorsichtig in die Hybridisierungsröhre (Filter, Church Puffer) gegeben.

Die Hybridisierung erfolgte bei 68 °C unter Rotation im Hybridisierungsofen. Nach 18 Stunden wurden die Filter folgendermaßen gewaschen: (1) 2 x 30 min mit 2 x SSC, 0.1 % SDS bei 68 °C, (2) 2 x 30 min mit 0.2 x SSC 0.1 % SDS bei 68 °C, (3) 1 x 0.2 x SSC, 0.1 % SDS bei Raumtemperatur.

Die Filter wurden blasenfrei und wasserdicht in Klarsichthülle verpackt und für 24 Stunden auf einer Phosphorimager-Platte (Raytest Isotopenmeßgeräte GmbH, BAS-Imaging Plate, Bestellnummer BAS-MS 2040S) aufgelegt. Die exponierte Phosphorimager-Platte wurde mit einem FUJI-FLA 2000 Phosphoimager mit einer Auflösung von 100 µm und 16 bit eingelesen (BASR-Reader, Raytest Isotopenmeßgeräte GmbH). Das Bild wurde anschließend mit dem Software-Programm "Array-Vision" (4.0 Rev. 1.7, Imaging Research Inc., St. Catharines, Kanada) nach Herstellerangaben analysiert. Dieses Programm bestimmt die Signalintensität (PSL) jeden Punkts und setzt diesen in Beziehung zur Hintergrundfärbung (Bkgd) auf dem Filter. Bei einem Vergleich verschiedener Filter erfolgt eine sog. Normalisierung, bei der die individuelle Stärke eines Signalpunkts auf die mittlere Signalintensität (sREF) des jeweiligen Filters bezogen wird.

$$(PSL - BKgd) / sREF = \text{normalisierte Signalintensität (nPSL)}$$

Das Expressionverhalten ergibt sich aus dem Verhältnis nPSL (aktivierte T-Zellen)/nPSL(unbehandelte T-Zellen).

Die für die einzelnen cDNAs erhobenen Expressionswerte im unstimulierten Zustand wurden als 0 und die Expressionswerte nach Stimulation mit PHA als 100 gesetzt. Die Beeinflussung durch Ciclosporin A wurde in dem Bereich zwischen 0 und 100 quantifiziert.

Fig. 5 zeigt das Ergebnis dieses Experiments, wobei gilt:

1 = CD 95 (bekannter Aktivierungsmarker)
2 = CD 69 (bekannter Aktivierungsmarker)
3 = IL-2 (bekannter Aktivierungsmarker)
4 = Profilin (SEQ ID NO. 267)
5 = EST (SEQ ID NO. 261)
6 = Ribosomales Protein L9 (SEQ ID NO. 291)
7 = 90 kDa Heat Shock Protein (SEQ ID NO. 256)
8 = keine Homologie (SEQ ID NO. 1)
9 = EST (SEQ ID NO. 26)
10 = Methionine t-RNA synthetase (SEQ ID NO. 83)
11 = EST (SEQ ID NO. 8)

A = Filter hybridisiert mit radioaktiver cDNA nichtaktivierter T-Zellen
B = Filter hybridisiert mit radioaktiver cDNA PHA-aktivierter T-Zellen
C = Filter hybridisiert mit radioaktiver cDNA PHA-aktivierter und Ciclosporin A-vorbehandelter T-Zellen

Es zeigt sich, daß die bekannten Aktiverungsmarker CD 69, CD 95 und IL-2 in nichtaktivierten T-Zellen nicht exprimiert sind, in PHA-aktivierten T-Zellen jedoch aufreguliert sind. Durch Ciclosporin A wird die PHA-induzierte Aufregulation dieser Marker bzw. Indikatoren vollständig inhibiert. Zusammengefaßt zeigt sich ein Schwarz/Weiß-Bild, was bei den T-Zellen keine Differenzierung zwischen Ruhezustand und Stimulation in Gegenwart des Immunsuppressivums Ciclosporin-A zuläßt.

Die erfindungsgemäßen neu identifizierten Indikatoren (Marker) 4 - 11 zeigen eine deutlich differenziertere Reaktion: die Indikatoren 4 - 7 zeigen bei PHA-Aktivierung eine Aufregulation, während die Indikatoren 8 - 11 eine Abregulation nach PHA-Stimulation zeigen. In Gegenwart von Ciclosporin A wurde die PHA-induzierte Expressionsänderung unterschiedlich beeinflußt. Während die PHA-induzierte Aufregulation von Indikator 7 durch Ciclosporin A deutlich beeinflußt wurde, wurde die PHA-induzierte Aufregulation von Indikator 4 durch Ciclosporin A nur gering beeinflußt. Auch wurde die PHA-induzierte Abregulation von Indikator 8 durch Ciclosporin A stark inhibiert, während die PHA-induzierte Abregulation von Indikator 11 durch Ciclosporin A nur schwach beeinflußt wurde.

Zusammengefaßt zeigt dieses Experiment, daß durch die vorliegende Erfindung das Repertoire der verfügbaren T-Zell-Marker bzw. T-Zell-Indikatoren

1. um solche erweitert wird, die nach Aktivierung aufreguliert werden,
2. um solche erweitert, die nach Aktivierung abreguliert werden, und
3. um solche erweitert wird, die keine Schwarz/Weiß Reaktion sondern eine graduelle Reaktion nach Exposition gegenüber einem Immunsuppressivum zeigen.

**Patentansprüche**

1. mRNA zur Verwendung als Indikator für den Aktivierungs- und Funktionszustand von T-Zellen, **dadurch gekennzeichnet,**

   **daß** sie bei aktivierten T-Zellen im Vergleich zum Normal- bzw. Ruhezustand vermehrt oder vermindert exprimiert ist und auf dem höheren bzw. niedrigeren Konzentrationsspiegel gehalten ist,
   und **daß** sie mit einer der in den Sequenzprotokollen SEQ ID NO. 1 bis NO. 334 dargestellten Nukleotidsequenzen oder einer davon abgeleiteten Nukleotidsequenz oder einer hierzu komplementären Nukleotidsequenz oder einer Teilsequenz einer dieser Nukleotidsequenzen hybridisiert, wobei SEQ ID NO. 1 bis SEQ ID NO. 334 Bestandteile dieses Anspruchs sind.

2. Polynukleotid mit einer in den Sequenzprotokollen SEQ ID NO. 1 bis NO. 334 angegebenen Nukleotidsequenz oder einer davon abgeleiteten Nukleotidsequenz oder einer dazu komplementären oder damit hybridisierenden Nukleotidsequenz zur Verwendung als Nachweisreagenz für den Aktivierungs- und Funktionszustand von T-Zellen.

3. Verwendung eines Polynukleotids mit einer in den Sequenzprotokollen SEQ ID NO. 1 bis NO. 334 angegebenen Nukleotidsequenz oder einer davon abgeleiteten Nukleotidsequenz oder einer dazu komplementären oder damit hybridisierenden Nukleotidsequenz als bzw. zur Herstellung von Diagnostika und/oder Therapeutika für den Nachweis und/oder die Beeinflussung und/oder Steuerung des Aktivierungs- und Funktionszustands von T-Zellen .

4. Polynukleotid mit einer in den Sequenzprotokollen SEQ ID NO. 1 bis NO. 334 angegebenen Nukleotidsequenzen oder einer dazu komplementären oder damit hybridisierenden Nukleotidsequenz oder einer davon abgeleiteten Nukleotidsequenz zur Verwendung als Diagnostikum und/oder Therapeutikum für Immunität, Autoimmunität, Transplantatabstoßungsreaktionen, akute und/oder chronische, immunologisch bedingte Entzündungsreaktionen und/oder Immunsuppression.

5. Verwendung eines Polynukleotids mit einer in den Sequenzprotokollen SEQ ID NO. 1 bis NO. 334 angegebenen Nukleotidsequenzen oder einer davon abgeleiteten Nukleotidsequenz oder einer dazu komplementären oder damit hybridisierenden Nukleotidsequenz zur Identifizierung von medizinisch oder pharmakologisch einsetzbaren Substanzen, die an das Polynukleotid bzw. an das/die von diesem kodierte Protein(e) binden und dadurch den Aktivierungs- und Funktionszustand von T-Zellen beeinflussen

6. Verwendung eines Polynukleotids mit einer in den Sequenzprotokollen SEQ ID NO. 1 bis NO. 334 dargestellten Nukleotidsequenzen oder einer davon abgeleiteten Nukleotidsequenz oder einer dazu komplementären oder damit hybridisierenden Nukleotidsequenz zur Herstellung von Testbestecken zur Messung eines Genexpressionsprofils in ex vivo isolierten Immunzellen, insbesondere T-Zellen, wobei der Test die folgenden Schritte umfaßt:

   (i) Isolation von mRNA aus Geweben, Zellen oder Körperflüssigkeiten bei Gesunden,
   (ii) Isolation von mRNA aus Geweben, Zellen oder Körperflüssigkeiten bei Erkrankten,
   (iii) Einsatz dieser mRNAs, bzw. davon abgeschriebener cDNA, in Bindungsverfahren zur Messung der Interaktion mit einer oder mehreren der in SEQ ID NO. 1 bis NO. 334 beschriebenen Polynukleotide,
   (iv) Detektion und Identifikation derjenigen mRNA-Spezies, die bei Gesunden und Erkrankten eine unterschiedliche Expressionsstärke zeigen.

7. Verwendung eines Polynukleotids mit einer in den Sequenzprotokollen SEQ ID NO. 1 bis NO. 334 dargestellten Nukleotidsequenzen oder einer davon abgeleiteten Nukleotidsequenz oder einer dazu komplementären oder damit hybridisierenden Nukleotidsequenz zur Herstellung von Testbestecken zur Messung eines Genexpressionsprofils in ex vivo isolierten Immunzellen, insbesondere T-Zellen, wobei der Test die folgenden Schritten umfaßt:

   (i) Durchführung einer bioinformatischen Analyse in verschiedenen Datenbanken hinsichtlich Vollängensequenz und/oder Sequenzvarianten der Nukleotidsequenz des Polynukleotids,
   (ii) Synthese von Oligonukleotiden anhand der gewonnenen Vollängensequenz und Sequenzvarianten,
   (iii) Aufbringen der Oligonukleotide auf ein Trägermaterial,
   (iv) Isolation von mRNA aus Geweben, Zellen oder Körperflüssigkeiten bei Gesunden,
   (v) Isolation von mRNA aus Geweben, Zellen oder Körperflüssigkeiten bei Erkrankten,
   (vi) Einsatz dieser mRNAs bzw. davon abgeschriebener cDNA in Bindungsverfahren zur Messung der Interaktion mit den auf dem Trägermaterial aufgebrachten Oligonukleotiden,
(vii) Detektion und Identifikation derjenigen mRNA-Spezies, die bei Gesunden und Erkrankten eine unterschiedliche Expressionsstärke zeigen.

**8.** Verwendung eines Polynukleotids mit einer in den Sequenzprotokollen SEQ ID NO. 1 bis NO. 334 dargestellten Nukleotidsequenzen oder einer davon abgeleiteten Nukleotidsequenz oder einer dazu komplementären oder damit hybridisierenden Nukleotidsequenz zur Herstellung von Testbestecken zur Messung eines Genexpressionsprofils in ex vivo isolierten Immunzellen, insbesondere T-Zellen, wobei der Test die folgenden Schritte umfaßt:

(i) Synthese von Oligonukleotidprimern für die Polymerase-Kettenreaktion (PCR) anhand entweder der Nukleotidsequenz des Polynukleotids oder der mittels bioinformatischer Analyse in verschiedenen Datenbanken gewonnenen Vollängensequenz und/oder Sequenzvarianten des Polynukleotids,
(ii) Isolation von mRNA aus Geweben, Zellen oder Körperflüssigkeit bei Gesunden,
(iii) Isolation von mRNA aus Geweben, Zellen oder Körperflüssigkeiten bei Erkrankten,
(iv) Einsatz dieser mRNAs bzw. davon abgeschriebener cDNA in einer Polymerase-Kettenreaktion (PCR) und Feststellung von Unterschieden in der Expressionsstärke einzelner Gene.
(v) Detektion und Identifikation derjenigen mRNA-Spezies, die bei Gesunden und Erkrankten eine unterschiedliche Expressionsstärke zeigen.

**9.** Polypeptid, das von einer der in den Sequenzprotokollen SEQ ID NO. 1 bis NO. 334 angegebenen Nukleotidsequenz oder einer davon abgeleiteten Nukleotidsequenz oder einer dazu komplementären oder damit hybridisierenden Nukleotidsequenz abgeleitet ist, und das bei aktivierten T-Zellen im Vergleich zum Normal- bzw. Ruhezustand vermehrt oder vermindert exprimiert ist und auf dem höheren bzw. niedrigeren Konzentrationsspiegel gehalten ist.

**10.** Reagenz zum Nachweis des Aktivierungs- und Funktionszustand von T-Zellen, **dadurch gekennzeichnet, daß** das Reagenz unter Verwendung wenigstens einer der in den Sequenzprotokollen SEQ ID NO. 1 bis NO. 334 angegebenen Nukleotidsequenz oder einer davon abgeleiteten Nukleotidsequenz oder einer dazu komplementären oder damit hybridisierenden Nukleotidsequenz hergestellt ist.

**11.** Reagenz nach Anspruch 10, **dadurch gekennzeichnet, daß** es eine Substanz enthält, die spezifisch mit einer der in den Sequenzprotokollen SEQ ID NO. 1 bis NO. 334 angegebenen Nukleotidsequenz oder einer davon abgeleiteten Nukleotidsequenz oder einer dazu komplementären oder damit hybridisierenden Nukleotidsequenz reagiert.

**12.** Reagenz nach Anspruch 11, **dadurch gekennzeichnet, daß** die Substanz ein Polynukleotid ist, das einen nachweisbaren Marker (z.B. ein Lumineszenzgen o.ä.) aufweist.

**13.** Reagenz nach Anspruch 11, **dadurch gekennzeichnet, daß** die Substanz ein modifiziertes Oligo- oder Polynukleotid ist.

**14.** Rekombinantes DNS-Vektormolekül, das eine oder mehrere der in den Sequenzprotokollen SEQ ID NO. 1 bis NO. 334 dargestellten Nukleotidsequenzen oder einer davon abgeleiteten Nukleotidsequenz oder eine dazu komplementäre oder damit hybridisierende Nukleotidsequenz aufweist, und das in T-Zellen transkribierbar ist.

**15.** Verwendung einer oder mehrerer der in den Sequenzprotokollen SEQ ID NO. 1 bis NO. 334 dargestellten Nukleotidsequenzen oder einer davon abgeleiteten Nukleotidsequenz oder dazu komplementären oder damit hybridisierenden Nukleotidsequenzen für die Herstellung polyklonaler, monoklonaler oder rekombinanter Antikörper gegen das/die von der/den betreffenden Nukleotidsequenz(en) kodierte(n) Protein(en).

**16.** Antikörper, der spezifisch mit wenigstens einem Protein reagiert, das von den in den Sequenzprotokollen SEQ ID NO. 1 bis NO. 334 dargestellten Nukleotidsequenzen oder einer davon abgeleiteten Nukleotidsequenz oder dazu komplementären oder damit hybridisierenden Nukleotidsequenzen ableitbar ist.

**17.** Verwendung eines Antikörpers nach Anspruch 16 zum Nachweis oder zur Beeinflussung des Aktivierungs- und Funktionszustands von T-Zellen.

**18.** Verwendung einer oder mehrerer der in den Sequenzprotokollen SEQ ID NO. 1 bis NO. 334 dargestellten Nukleotidsequenzen oder einer davon abgeleiteten Nukleotidsequenz oder dazu komplementären oder damit hybridisierenden Nukleotidsequenzen zur Herstellung von Bindemolekülen, insbesondere RNA-Antisense-Moleküle, modifizierten Nukleinsäuren mit Antisense-Funktion und/oder Ribozymen, für diese Nukleotidsequenzen und/oder damit verwandten Nukleotidsequenzen.

**19.** Bindemoleküle, insbesondere RNA-Antisense-Moleküle, modifizierte Nukleinsäuren mit Antisense-Funktion und/oder Ribozyme, die spezifisch mit einer oder mehrerer der in den Sequenzprotokollen SEQ ID NO. 1 bis NO. 334 dargestellten Nukleotidsequenzen oder einer davon abgeleiteten Nukleotidsequenz oder dazu komplementären oder damit hybridisierenden Nukleotidsequenzen reagieren.

**20.** Verwendung eines Bindemoleküls nach Anspruch 19 zum Nachweis oder zur Beeinflussung des Aktivierungs- und Funktionszustand von T-Zellen.

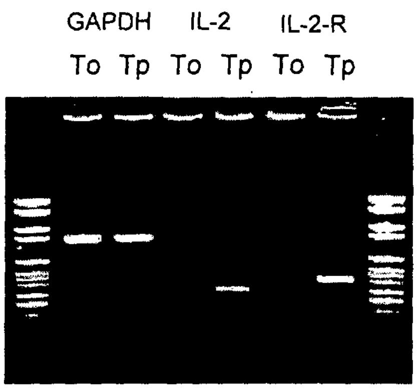


Fig.1

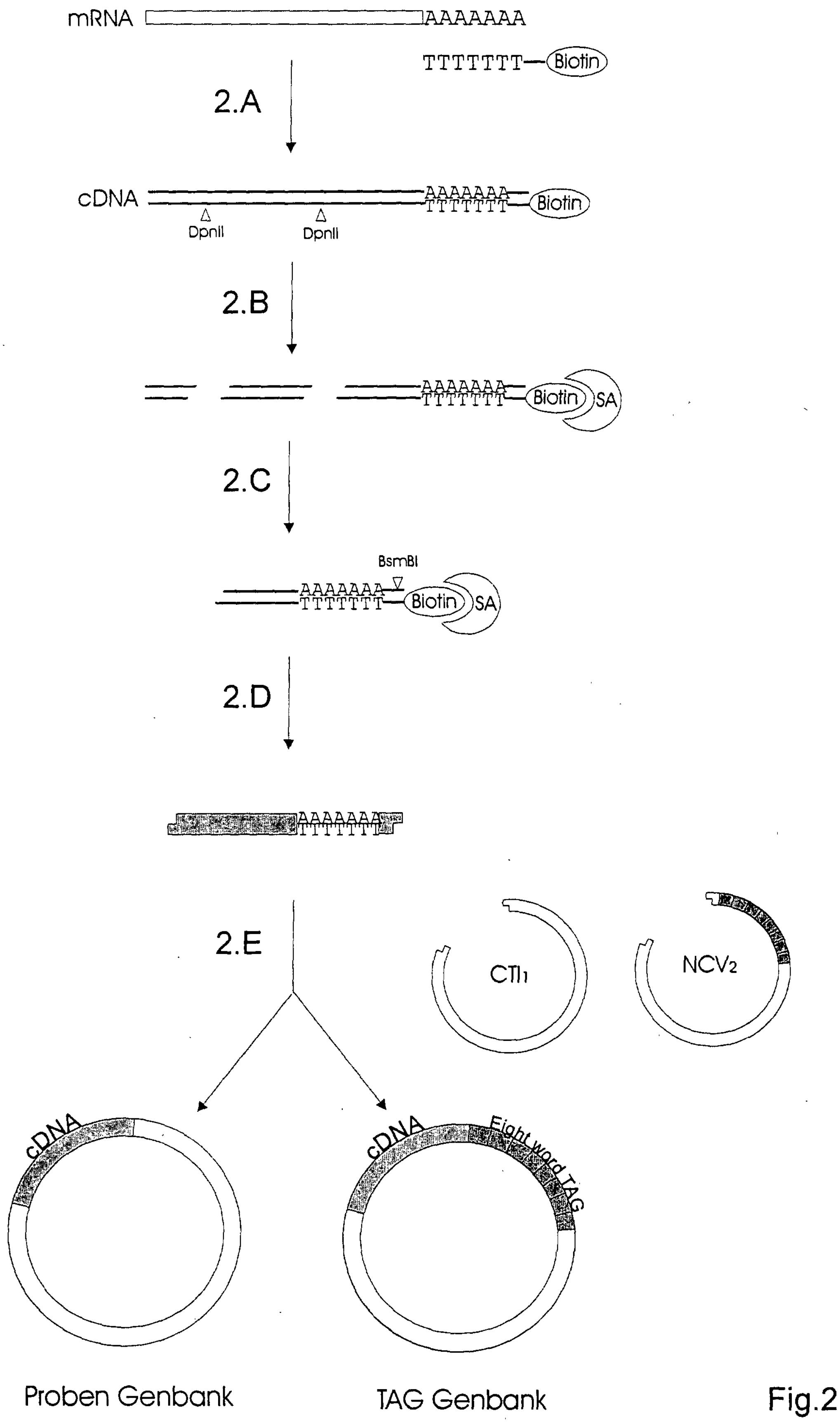
mRNA
AAAAAAA
TTTTTTT
Biotin
2.A
cDNA
AAAAAAA
TTTTTTT
Biotin
DpnII
DpnII
2.B
AAAAAAA
TTTTTTT
Biotin
SA
2.C
BsmBI
AAAAAAA
TTTTTTT
Biotin
SA
2.D
AAAAAAA
TTTTTTT
2.E
CTI1
NCV2
cDNA
cDNA
Eight word TAG
Proben Genbank
TAG Genbank

Fig.2

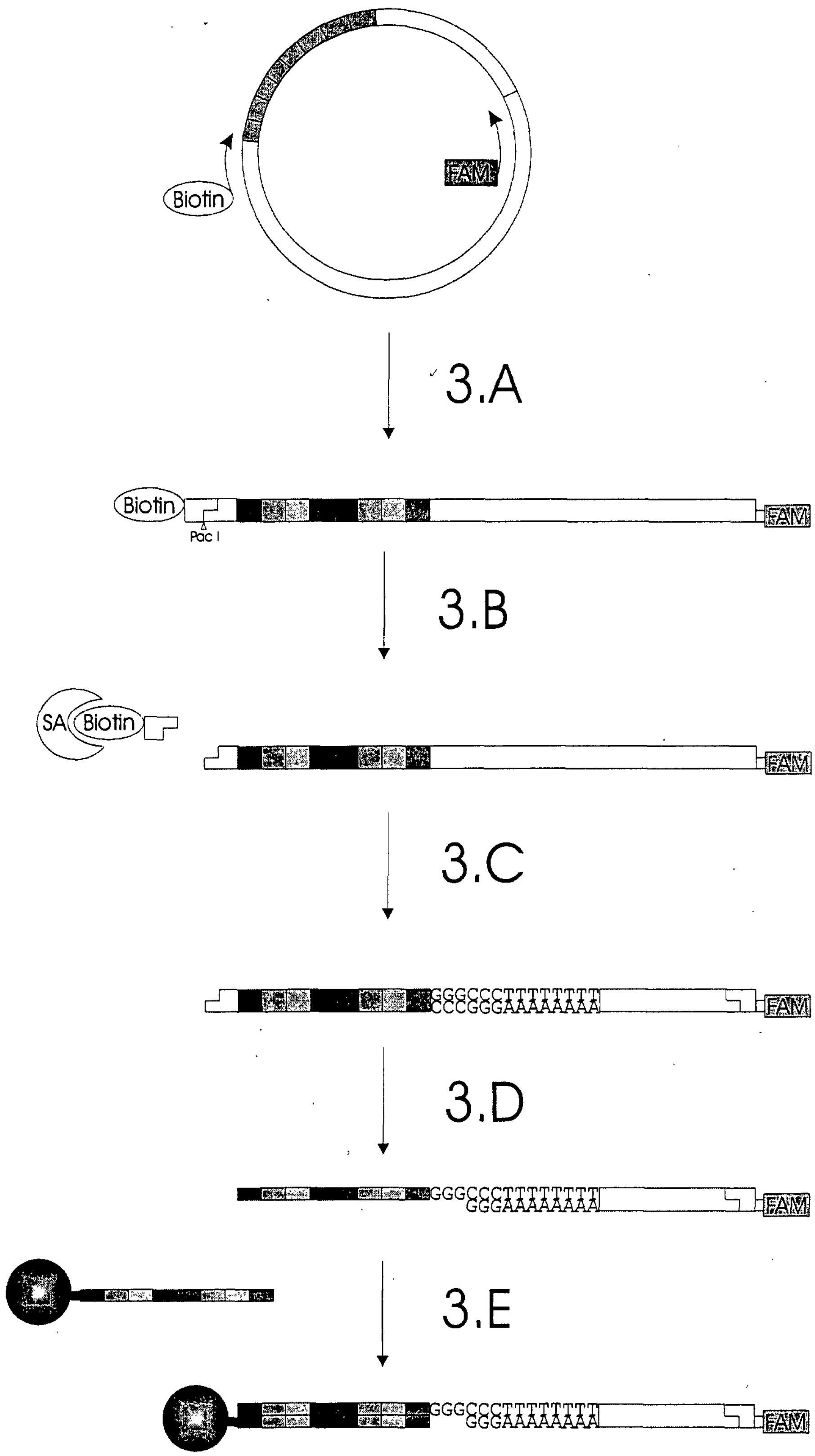


Fig.3

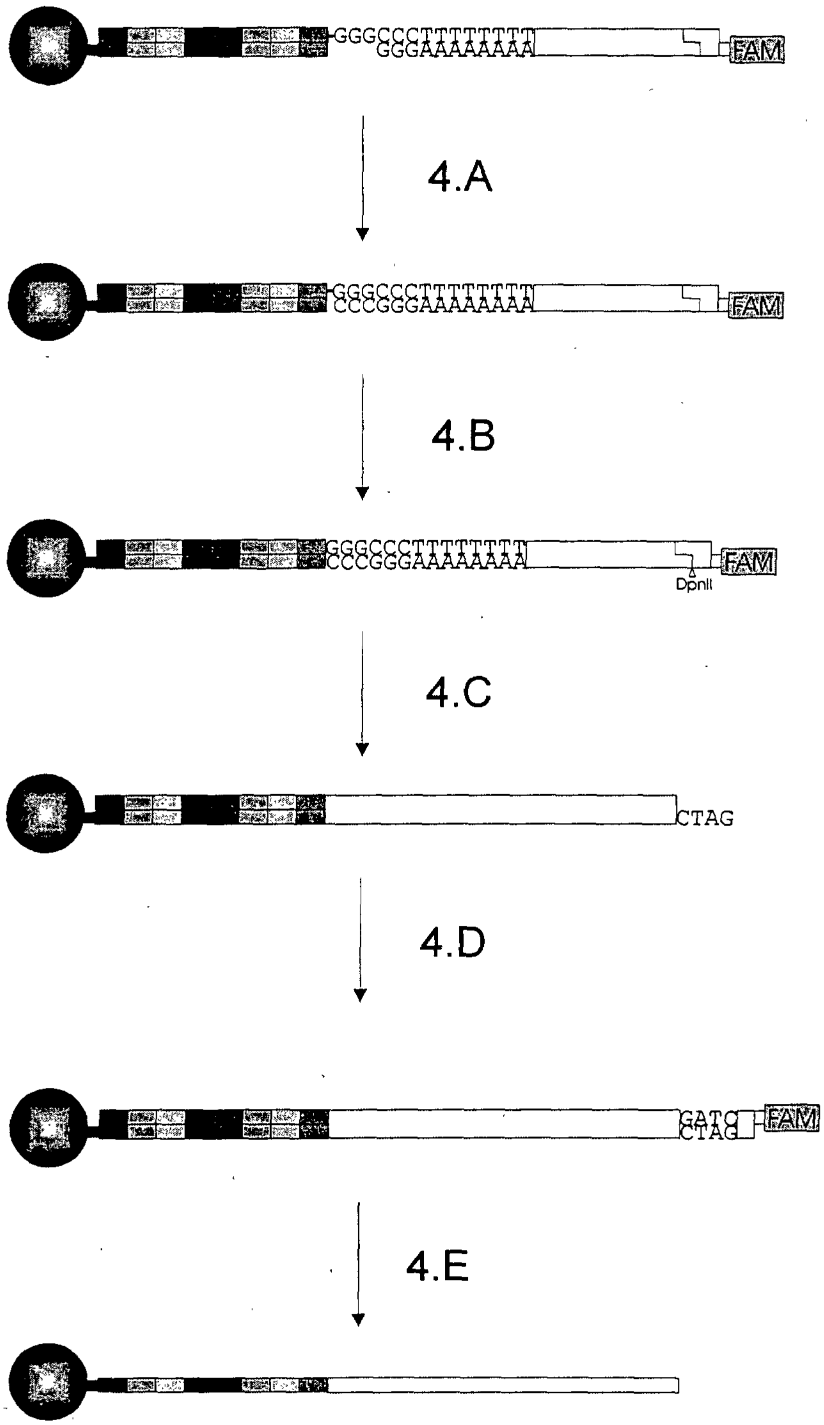
GGGCCCTTTTTTTT
GGGAAAAAAAA
FAM
4.A
GGGCCCTTTTTTTT
CCCGGGAAAAAAAA
FAM
4.B
GGGCCCTTTTTTTT
CCCGGGAAAAAAAA
FAM
DpnII
4.C
CTAG
4.D
GATC
CTAG
FAM
4.E

Fig.4

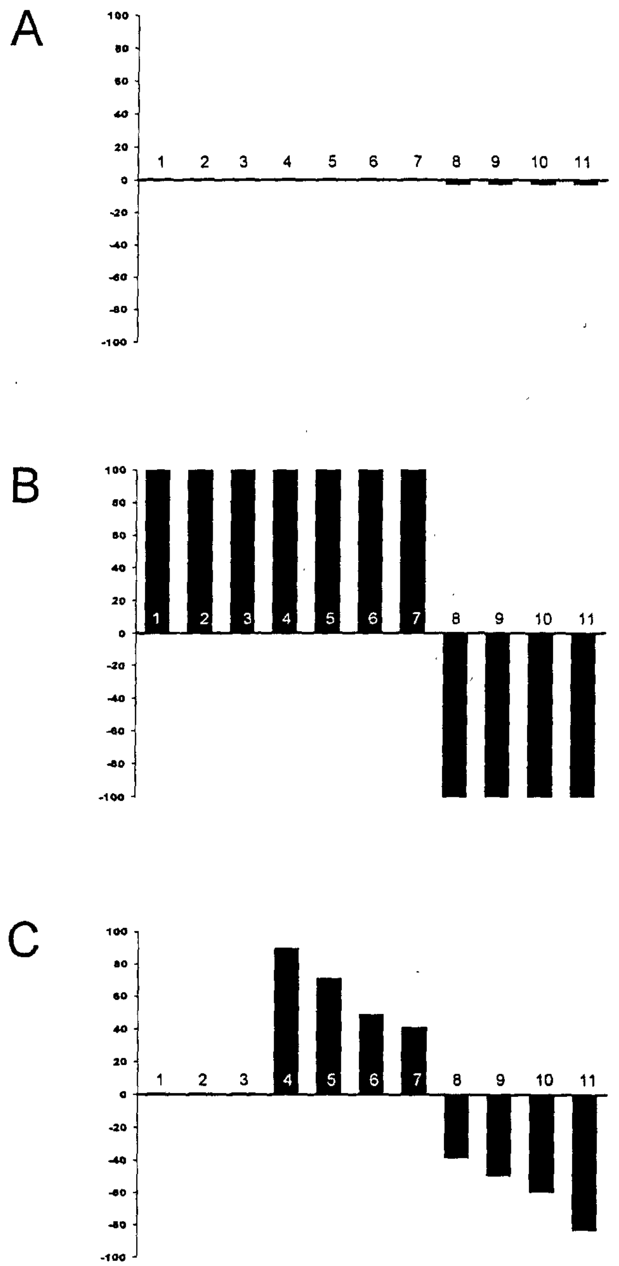
A
100
80
60
40
20
0
-20
-40
-60
-80
-100
1 2 3 4 5 6 7 8 9 10 11
B
100
80
60
40
20
0
-20
-40
-60
-80
-100
1 2 3 4 5 6 7 8 9 10 11
C
100
80
60
40
20
0
-20
-40
-60
-80
-100
1 2 3 4 5 6 7 8 9 10 11

Fig.5